(19) Europäisches Patentamt
European Patent Office
Office européen des brevets

(11) Publication number : **0 517 498 A1**

(12) # EUROPEAN PATENT APPLICATION

(21) Application number : **92305079.3**

(22) Date of filing : **03.06.92**

(51) Int. Cl.[5] : **C09K 19/32,** C09K 19/34, C09K 19/42, C07C 43/225, C07C 43/20, C07C 43/205, C07C 69/76, C07C 69/94, C07D 213/30, C07D 213/06, C07D 213/55, C07D 213/64, C07D 213/79, C07D 239/26, C07D 239/34

(30) Priority : **03.06.91 JP 159787/91**
**11.06.91 JP 167605/91**

(43) Date of publication of application :
**09.12.92 Bulletin 92/50**

(84) Designated Contracting States :
**DE FR GB IT**

(71) Applicant : **SANYO CHEMICAL INDUSTRIES LTD.**
**11-1, Ichinohashinomoto-cho Higashiyama-ku, Kyoto-shi**
**Kyoto 605 (JP)**

(72) Inventor : **Satoh, Masahiro, Corporouse Fushimi 112**
**No. 32, Fukakusa-ikenouchi-cho**
**Fushimi-ku, Kyoto (JP)**
Inventor : **Sugita, Naoko**
**No. 12-44, Higashinominamiinoue-cho**
**Yamashina-ku, Kyoto (JP)**
Inventor : **Watanabe, Tetsuya**
**71-38, Muranohigashimachi**
**Hirakata-shi, Osaka-fu (JP)**
Inventor : **Yoshio, Kunikiyo**
**No. 1922-361, Noji-cho**
**Kusatsu, Shiga-ken (JP)**
Inventor : **Yanagi, Tatsuroh**
**No. 10-1, Imakumano-minamihiyoshi-cho**
**Higashiyama-ku, Kyoto (JP)**

(74) Representative : **Marlow, Nicholas Simon**
**Reddie & Grose 16, Theobalds Road**
**London WC1X 8PL (GB)**

(54) **Liquid crystal compounds and compositions.**

(57) Liquid crystal naphtalene compounds, represented by the following formula (1) are disclosed.

R-Z-A-NAP-Z'-R'     (1)

In the formula (1), R is an alkyl group containing 1 to 18 carbon atoms ; R' is an alkyl group containing 1 to 21 carbon atoms ; NAP represents 2,6-naphthylene group ; A, Z and Z' are as follows : 1) A is Pyr> and (a) Z is - or &num ; and Z' is O, or (b) Z is - and Z' is COO ; 2) if A is Pym>, (a) Z is - and Z' is O, or (b) Z is O and Z' is -, O or &num ; ; 3) A is FPhF and (a) Z is - or O and Z' is -, O or COO, (b) Z is OCO and Z' is - or O, or (c) Z is &num ; and Z' is O ; 4) A is PhF and (a) Z is - or &num ; and Z' is O, (b) Z is O and Z' is -, or (c) Z is - and Z' is COO ; 5) A is FPh and (a) Z is O and Z' is-, (b) Z is -, O, &num ; or OCO and Z' is O, or (c) Z is O and Z' is COO ; 6) A is <Pyr and Z is -, O or &num ; and Z' is O ; or 7) A is Ph and one of Z and Z' is - and the other is O ;

· wherein : Pyr> = 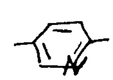 , <Pyr = 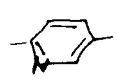 , Pym> = 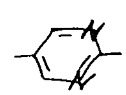 ,

FPhF = 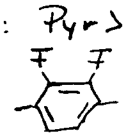 , PhF = 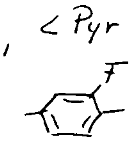 , FPh = 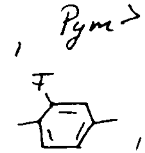 , — = a single bond

and # represents ·C≡C· .

As a component of liquid crystal compositions showing chiral smectic C phase, these compounds can provide liquid crystal compositions of improved orientation and free from zigzag deffects, and liquid crystal display elements of high contrast ratio.

EP 0 517 498 A1

## BACKGROUND OF THE INVENTION

### 1. Field of the Invention

This invention relates to liquid crystal compounds and liquid crystal compositions, useful for liquid crystal display elements and the like. More particularly, it relates to liquid crystal naphthalene compounds showing non-chiral smectic C phase (hereinafter referred to as Sc phase) or chiral smectic C phase (hereinafter referred to as Sc* phase) and liquid crystal compositions containing these liquid crystal compounds.

### 2. Description of the Prior Art

As liquid crystal compounds showing Sc phase or Sc* phase, used in ferroelectric liquid crystal materials, there have been known liquid crystal phenyl pyrimidine compounds, such as 5-alkyl-2-(4′-alkoxyphenyl)pyrimidines.

However, these liquid crystal compounds and compositions containing these compounds are likely result in zigzag deffects, because of poor orientation, when introduced into cells, and cannot provide sufficiently high contrast ratio.

The present invention can provide a liquid crystal compound showing Sc or Sc* phase of improved orientation.

The present invention can provide a liquid crystal compound capable of providing ferroelectric liquid crystal composition substantially free from zigzag deffects.

This invention can further provide a liquid crystal compound and composition capable of providing liquid crystal display elements of improved contrast ratio.

The invention can yet further provide a liquid crystal compound, showing Sc or Sc* phase over a wide temperature range.

The present invention provides liquid crystal compounds represented by the following general formula (1):

R-Z-A-NAP-Z′-R′ (1)

In the formula (1), R is an alkyl group containing 1 to 18 carbon atoms; R′ is an alkyl group containing 1 to 21 carbon atoms; A is a cyclic group selected from the group consisting of Pyr>, <Pyr, Pym>, FPhF, PhF, FPh and Ph; Z is -, #, O or OCO, and Z′ is O, COO, - or #; with the provisos that

1) if A is Pyr>,
   (a) Z is - or # and Z′ is O, or
   (b) Z is - and Z′ is COO;
2) if A is Pym>,
   (a) Z is - and Z′ is O, or
   (b) Z is O and Z′ is -, O or #;
3) if A is FPhF,
   (a) Z is - or O and Z′ is -, O or COO,
   (b) Z is OCO an Z′ is - or O, or
   (c) Z is # and Z′ is O;
4) if A is PhF,
   (a) Z is - or # and Z′ is O,
   (b) Z is O and Z′ is -, or
   (c) Z is - and Z′ is COO;
5) if A is FPh,
   (a) Z is O and Z′ is -, or
   (b) Z is -, O, # or OCO and Z′ is O, or
   (c) Z is O and Z′ is COO;
6) if A is <Pyr, Z is -, O or # and Z′ is O; and
7) if A is Ph, one of Z and Z′ is - and the other is O.

In the above and hereinafter, NAP, Pyr>, <Pyr, Pym>, FPhF, PhF, FPh, Ph, - and # represent, respectively, 2,6-naphthylene group,

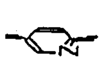 , 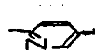 , 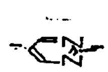 , 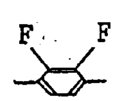 , 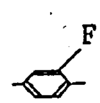 ,

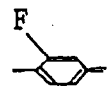 , 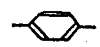

(1,4-phenylene group), a single bond (direct bond) and C≡C (C-C triple bond).

## DETAILED DESCRIPTION OF THE PREFERRED EMBODIMENTS

In the general formula (1), R and R′ are the same or different alkyl groups containing usually 1 - 18 carbon atoms, which may be saturated or unsaturated and may be substituted with at least one substituent selected from the group consisting of fluorine, chlorine, alkoxy groups containing 1 to 10 or more carbon atoms, trifluoromethyl group and cyano group.

Illustrative of suitable alkyl groups are straight-chain alkyl groups, branched alkyl groups, cyclic alkyl groups, F-substituted alkyl groups, Cl-substituted alkyl groups and alkoxy-substituted alkyl groups, as written in EP 0 385 692 as examples of R and R′; trifluoromethyl-substituted alkyl groups, such as 1-trifluoromethylpropyl, 1-trifluoromethylbutyl, 1-trifluoromethylpentyl, 1-trifluoromethylhexyl, 1-trifluoromethylheptyl, 1-trifluoromethyloctyl, 1-trifluoromethylnonyl, 1-trifluoromethyldecyl, 1-trifluoromethylundecyl, 1-trifluoromethyldodecyl, 1-trifluoromethyl-2-methoxyethyl, 1-trifluoromethyl-2-ethoxyethyl, 1-trifluoromethyl-2-propoxyethyl, 1-trifluoromethyl-2-butoxyethyl, 1-trifluoromethyl-2-pentyloxyethyl, 1-trifluoromethyl-2-hexyloxyethyl, 1-trifluoromethyl-2-isopropyloxyethyl, 1-trifluoromethyl-2-(1′-methylpropyloxy)ethyl, 1-trifluoromethyl-2-(1′-methylbutyloxy)ethyl, 1-trifluoromethyl -2-(1′-methylpentyloxy)ethyl, 1-trifluoromethyl-2-(1′-methylhexyloxy)ethyl, 1-trifluoromethyl-2-(1′-methylheptyloxy)ethyl, 1-trifluoromethyl-2-(2′-methylbutyloxy)ethyl, 1-trifluoromethyl-3-methoxypropyl, 1-trifluoromethyl-3-ethoxypropyl, 1-trifluoromethyl-3-propoxypropyl, 1-trifluoromethyl-3-butyloxypropyl, 1-trifluoromethyl-3-pentyloxypropyl, 1-trifluoromethyl-3-hexyloxypropyl, 1-trifluoromethyl-3-iso-propyloxypropyl, 1-trifluoromethyl-3-(1′-methylpropyloxy)propyl, 1-trifluoromethyl-3-(1′-methylbutyloxy)propyl, 1-trifluoromethyl-3-(1′-methylpentyloxy)propyl, 1-trifluoromethyl-3-(1′-methylhexyloxy)propyl, 1-trifluoromethyl-3-(1′-methylheptyloxy)propyl, 1-trifluoromethyl-3-(2′-methylbutyloxy)propyl, 1-trifluoromethyl-4-methoxybutyl, 1-trifluoromethyl-4-ethoxybutyl, 1-trifluoromethyl-4-propyloxybutyl, 1-trifluoromethyl-4-butyloxybutyl, 1-trifluoromethyl-4-pentyloxybutyl, 1-trifluoromethyl-4-hexyloxybutyl, 1-trifluoromethyl-4-iso-propyloxybutyl, 1-trifluoromethyl-4-(1′-methylpropyloxy)butyl, 1-trifluoromethyl-4-(1′-methylbutyloxy)butyl, 1-trifluoromethyl-4-(1′-methylpentyloxy)butyl, 1-trifluoromethyl-4-(1′-methylhexyloxy)butyl, 1-trifluoromethyl-4-(1′-methylheptyloxy)butyl, and 1-trifluoromethyl-4-(2′-methylbutyloxy)butyl; fluoro-substituted alkyl groups, such as 2-fluoropropyl, 2-fluorobutyl, 2-fluoropentyl, 2-fluorohexyl, 2-fluoroheptyl, 2-fluorooctyl, 2-fluorononyl, 2-fluorodecyl, 2-fluoroundecyl and 2-fluorododecyl; cyano-substituted alkyl groups, such as 1-cyanopropyl, 1-cyanobutyl, 1-cyanopentyl, 1-cyanohexyl, 1-cyanoheptyl, 1-cyanooctyl, 1-cyanononyl, 1-cyanodecyl, 1-cyanoundecyl and 1-cyanododecyl; and unsaturated alkyl groups (alkenyl groups), such as 2-propenyl, 3-butenyl, 4-pentenyl, 5-hexenyl, 6-heptenyl, 7-octenyl, 8-nonenyl, 9-decenyl, 11-dodecenyl, 17-octadecenyl, trans-2-decenyl, trans-5-decenyl, cis-3-nonenyl, cis-6-nonenyl, trans-2-heptenyl, cis-2-hexenyl, trans-2-hexenyl, cis-3-hexenyl, cis-4-hexenyl, and the like.

Alkyl groups R and R′ may contain one or more asymmetric carbon atoms, and may be optically active (hereinafter referred to as oa). Illustrative of suitable oa alkyl groups are oa alkyl groups, F-substituted oa alkyl groups, Cl-substituted oa alkyl groups and alkoxy-substituted oa alkyl groups, as written in EP 0 385 692 as examples of R and R′; as well as oa ones among trifluoromethyl-substituted alkyl groups and cyano-substituted as mentioned hereinabove. Alkyl groups of R′ may be of the formula: $-(CH_2)_nC^*H(CH_3)R''$, wherein R″ is an alkyl group containing 2-10 (particularly 2-6) carbon atoms and n is an integer of 0-9 (particularly 0-5).

Among these, preferred are alkyl groups containing 1 to 16, particularly 1 to 14 carbon atoms.

Illustrative examples of the compounds represented by the formula (1) include those shown in Table 1 to Table 11. In these Tables and also hereinafter, Me, Et, PRO, BUT, PEN, HEX, HEP, OCT, NON, DEC, 2MB, 4MH, 1MH, 2FO, HEPE, OCTE, NONE and DECE represent methyl, ethyl, n-propyl, n-butyl, n-pentyl, n-hexyl, n-heptyl, n-octyl, n-nonyl, n-decyl, 2-methylbutyl[$-CH_2{}^*CH(Me)Et$], 4-methylhexyl[$-(CH_2)_3{}^*CH(Me)Et$], 1-methylheptyl[-*CH(Me)HEX], 2-fluorooctyl[$-CH_2{}^*CHF$-HEX], 6-heptenyl, 7-octenyl, 8-nonenyl and 9-decenyl, respectively.

Table 1. Formula (1) wherein Z is - and Z′ is O

| No. | R | A | R′ | No. | R | A | R′ | No. | R | A | R′ |
|---|---|---|---|---|---|---|---|---|---|---|---|
| 1 | HEP | Pym> | NON | 26 | HEP | PhF | NON | 51 | OCT | Pym> | DECE |
| 2 | HEP | Pym> | DEC | 27 | HEP | PhF | DEC | 52 | NON | Pym> | NONE |
| 3 | OCT | Pym> | OCT | 28 | OCT | PhF | HEP | 53 | DEC | Pym> | OCTE |
| 4 | OCT | Pym> | NON | 29 | OCT | PhF | OCT | 54 | OCT | Pyr> | DECE |
| 5 | OCT | Pym> | DEC | 30 | OCT | PhF | NON | 55 | NON | Pyr> | NONE |
| 6 | NON | Pym> | HEP | 31 | OCT | PhF | DEC | 56 | DEC | Pyr> | OCTE |
| 7 | NON | Pym> | OCT | 32 | NON | PhF | HEP | 57 | OCT | < Pyr | DECE |
| 8 | NON | Pym> | NON | 33 | NON | PhF | OCT | 58 | NON | < Pyr | NONE |
| 9 | DEC | Pym> | HEX | 34 | NON | PhF | NON | 59 | DEC | < Pyr | OCTE |
| 10 | DEC | Pym> | HEP | 35 | DEC | PhF | HEP | 60 | OCT | PhF | DECE |
| 11 | DEC | Pym> | OCT | 36 | DEC | PhF | OCT | 61 | NON | PhF | NONE |
| 12 | DEC | Pym> | NON | 37 | HEP | FPhF | NON | 62 | DEC | PhF | OCTE |
| 13 | HEP | Pyr> | NON | 38 | HEP | FPhF | DEC | 63 | OCT | FPhF | DECE |
| 14 | HEP | Pyr> | DEC | 39 | OCT | FPhF | HEP | 64 | NON | FPhF | NONE |
| 15 | OCT | Pyr> | HEP | 40 | OCT | FPhF | OCT | 65 | DEC | FPhF | OCTE |
| 16 | OCT | Pyr> | OCT | 41 | OCT | FPhF | NON | 66 | OCT | FPhF | DECE |
| 17 | OCT | Pyr> | NON | 42 | OCT | FPhF | DEC | 67 | NON | FPhF | NONE |
| 18 | OCT | Pyr> | DEC | 43 | NON | FPhF | HEP | 68 | DEC | FPhF | OCTE |
| 19 | NON | Pyr> | HEP | 44 | NON | FPhF | OCT | 69 | OCT | Ph | DEC |
| 20 | NON | Pyr> | OCT | 45 | NON | FPhF | NON | 70 | DEC | Ph | OCT |
| 21 | NON | Pyr> | NON | 46 | DEC | FPhF | HEP | 71 | OCT | Ph | DECE |
| 22 | DEC | Pyr> | HEP | 47 | DEC | FPhF | OCT | 72 | DEC | Pyr> | 2FO |
| 23 | DEC | Pyr> | OCT | 48 | DEC | FPhF | NON | 73 | DEC | PhF | 2FO |
| 24 | OCT | < Pyr | OCT | 49 | OCT | FPh | DEC | 74 | DEC | FPhF | 2FO |
| 25 | OCT | < Pyr | NON | 50 | DEC | FPh | DEC | 75 | DEC | FPh | 2FO |

Table 2. Formula (1) wherein Z is -, Z′ is O, and R′ is $-(CH_2)_nC{*}H(Me)R''$

| wherein A is Pyr> | | | | wherein A is PhF | | | | wherein A is FPhF | | | |
|---|---|---|---|---|---|---|---|---|---|---|---|
| No. | R | n | R″ | No. | R | n | R″ | No. | R | n | R″ |
| 101 | DEC | 1 | Et | 121 | DEC | 1 | Et | 141 | DEC | 1 | Et |
| 102 | DEC | 3 | Et | 122 | DEC | 3 | Et | 142 | DEC | 3 | Et |
| 103 | DEC | 5 | Et | 123 | DEC | 5 | Et | 143 | DEC | 5 | Et |
| 104 | DEC | 0 | HEX | 124 | DEC | 0 | HEX | 144 | DEC | 0 | HEX |
| 105 | DEC | 1 | HEX | 125 | DEC | 1 | HEX | 145 | DEC | 1 | HEX |
| 106 | NON | 1 | Et | 126 | NON | 1 | Et | 146 | NON | 1 | Et |
| 107 | NON | 3 | Et | 127 | NON | 3 | Et | 147 | NON | 3 | Et |
| 108 | NON | 5 | Et | 128 | NON | 5 | Et | 148 | NON | 5 | Et |
| 109 | NON | 0 | HEX | 129 | NON | 0 | HEX | 149 | NON | 0 | HEX |
| 110 | NON | 1 | HEX | 130 | NON | 1 | HEX | 150 | NON | 1 | HEX |
| 111 | OCT | 1 | Et | 131 | OCT | 1 | Et | 151 | OCT | 1 | Et |
| 112 | OCT | 3 | Et | 132 | OCT | 3 | Et | 152 | OCT | 3 | Et |
| 113 | OCT | 5 | Et | 133 | OCT | 5 | Et | 153 | OCT | 5 | Et |
| 114 | OCT | 0 | HEX | 134 | OCT | 0 | HEX | 154 | OCT | 0 | HEX |
| 115 | OCT | 1 | HEX | 135 | OCT | 1 | HEX | 155 | OCT | 1 | HEX |
| 116 | UND | 1 | Et | 136 | UND | 1 | Et | 156 | UND | 1 | Et |
| 117 | UND | 3 | Et | 137 | UND | 3 | Et | 157 | UND | 3 | Et |
| 118 | UND | 5 | Et | 138 | UND | 5 | Et | 158 | UND | 5 | Et |
| 119 | UND | 0 | HEX | 139 | UND | 0 | HEX | 159 | UND | 0 | HEX |
| 120 | UND | 1 | HEX | 140 | UND | 1 | HEX | 160 | UND | 1 | HEX |

Table 3. Formula (1) wherein Z is O and Z′ is -

| No. | R | A | R′ | No. | R | A | R′ | No. | R | A | R′ |
|---|---|---|---|---|---|---|---|---|---|---|---|
| 201 | HEP | FPhF | NON | 222 | DECE | FPhF | OCT | 243 | DECE | FPh | HEP |
| 202 | HEP | FPhF | DEC | 223 | HEP | FPh | NON | 244 | DECE | FPh | OCT |
| 203 | OCT | FPhF | HEP | 224 | HEP | FPh | DEC | 245 | NON | PhF | OCT |
| 204 | OCT | FPhF | OCT | 225 | OCT | FPh | HEP | 246 | NON | PhF | NON |
| 205 | OCT | FPhF | NON | 226 | OCT | FPh | OCT | 247 | DECE | PhF | OCT |
| 206 | OCT | FPhF | DEC | 227 | OCT | FPh | NON | 248 | HEP | Pym> | DEC |
| 207 | NON | FPhF | HEP | 228 | OCT | FPh | DEC | 249 | OCT | Pym> | DEC |
| 208 | NON | FPhF | OCT | 229 | NON | FPh | HEP | 250 | DECE | Pym> | OCT |
| 209 | NON | FPhF | NON | 230 | NON | FPh | OCT | 251 | DEC | Ph | HEP |
| 210 | DEC | FPhF | HEP | 231 | NON | FPh | NON | 252 | DEC | Ph | OCT |
| 211 | DEC | FPhF | OCT | 232 | DEC | FPh | HEP | 253 | DECE | Ph | OCT |
| 212 | HEPE | FPhF | NON | 233 | DEC | FPh | OCT | 254 | 2MB | FPhF | DEC |
| 213 | HEPE | FPhF | DEC | 234 | DECE | FPh | NON | 255 | 1MH | FPhF | DEC |
| 214 | OCTE | FPhF | HEP | 235 | HEPE | FPh | DEC | 256 | 2FO | FPhF | DEC |
| 215 | OCTE | FPhF | OCT | 236 | OCTE | FPh | HEP | 257 | 2MB | FPh | DEC |
| 216 | OCTE | FPhF | NON | 237 | OCTE | FPh | OCT | 258 | 1MH | FPh | DEC |
| 217 | OCTE | FPhF | DEC | 238 | OCTE | FPh | NON | 259 | 2FO | FPh | DEC |
| 218 | NONE | FPhF | HEP | 239 | OCTE | FPh | DEC | 260 | 2FO | PhF | DEC |
| 219 | NONE | FPhF | OCT | 240 | NONE | FPh | HEP | 261 | 2FO | Ph | DEC |
| 220 | NONE | FPhF | NON | 241 | NONE | FPh | OCT | | | | |
| 221 | DECE | FPhF | HEP | 242 | NONE | FPh | NON | | | | |

Table 4. Formula (1) wherein Z is O and Z' is O

| No. | R | A | R' | No. | R | A | R' | No. | R | A | R' |
|---|---|---|---|---|---|---|---|---|---|---|---|
| 301 | HEP | FPhF | NON | 326 | OCT | FPhF | OCTE | 351 | OCTE | FPh | DEC |
| 302 | HEP | FPhF | DEC | 327 | OCT | FPhF | NONE | 352 | NONE | FPh | HEP |
| 303 | OCT | FPhF | HEP | 328 | OCT | FPhF | DECE | 353 | NONE | FPh | OCT |
| 304 | OCT | FPhF | OCT | 329 | NON | FPhF | HEPE | 354 | NONE | FPh | NON |
| 305 | OCT | FPhF | NON | 330 | NON | FPhF | OCTE | 355 | DECE | FPh | HEP |
| 306 | OCT | FPhF | DEC | 331 | NON | FPhF | NONE | 356 | DECE | FPh | OCT |
| 307 | NON | FPhF | HEP | 332 | DEC | FPhF | HEPE | 357 | HEP | FPh | NONE |
| 308 | NON | FPhF | OCT | 333 | DEC | FPhF | OCTE | 358 | HEP | FPh | DECE |
| 309 | NON | FPhF | NON | 334 | HEP | FPh | NON | 359 | OCT | FPh | HEPE |
| 310 | DEC | FPhF | HEP | 335 | HEP | FPh | DEC | 360 | OCT | FPh | OCTE |
| 311 | DEC | FPhF | OCT | 336 | OCT | FPh | HEP | 361 | OCT | FPh | NONE |
| 312 | HEPE | FPhF | NON | 337 | OCT | FPh | OCT | 362 | OCT | FPh | DECE |
| 313 | HEPE | FPhF | DEC | 338 | OCT | FPh | NON | 363 | NON | FPh | HEPE |
| 314 | OCTE | FPhF | HEP | 339 | OCT | FPh | DEC | 364 | NON | FPh | OCTE |
| 315 | OCTE | FPhF | OCT | 340 | NON | FPh | HEP | 365 | NON | FPh | NONE |
| 316 | OCTE | FPhF | NON | 341 | NON | FPh | OCT | 366 | DEC | FPh | HEPE |
| 317 | OCTE | FPhF | DEC | 342 | NON | FPh | NON | 367 | DEC | FPh | OCTE |
| 318 | NONE | FPhF | HEP | 343 | DEC | FPh | HEP | 368 | OCTE | FPhF | OCTE |
| 319 | NONE | FPhF | OCT | 344 | DEC | FPh | OCT | 369 | OCTE | FPhF | NONE |
| 320 | NONE | FPhF | NON | 345 | HEPE | FPh | NON | 370 | OCTE | FPhF | DECE |
| 321 | DECE | FPhF | HEP | 346 | HEPE | FPh | DEC | 371 | NONE | FPhF | OCTE |
| 322 | DECE | FPhF | OCT | 347 | OCTE | FPh | HEP | 372 | NONE | FPhF | NONE |
| 323 | HEP | FPhF | NONE | 348 | OCTE | FPh | OCT | 373 | DECE | FPhF | OCTE |
| 324 | HEP | FPhF | DECE | 349 | OCTE | FPh | NON | 374 | DECE | FPh | OCTE |
| 325 | OCT | FPhF | HEPE | 350 | OCTE | FPh | DEC | 375 | OCTE | FPh | DECE |

Table 4. (continued)

| No. | R | A | R' | No. | R | A | R' | No. | R | A | R' |
|---|---|---|---|---|---|---|---|---|---|---|---|
| 376 | OCT | Pym> | OCT | 391 | DEC | FPhF | 2MB | 406 | 2FO | FPh | DEC |
| 377 | OCT | Pym> | NON | 392 | DEC | FPhF | 4MH | 407 | 1MH | <Pyr | DEC |
| 378 | OCT | Pym> | DEC | 393 | DEC | FPhF | 1MH | 408 | 2FO | <Pyr | DEC |
| 379 | NON | Pym> | OCT | 394 | DEC | FPhF | 2FO | 409 | DEC | <Pyr | MOE |
| 380 | NON | Pym> | NON | 395 | 2MB | FPhF | DEC | 410 | DEC | <Pyr | 2FO |
| 381 | DEC | Pym> | HEP | 396 | 4MH | FPhF | DEC | 411 | DECE | FPhF | 1MH |
| 382 | DEC | Pym> | OCT | 397 | 1MH | FPhF | DEC | 412 | DECE | FPhF | 2FO |
| 383 | NON | Pym> | OCTE | 398 | 2FO | FPhF | DEC | 413 | 1MH | FPhF | DECE |
| 384 | NON | Pym> | NONE | 399 | OCT | FPh | 2MB | 414 | 2FO | FPhF | DECE |
| 385 | NON | <Pyr | OCT | 400 | DEC | FPh | 4MH | 415 | DECE | FPh | 1MH |
| 386 | NON | <Pyr | NON | 401 | OCT | FPh | 1MH | 416 | DECE | FPh | 2FO |
| 387 | DEC | <Pyr | HEP | 402 | DEC | FPh | 2FO | 417 | 1MH | FPh | DECE |
| 388 | DEC | <Pyr | OCT | 403 | 2MB | FPh | DEC | 418 | 2FO | FPh | DECE |
| 389 | DEC | <Pyr | OCTE | 404 | 4NH | FPh | DEC | 419 | DECE | <Pyr | 2FO |
| 390 | DEC | <Pyr | NONE | 405 | 1MH | FPh | DEC | 420 | 2FO | <Pyr | DECE |

Table 5. Formula (1) wherein Z is # and Z′ is O

| No. | R | A | R′ | No. | R | A | R′ | No. | R | A | R′ |
|---|---|---|---|---|---|---|---|---|---|---|---|
| 501 | HEX | Pyr> | OCT | 526 | OCT | PhF | HEP | 551 | OCT | Pyr> | 2MB |
| 502 | HEX | Pyr> | NON | 527 | OCT | PhF | OCT | 552 | OCT | Pyr> | 1MH |
| 503 | HEX | Pyr> | DEC | 528 | HEX | PhF | DECE | 553 | OCT | Pyr> | 2FO |
| 504 | HEP | Pyr> | OCT | 529 | HEP | PhF | NONE | 554 | OCT | FPhF | 2MB |
| 505 | HEP | Pyr> | NON | 530 | OCT | PhF | OCTE | 555 | OCT | FPhF | 1MH |
| 506 | OCT | Pyr> | HEP | 531 | HEX | FPh | OCT | 556 | OCT | FPhF | 2FO |
| 507 | OCT | Pyr> | OCT | 532 | HEX | FPh | NON | 557 | OCT | PhF | 2MB |
| 508 | HEX | Pyr> | DECE | 533 | HEX | FPh | DEC | 558 | OCT | PhF | 1MH |
| 509 | HEP | Pyr> | NONE | 534 | HEP | FPh | OCT | 559 | OCT | PhF | 2FO |
| 510 | OCT | Pyr> | OCTE | 535 | HEP | FPh | NON | 560 | OCT | FPh | 2MB |
| 511 | HEX | FPhF | OCT | 536 | OCT | FPh | HEP | 561 | OCT | FPh | 1MH |
| 512 | HEX | FPhF | NON | 537 | OCT | FPh | OCT | 562 | OCT | FPh | 2FO |
| 513 | HEX | FPhF | DEC | 538 | HEX | FPh | DECE | 563 | OCT | < Pyr | 2MB |
| 514 | HEP | FPhF | OCT | 539 | HEP | FPh | NONE | 564 | OCT | < Pyr | 1MH |
| 515 | HEP | FPhF | NON | 540 | OCT | FPh | OCTE | 565 | OCT | < Pyr | 2FOE |
| 516 | OCT | FPhF | HEP | 541 | HEX | < Pyr | OCT | | | | |
| 517 | OCT | FPhF | OCT | 542 | HEX | < Pyr | NON | | | | |
| 518 | HEX | FPhF | DECE | 543 | HEX | < Pyr | DEC | | | | |
| 519 | HEP | FPhF | NONE | 544 | HEP | < Pyr | OCT | | | | |
| 520 | OCT | FPhF | OCTE | 545 | HEP | < Pyr | NON | | | | |
| 521 | HEX | FPhF | OCT | 546 | OCT | < Pyr | HEP | | | | |
| 522 | HEX | FPhF | NON | 547 | OCT | < Pyr | OCT | | | | |
| 523 | HEX | FPhF | DEC | 548 | HEX | < Pyr | DECE | | | | |
| 524 | HEP | FPhF | OCT | 549 | HEP | < Pyr | NONE | | | | |
| 525 | HEP | FPhF | NON | 550 | OCT | < Pyr | OCTE | | | | |

Table 6. Formula (1) wherein Z is O and Z′ is #

| No. | R | A | R′ | No. | R | A | R′ | No. | R | A | R′ |
|---|---|---|---|---|---|---|---|---|---|---|---|
| 601 | HEP | Pym> | HEP | 605 | OCT | Pym> | OCT | 609 | DEC | Pym> | PEN |
| 602 | HEP | Pym> | OCT | 606 | NON | Pym> | PEN | 610 | DEC | Pym> | HEX |
| 603 | OCT | Pym> | HEX | 607 | NON | Pym> | HEX | | | | |
| 604 | OCT | Pym> | HEP | 608 | NON | Pym> | HEP | | | | |

Table 7. Formula (1) wherein Z is - and Z′ is -

| No. | R | A | R′ | No. | R | A | R′ | No. | R | A | R′ |
|---|---|---|---|---|---|---|---|---|---|---|---|
| 651 | HEP | Pym> | NON | 655 | OCT | Pym> | DEC | 659 | DEC | Pym> | HEP |
| 652 | HEP | Pym> | DEC | 656 | NON | Pym> | HEP | 660 | DEC | Pym> | OCT |
| 653 | OCT | Pym> | OCT | 657 | NON | Pym> | OCT | | | | |
| 654 | OCT | Pym> | NON | 658 | NON | Pym> | NON | | | | |

Table 8. Formula (1) wherein Z is O and Z′ is COO

| No. | R | A | R′ | No. | R | A | R′ | No. | R | A | R′ |
|---|---|---|---|---|---|---|---|---|---|---|---|
| 701 | OCT | FPhF | OCT | 713 | DECE | FPhF | OCT | 725 | DEC | FPh | OCT |
| 702 | OCT | FPhF | NON | 714 | DEC | FPhF | 2MB | 726 | OCT | FPh | DECE |
| 703 | OCT | FPhF | DEC | 715 | DEC | FPhF | 1MH | 727 | NON | FPh | NONE |
| 704 | NON | FPhF | OCT | 716 | DEC | FPhF | 2FO | 728 | DEC | FPh | OCTE |
| 705 | NON | FPhF | NON | 717 | DECE | FPhF | 1MH | 729 | NONE | FPh | NON |
| 706 | DEC | FPhF | HEP | 718 | DECE | FPhF | 2FO | 730 | DECE | FPh | OCT |
| 707 | DEC | FPhF | OCT | 719 | OCT | FPh | OCT | 731 | DEC | FPh | 2MB |
| 708 | OCT | FPhF | DECE | 720 | OCT | FPh | NON | 732 | DEC | FPh | 1MH |
| 709 | NON | FPhF | NONE | 721 | OCT | FPh | DEC | 733 | DEC | FPh | 2FO |
| 710 | DEC | FPhF | OCTE | 722 | NON | FPh | OCT | 734 | DECE | FPh | 1MH |
| 711 | OCTE | FPhF | DEC | 723 | NON | FPh | NON | 735 | DECE | FPh | 2FO |
| 712 | NONE | FPhF | NON | 724 | DEC | FPh | HEP | | | | |

Table 9. Formula (1) wherein Z is OCO and Z′ is O

| No. | R | A | R′ | No. | R | A | R′ | No. | R | A | R′ |
|---|---|---|---|---|---|---|---|---|---|---|---|
| 751 | OCT | FPhF | OCT | 763 | OCT | FPhF | DECE | 775 | HEP | FPh | DEC |
| 752 | NON | FPhF | OCT | 764 | 4MH | FPhF | DEC | 776 | OCT | FPh | DEC |
| 753 | DEC | FPhF | OCT | 765 | 2MB | FPhF | DEC | 777 | DECE | FPh | OCT |
| 754 | OCT | FPhF | NON | 766 | 1MH | FPhF | DEC | 778 | NONE | FPh | NON |
| 755 | NON | FPhF | NON | 767 | 2FO | FPhF | DEC | 779 | OCTE | FPh | DEC |
| 756 | HEP | FPhF | DEC | 768 | 1MH | FPhF | DECE | 780 | DEC | FPh | OCTE |
| 757 | OCT | FPhF | DEC | 769 | 2FO | FPhF | DECE | 781 | NON | FPh | NONE |
| 758 | DECE | FPhF | OCT | 770 | OCT | FPh | OCT | 782 | OCT | FPh | DECE |
| 759 | NONE | FPhF | NON | 771 | NON | FPh | OCT | 783 | 2MB | FPh | DEC |
| 760 | OCTE | FPhF | DEC | 772 | DEC | FPh | OCT | 784 | 1MH | FPh | DEC |
| 761 | DEC | FPhF | OCTE | 773 | OCT | FPh | NON | 785 | 2FO | FPh | DEC |
| 762 | NON | FPhF | NON | 774 | NON | FPh | NON | 786 | 1MH | FPhF | DECE |
| | | | | | | | | 787 | 2FO | FPhF | DECE |

Table 10. Formula (1) wherein Z is - and Z′ is COO

| No. | R | A | R′ | No. | R | A | R′ | No. | R | A | R′ |
|---|---|---|---|---|---|---|---|---|---|---|---|
| 801 | OCT | Pyr> | OCT | 815 | NON | FPhF | 1MH | 829 | NON | PhF | OCT |
| 802 | OCT | Pyr> | NON | 816 | DEC | FPhF | 2FO | 830 | OCT | PhF | DECE |
| 803 | OCT | Pyr> | DEC | 817 | DEC | FPhF | 1MH | 831 | DEC | Pyr> | NONE |
| 804 | NON | Pyr> | OCT | 818 | OCT | FPhF | 2FO | 832 | DEC | Pyr> | OCTE |
| 805 | NON | Pyr> | NON | 819 | NON | FPhF | NON | 833 | DEC | Pyr> | 2FO |
| 806 | DEC | Pyr> | HEP | 820 | DEC | FPhF | OCT | 834 | DEC | PhF | 1MH |
| 807 | DEC | Pyr> | OCT | 821 | OCT | PhF | OCT | 835 | DEC | PhF | 2MB |
| 808 | OCT | Pyr> | DECE | 822 | OCT | PhF | NON | 836 | DEC | PhF | 1MH |
| 809 | NON | Pyr> | NONE | 823 | OCT | PhF | DEC | 837 | DEC | PhF | 2FO |
| 810 | DEC | Pyr> | OCTE | 824 | NON | PhF | OCT | 838 | DEC | FPhF | 1MH |
| 811 | OCT | FPhF | DEC | 825 | NON | PhF | NON | 839 | DEC | FPhF | 2FO |
| 812 | OCT | FPhF | NON | 826 | DEC | PhF | HEP | 840 | DEC | FPhF | 2FO |
| 813 | OCT | FPhF | OCT | 827 | DEC | PhF | HEP | | | | |
| 814 | NON | FPhF | 2MB | 828 | OCT | PhF | HEP | | | | |

| Table 11. Formula (1) whrein Z is OCO and Z' is - | | | | | | | | | | | |
|---|---|---|---|---|---|---|---|---|---|---|---|
| No. | R | A | R' | No. | R | A | R' | No. | R | A | R' |
| 901 | OCT | FPhF | OCT | 906 | HEP | FPhF | DEC | 911 | 2MB | FPhF | DEC |
| 902 | NON | FPhF | OCT | 907 | OCT | FPhF | DEC | 912 | 1MH | FPhF | DEC |
| 903 | DEC | FPhF | OCT | 908 | DECE | FPhF | OCT | 913 | 2FO | FPhF | DEC |
| 904 | OCT | FPhF | NON | 909 | NONE | FPhF | NON | | | | |
| 905 | NON | FPhF | NON | 910 | OCTE | FPhF | DEC | | | | |

Compounds of the present invention can be produced in accordance with the following reactions.

I. Compounds of the formula : R-A-NAP-OR' (I)

[1a] In case where A is Pym> :

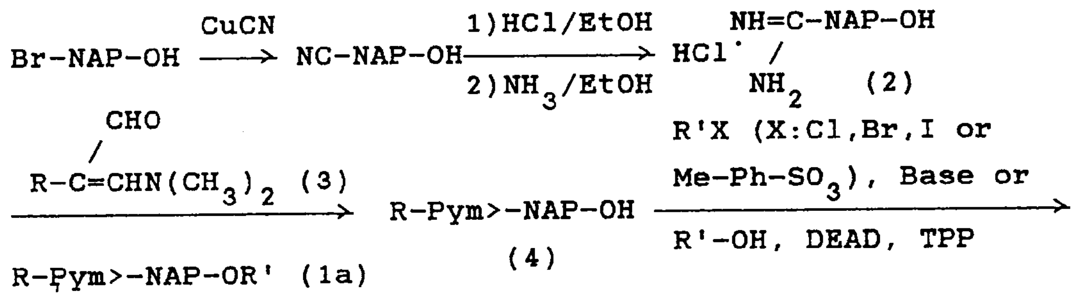


That is, 6-cyano-2-naphthol, obtained by reacting 6-bromo-2-naphthol with cuprous cyanide(I), is reacted within anhydrous ethanol with hydrogen chloride gas and then with ammonia gas to obtain an amidine salt (2). This amidine salt (2) is reacted with an acrolein (3) within ethanol in the presence of a base (such as sodium ethoxide) to obtain a compound (4). Compound (1a) of the present invention can be obtained by reacting the compound (4) with an alkyl halide in the presence of a base (such as sodium hydroxide), or with alcohol in the presence of diethyl azodiformate (DEAD) and triphenyl phosphine (TPP).

Some of compounds represented by the formula (3) as the raw material are commercially available, and some other can be produced as follows:

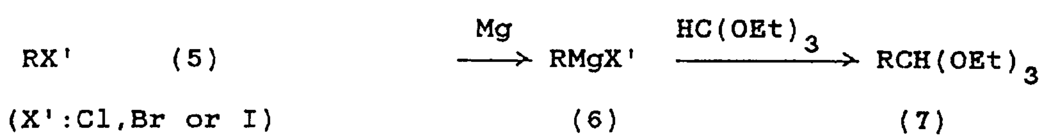


Grignard's reagent (6) prepared by reacting metallic magnesium with a compound (5) is reacted with ethyl orthoformate to obtain a compound (7), followed by reacting therewith Vilsmeier's reagent to obtain a compound (3) as the raw material.

[1b] In case where A is Pyr> :

$$\text{Br-NAP-OH} \xrightarrow[\text{Base}]{\text{R'X}} \underset{(8)}{\text{Br-NAP-OR'}} \xrightarrow{\text{Mg}} \underset{(9)}{\text{Br-Mg-NAP-OR'}} \xrightarrow[\text{[Pd]}]{\text{Br-Pyr>-Br}}$$

$$\underset{(10)}{\text{Br-Pyr>-NAP-OR'}} \xrightarrow[\text{[Pd]}]{\text{R"-\#-H (11)}} \underset{(12)}{\text{R"-\#-Pyr>-NAP-OR'}} \xrightarrow[\text{Pd/C}]{H_2}$$

$$\text{R-Pyr>-NAP-OR' (1b) [R:R"}CH_2CH_2\text{]}$$

A compound (8), obtained by etherifying 6-bromo-2-naphthol with an alkylating agent (such as alkyl halide), is reacted with metallic magnesium to prepare Grignard's reagent (9), followed by reacting therewith 2,5-dibromopyridine in the presence of palladium catalyst to obtain a compound (10). Compound (12) of the present invention can be obtained by reacting the compound (10) with 1-alkyne (11) in the presence of palladium catalyst. Compound (1b) of the present invention can be obtained by hydrogenating Compound (12) with using palladium carbon.

Compound (1b) may also be produced as follows from a compound (51) prepared as described bellow.

$$\text{(51)} \xrightarrow{\text{R'X,Base, or R'OH,DEAD,TPP}} \text{(1b)}$$

[1c] In case where A is <Pyr :

$$\text{Br-Mg-NAP-OR' (9)} \xrightarrow[\text{[Pd]}]{\text{R-<Pyr-I (13)}} \text{R-<Pyr-NAP-OR' (1c)}$$

Compound (1c) of the present invention can be obtained by reacting a compound (9) with a compound (13) in the presence of palladium catalyst.

Compound (13) as the raw material can be produced as follows:

$$\text{Cl-<Pyr-}NO_2 \xrightarrow[\text{[Pd]}]{\text{(11)}} \underset{(14)}{\text{R"-\#-<Pyr-}NO_2} \xrightarrow[\text{Pd/C}]{H_2} \underset{(15)}{\text{R-<Pyr-}NH_2} \xrightarrow[\text{KI}]{NaNO_2} \text{(13)}$$

Compound (14) can be obtained by reacting 2-chloro-5-nitropyridine with 1-alkyne (11) in the presence of palladium catalyst. Then the compound (14) is hydrogenated and reduced using palladium carbon into a compound (15). The compound (15) is diazotized with sodium nitrite, and then reacted with potassium iodide to obtain a compound (13) as the raw material.

Compound (1c) may be produced as follows from a compound (26) prepared as described bellow.

$$\text{(26)} \xrightarrow{\text{(13), [Pd]}} \text{(1c)}$$

Compound (1c) may also be produced as follows.

$$\text{Br-<Pyr-Br} \xrightarrow{\text{(11), [Pd]}} \text{R"-\#-<Pyr-Br} \xrightarrow{PtO_2\text{- }H_2}$$

$$\text{R-<Pyr-Br} \xrightarrow[\text{[Pd]}]{\text{(9) or (26)}} \text{(1c)}$$

[1d] In case where A is PhF :

$$\text{(9)} \xrightarrow[\text{[Pd]}]{\text{Br-PhF-I}} \text{Br-PhF-NAP-OR' (16)} \xrightarrow[\text{[Pd]}]{\text{(11)}} \text{R''-\#-PhF-NAP-OR' (17)}$$

$$\xrightarrow[\text{Pd/C}]{H_2} \text{R-PhF-NAP-OR' (1d) [R:R''}CH_2CH_2\text{]}$$

Grignard's reagent (9) is reacted with 1-bromo-3-fluoro-4-iodobenzene in the presence of palladium catalyst to obtain a compound (16). The compound (16) is reacted with 1-alkyne (11) in the presence of palladium catalyst to obtain a compound (17). Compound (1d) of the present invention can be obtained by hydrogenating Compound (17) with using palladium carbon.

[1e] In case where A is FPhF :

$$\text{H-Ph-}CH_2\text{O-FPhF-I} \xrightarrow[\text{[Pd]}]{\text{(9)}} \text{H-Ph-}CH_2\text{O-FPhF-NAP-OR' (18)} \xrightarrow[\text{Pd/C}]{H_2}$$

$$\text{HO-FPhF-NAP-OR' (19)} \xrightarrow{\text{TFMSA}} CF_3SO_2\text{-O-FPhF-NAP-OR' (20)}$$

$$\xrightarrow[\text{[Pd]}]{\text{(11)}} \text{R''-\#-FPhF-NAP-OR' (21)} \xrightarrow[\text{Pd/C}]{H_2} \text{R-FPhF-NAP-OR' (1e) [R:R''}CH_2CH_2\text{]}$$

Grignard's reagent (9) is reacted with 4-benzyloxy-2,3-difluoro-iodobenzene in the presence of palladium catalyst to obtain a compound (18). The compound (18) is hydrogenolyzed using palladium carbon into a compound (19), which is reacted with trifluolomethane sulfonic anhydride (TFMSA) to obtain a compound (20). The compound (20) is reacted with 1-alkyne (11) in the presence of palladium catalyst to obtain a compound (21). Compound (1e) of the present invention can be obtained by hydrogenating the compound (21) with using palladium carbon.

The raw material 4-benzyloxy-2,3-difluoro-iodobenzene can be produced by iodizing 2,3-difluorophenol with sodium hypochlorite and sodium iodide, followed by etherifying the product with benzyl chloride in the presence of a base.

$$\text{HO-FPhF-H} \xrightarrow[\text{NaI}]{\text{NaOCl}} \text{HO-FPhF-I} \xrightarrow[\text{Base}]{\text{H-Ph-}CH_2\text{Cl}} \text{H-Ph-}CH_2\text{O-FPhF-I}$$

Compound (1e) may be produced from a compound (57) prepared described bellow, by either of the followings:

$$\text{(57)} \xrightarrow{\text{(8), [Pd]}} \text{(1e); or}$$

$$\text{Br-NAP-OCO-Me} \xrightarrow{\text{(57), [Pd]}} \text{R-FPhF-NAP-OCO-Me} \xrightarrow[\text{H}^+]{\text{OH}^-}$$

$$\text{R-FPhF-NAP-OH} \xrightarrow{\text{R'X, Base or R'OH, DEAD,TPP}} \text{(1e)}$$

[1f] In case where A is FPh :

$$\text{I-FPh-Br} \xrightarrow[\text{[Pd]}]{\text{(11)}} \underset{\text{(22)}}{\text{R"-\#-FPh-Br}} \xrightarrow{\text{Mg}} \underset{\text{(23)}}{\text{R"-\#-FPh-MgBr}} \xrightarrow[\text{H}^+]{\text{B(OMe)}_3}$$

$$\underset{\text{(24)}}{\text{R"-\#-FPh-B(OH)}_2} \xrightarrow[\text{[Pd]}]{\text{(8)}} \underset{\text{(25)}}{\text{R"-\#-FPh-NAP-OR'}} \xrightarrow[\text{Pd/C}]{\text{H}_2} \underset{\text{(1f)}}{\text{R-FPh-NAP-OR'}}$$

Compound (1f) may also be produced as follows:

$$\text{(9)} \xrightarrow[\text{H}^+]{\text{B(OMe)}_3} \text{(HO)}_2\text{B-NAP-OR' (26)} \xrightarrow[\text{[Pd]}]{\text{(22)}} \text{(25)} \xrightarrow[\text{Pd/C}]{\text{H}_2} \text{(1f)}$$

The raw material (26) can be produced by reacting trimethyl borate with Grignard reagent prepared from a compound (8), followed by hydrolysis with an acid.

$$\text{(8)} \xrightarrow{\text{Mg, B(OMe)}_3\text{, H}^+} \text{(26)}$$

[1g] In case where A is Ph :

$$\text{R-Ph-X' (X':Br,I) (27)} \xrightarrow[\text{[Pd]}]{\text{(9) or (26)}} \text{R-Ph-NAP-OR' (1g)}$$

II. Compounds of the formula : RO-A-NAP-R′ (II)

[2a] In case where A is FPhF :

$$\text{Br-NAP-OH} \xrightarrow[\text{NaOH}]{\text{H-Ph-CH}_2\text{Cl}} \text{Br-NAP-OCH}_2\text{-Ph-H} \xrightarrow[\text{[Pd]}]{\text{(11)}}$$

$$\underset{\text{(28)}}{\text{R"-\#-NAP-OCH}_2\text{-Ph-H}} \xrightarrow[\text{Pd/C}]{\text{H}_2} \underset{\text{(29) [R':R"CH}_2\text{CH}_2\text{]}}{\text{R'-NAP-OH}} \xrightarrow{\text{TFMSA}} \underset{\text{(30)}}{\text{R'-NAP-OSO}_2\text{CF}_3}$$

$$\xrightarrow{\text{R-O-FPhF-B(OH)}_2\text{ (31)}} \text{R-O-FPhF-NAP-R' (2a)}$$

6-bromo-2-benzyloxynaphthalene, obtained by reacting 6-bromo-2-naphthol with benzyl chloride in the presence of a base, is reacted with 1-alkyne (11) in the presence of palladium catalyst to obtain a compound (28). Then the compound (28) is hydrogenated and hydrogenolyzed using palladium carbon into a compound (29). A compound (30) prepared by reacting the compound (29) with TFMSA is reacted with a compound (31) in the presence of palladium catalyst to obtain a compound (2a) of this invention.

Compound (31) as the raw material can be produced as follows:

$$\text{HO-FPhF-H} \xrightarrow[\text{ROH, DEAD, TPP}]{\text{RX, Base or}} \text{R-O-FPhF-H (32)} \xrightarrow[\text{B(OMe)}_3\text{, H}^+]{\text{n-BuLi}} \text{(31)}$$

A compound (32) prepared by alkylating 2,3-difluorophenol is reacted with n-butyl lithium at a temperature below -55°C, and then reacted with trimethyl borate, followed by hydrolyzying the product with an acid to obtain a compound (31) as the raw material.

Compound (2a) may be produced as follows :

$$\text{H-FPhF-H} \xrightarrow[\text{B(OMe)}_3\text{, H}^+]{\text{n-BuLi}} \text{H-FPhF-B(OH)}_2 \xrightarrow{\text{(30)}} \underset{\text{(33)}}{\text{H-FPhF-NAP-R'}}$$

$$\xrightarrow[\text{B(OMe)}_3\text{, H}^+]{\text{n-BuLi}} \underset{\text{(34)}}{\text{(HO)}_2\text{B-FPhF-NAP-R'}} \xrightarrow{H_2O_2} \underset{\text{(35)}}{\text{HO-FPhF-NAP-R'}}$$

$$\xrightarrow[\text{ROH, DEAD, TPP}]{\text{RX, Base or}} \text{(2a)}$$

2,3-difluorophenyl boric acid, prepared by reacting o-difluorobenzene with n-butyl lithium at a temperature below -55°C and then with trimethyl borate followed by hydrolysis with an acid, is reacted with a compound (30) in the presence of palladium catalyst to obtain a compound (33). A compound (34), prepared by reacting the compound (33) with n-butyl lithium at a temperature below -55°C and then with trimethyl borate followed by hydrolysis with an acid, is reacted with hydrogen peroxide to obtain a compound (35), which is further etherified to produce a compound (2a).

[2b] In case where A is Ph(F) [FPh, PhF of Ph] :

$$\text{HO-Ph(F)-Br (36)} \xrightarrow[\text{NaOH}]{\text{RX}} \text{R-O-Ph(F)-Br (37)} \xrightarrow[\text{B(OMe)}_3\text{, H}^+]{\text{Mg}}$$

$$\text{R-O-Ph(F)-B(OH)}_2\text{ (38)} \xrightarrow{\text{(30)}} \text{R-O-Ph(F)-NAP-R' (2b)}$$

A compound (37) prepared by alkylating a compound (36) is reacted with metallic magnesium to prepare Grignard's reagent, followed by reacting therewith trimethyl borate and then hydrolysis with an acid to obtain a compound (38). Compound (2b) of the present invention can be obtained by reacting the compound (38) with a compound (30) in the presence of palladium catalyst.

[2c] In case where A is Pym> :

$$\begin{matrix} \text{CHO} \\ / \\ \text{RO-C=CHN(CH}_3)_2 \end{matrix} \;(39) \xrightarrow{(2)} \text{RO-Pym>-NAP-OH}\;(40) \xrightarrow{\text{TFMSA}}$$

$$\text{RO-Pym>-NAP-OSO}_2\text{CF}_3\;(41) \xrightarrow[\text{[Pd]}]{(11)} \text{RO-Pym>-NAP-\#-R''}\;(42)$$

$$\xrightarrow{\text{H}_2\text{,Pd/C}} \text{RO-Pym>-NAP-R'}\;(2c)\;\;[\text{R'}:\text{R''CH}_2\text{CH}_2]$$

An amidine salt (2) prepared as described above is reacted with an acrolein (39) within ethanol in the presence of a base (such as sodium ethoxide) to obtain a compound (40). A compound (41) prepared by reacting the compound (40) with TFMSA is reacted with 1-alkyne (11) in the presence of palladium catalyst to obtain a compound (42), which is further hydrogenated with palladium carbon to produce Compound (2c) of the present invention.

III. Compounds of the formula : RO-A-NAP-OR′ (III)

[3a] In case where A is FPhF :

$$\text{Br-NAP-OH} \xrightarrow[\text{R'OH,DEAD,TPP}]{\text{R'X,Base or}} \underset{(43)}{\text{Br-NAP-OR'}} \xrightarrow[\text{[Pd]}]{(31)} \underset{(3a)}{\text{RO-FPhF-NAP-OR'}}$$

A compound (43) prepared reacting 6-bromo-2-naphthol with an alkylating agent R′X (such as alkyl halide) in the presence of a base (such as NaOH) is reacted with a compound (31) prepared as described above in the presence of palladium catalyst to produce Compound (3a) of the present invention.

Compound (3a) may be produced as follows:

$$(19) \xrightarrow{\text{R'X,Base, or ROH,DEAD,TPP}} (3a)$$

The compound (19) prepared as above is etherified with R′X using a base (such as NaOH), or with an alcohol ROH in the presence of DEAD and TPP to obtain (3a).

Compound (3a) may be produced as follows :

$$(31) \xrightarrow[\text{[Pd]}]{\text{Br-NAP-OCOMe}} \text{RO-FPhF-NAP-OCOMe} \xrightarrow[\text{H}^+]{\text{OH}^-}$$

$$\text{RO-FPhF-NAP-OH} \xrightarrow[\text{R'OH,DEAD,TPP}]{\text{R'X,Base or}} (3a)$$

[3b] In case where A is FPh :

$$(38) \xrightarrow[\text{[Pd]}]{(8)} \text{RO-FPh-NAP-OR'}\;(3b)$$

Compound (3b) of the present invention can be produced by reacting a compound (38) with a compound (8) in the presence of palladium catalyst.

Compound (3b) may be produced as follows:

$$\text{Br-NAP-OCOMe} \xrightarrow[\text{[Pd]}]{(38)} \text{RO-FPh-NAP-OCOMe} \xrightarrow[H^+]{OH^-} \text{RO-FPh-NAP-OH}$$

$$\xrightarrow[\text{R'OH, DEAD, TPP}]{\text{R'X, Base, or}} (3b); \quad (26) \xrightarrow[\text{[Pd]}]{\text{RO-FPh-Br}} (3b); \text{ or}$$

$$(26) \xrightarrow[\text{[Pd]}]{\text{MeCOO-FPh-Br}} \text{MeCOO-FPh-NAP-OR'} \xrightarrow[H^+]{OH^-}$$

$$\text{HO-FPh-NAP-OR'} \xrightarrow{\text{RX, Base, or ROH, DEAD, TPP}} (3b)$$

[3c] In case where A is Pym> :

$$(40) \xrightarrow{\text{R'X, Base, or R'OH, DEAD, TPP}} \text{RO-Pym>-NAP-OR'} \quad (3c)$$

Compound (3c) of the invention can be produced by etherifying a compound (40).

[3d] In case where A is <Pyr :

$$\underset{(44)}{\text{RONa}} \xrightarrow{\text{Br-<Pyr-Br}} \underset{(45)}{\text{RO-<Pyr-Br}} \xrightarrow[\text{[Pd]}]{(26)\text{ or }(9)} \underset{(3d)}{\text{RO-<Pyr-NAP-OR'}}$$

Compound (3d) of the invention can be produced by reacting a compound (26) or (9) in the presence of palladium catalyst with a compound (45) prepared by reacting a compound (44) with 2,5-dibromopyridine.

IV. Compounds of the formula : R-#-A-NAP-OR′ (IV)

[4a] In case where A is FPhF :

$$\text{H-FPhF-H} \xrightarrow[I_2]{\text{n-Bu-Li}} \text{I-FPhF-H} \xrightarrow[\text{[Pd]}]{\text{R-\#-H (11')}} \text{R-\#-FPhF-H} \quad (47)$$

$$\xrightarrow[\text{B(OMe)}_3\text{, }H^+]{\text{n-Bu-Li}} \underset{(48)}{\text{R-\#-FPhF-B(OH)}_2} \xrightarrow[\text{[Pd]}]{(8)} \text{R-\#-FPhF-NAP-OR'} \quad (4a)$$

[4b] In case where A is <Pyr :

$$\text{Br-<Pyr-Br} \xrightarrow[\text{[Pd]}]{(11')} \underset{(49)}{\text{R-\#-<Pyr-Br}} \xrightarrow[\text{[Pd]}]{(9)\text{ or }(26)} \underset{(4b)}{\text{R-\#-<Pyr-NAP-OR'}}$$

[4c] In case where A is Pyr>, PhF or FPh

The same as (12), (17) or (25), using (11′).

V. Compounds of the formula : RO-A-NAP-#-R′ (V)

The same as (42), using R′-#-H (11″).

VI. Compounds of the formula : R-A-NAP-COO-R′ (VI)

[6a] In case where A is Pyr>

$$\text{Br-NAP-OCH}_2\text{-Ph-H} \xrightarrow{\text{Mg}} \text{BrMg-NAP-OCH}_2\text{-Ph-H} \xrightarrow[\text{[Pd]}]{\text{Br-Pyr>-Br}}$$

$$\text{Br-Pyr>-NAP-OCH}_2\text{-Ph-H} \xrightarrow[\text{[Pd]}]{\text{(11)}} \text{R''-\#-Pyr>-NAP-OCH}_2\text{-Ph-H (50)}$$

$$\xrightarrow[\text{Pd/C}]{\text{H}_2} \text{R-Pyr>-NAP-OH (51)} \xrightarrow{\text{TFMSA}} \text{R-Pyr>-NAP-O-SO}_2\text{CF}_3 \text{ (52)}$$

$$\xrightarrow[\text{MeOH}]{\text{CO, [Pd]}} \text{R-Pyr>-NAP-COO-Me (53)} \xrightarrow[\text{H}^+]{\text{OH}^-} \text{R-Pyr>-NAP-COOH (54)}$$

$$\xrightarrow[\text{DEAD, TPP}]{\text{R'OH}} \text{R-Pyr>-NAP-COOR' (6a)}$$

[6b] In case where A is FPhF

$$\text{HO-NAP-COOR' (55)} \xrightarrow{\text{TFMSA}} \text{CF}_3\text{SO}_2\text{O-NAP-COOR' (56)}$$

$$\xrightarrow{\text{R-FPhF-B(OH)}_2 \text{ (57)}} \text{R-FPhF-NAP-COOR' (6b)}$$

The raw material (57) can be prepared as follows:

$$\text{H-FPhF-H} \xrightarrow[\text{R''CH}_2\text{-CHO}]{\text{n-BuLi}} \underset{\text{OH (58)}}{\text{R''CH}_2\text{-CH-FPhF-H}} \xrightarrow{\text{H}^+} \underset{\text{(59)}}{\text{R''CH=CH-FPhF-H}}$$

$$\xrightarrow[Pd/C]{H_2} \text{R-FPhF-H (R:R''CH}_2\text{CH}_2\text{) (60)} \xrightarrow[B(OMe)_3,\,H^+]{n\text{-BuLi}} (57)$$

Compound (6b) may also be produced as follows:

$$\text{HO-NAP-COOEt} \xrightarrow{TFMSA} \text{CF}_3\text{SO}_2\text{O-NAP-COOEt (61)} \xrightarrow{(57)}$$

$$\text{R-FPhF-NAP-COOEt (62)} \xrightarrow[H^+]{OH^-} \text{R-FPhF-NAP-COOH} \xrightarrow[DEAD,\,TPP]{R'OH} (6b)$$

[6c] In case where A is PhF

$$\text{BrMg-NAP-OCH}_2\text{-Ph-H} \xrightarrow[[Pd]]{Br\text{-}PhF\text{-}I} \text{Br-PhF-NAP-OCH}_2\text{-Ph-H} \xrightarrow[[Pd]]{(11)}$$

$$\text{R''-\#-PhF-NAP-OCH}_2\text{-Ph-H (63)} \xrightarrow[Pd/C]{H_2} \text{R-PhF-NAP-OH (64)} \xrightarrow{TFMSA}$$

$$\text{R-PhF-NAP-OSO}_2\text{CF}_3\text{ (65)} \xrightarrow[MeOH]{CO,\,[Pd]} \text{R-PhF-NAP-COO-Me (66)}$$

$$\xrightarrow[H^+]{OH^-} \text{R-PhF-NAP-COOH (67)} \xrightarrow[DEAD,\,TPP]{R'OH} \text{R-PhF-NAP-COOR' (6c)}$$

VII. Compounds of the formula: RO-FPhF-NAP-COO-R′ (VII)

$$\text{HO-FPhF-H} \xrightarrow[ROH,\,DEAD,\,TPP]{RX,\,Base\ or} \text{RO-FPhF-H (32)} \xrightarrow[B(OMe)_3,\,H^+]{n\text{-BuLi}}$$

$$(31) \xrightarrow[[Pd]]{(56)} (VII)$$

Compound (VII) may be produced as follows:

$$(61) \xrightarrow{(31),\ [Pd]} \text{RO-FPhF-NAP-COOEt (70)} \xrightarrow[H^+]{OH^-}$$

$$\text{RO-FPhF-NAP-COOH (71)} \xrightarrow[DEAD,\,TPP]{R'OH} (VII)$$

VIII. Compounds of the formula: R-FPhF-NAP-R′ (VIII)

$$(30) \xrightarrow{(57),\ [Pd]} (VIII)$$

IX. Compounds of the formula: R-OCO-FPhF-NAP-OR′ (IX)

$$\text{H-FPhF-H} \xrightarrow[B(OMe)_3\text{-}H^+]{n\text{-}BuLi} \text{H-FPhF-B(OH)}_2 \xrightarrow[[Pd]]{(8)} \text{H-FPhF-NAP-OR'}\ (72)$$

$$\xrightarrow[CO_2,\ H^+]{n\text{-}BuLi} \text{HOOC-FPhF-NAP-OR'}\ (73) \xrightarrow[DEAD,\ TPP]{ROH} (IX)$$

X. Compounds of the formula: R-OCO-FPhF-NAP-R′ (X)

$$\text{H-FPhF-B(OH)}_2 \xrightarrow{(30),\ [Pd]} \text{H-FPhF-NAP-R'}\ (74) \xrightarrow[CO_2,\ H^+]{n\text{-}BuLi}$$

$$\text{HOOC-FPhF-NAP-R'}\ (75) \xrightarrow[DEAD,\ TPP]{ROH} (X)$$

XI. Compounds of the formula: R-O-FPh-NAP-COO-R′ (XI)

$$(38) \xrightarrow{(56),\ [Pd]} (XI)\ ;\ or$$

$$(38) \xrightarrow{(61),\ [Pd]} \text{R-O-FPh-NAP-COO-Et} \xrightarrow[H^+]{OH^-}$$

$$\text{R-O-FPh-NAP-COOH} \xrightarrow{R'OH,\ DEAD,\ TPP} (XI)$$

XII. Compounds of the formula: R-OCO-FPh-NAP-O-R′ (XII)

$$\text{R-OCO-FPh-I} \xrightarrow{(26),\ [Pd]} (XII)\ ;\ or$$

$$\text{Et-OCO-FPh-I} \xrightarrow{(26),\ [Pd]} \text{Et-OCO-FPh-NAP-O-R'} \xrightarrow[H^+]{OH^-}$$

$$\text{HOOC-FPh-NAP-O-R'} \xrightarrow{ROH,\ DEAD,\ TPP} (XII)$$

In general, liquid crystals are used in the forms of liquid crystal compositions containing two or more components; and compounds of the present invention can be used as a component of liquid crystal compositions. Liquid crystal composition according to the present invention comprises a mixture of a plurality of compounds, comprising at least one compound of this invention. Liquid crystal compositions of the invention include [1] ones showing Sc phase, and [2] ones showing Sc* phase.

Liquid crystal compositions [1] of the present invention comprise at least one liquid crystal compound of

this invention as the essential component, and can optionally contain one or more other liquid crystal compounds, which may be selected from the group consisting of other liquid crystal compounds showing Sc phase, liquid crystal compounds showing smectic phase other than Sc or Sc* phase, and liquid crystal compounds showing nematic phase. These liquid crystal compounds include, for example, those written in EP 220,296. Illustrative of suitable liquid crystal compounds showing Sc phase are 2-4′-alkoxyphenyl-5-alkylpyrimidine, 2-4′-alkylphenyl-5-alkoxypyrimidine, 2-4′-alkoxyphenyl-5-alkoxypyrimidine, 2-4′-alkylphenyl-5-alkylpyrimidine, 2-p-alkoxycarbonylphenyl-5-alkylprimidine, 2-4′-alkoxy-3′-fluorophenyl-5-alkylpyrimidine, 2-4′-alkoxy-2′,3′-difluorophenyl-5-alkylpyrimidine, 2-4′-alkoxyphenyl-5-alkylpyridine, 2-4′-alkoxy-3′-fluorophenyl-5-alkylpyridine, 2-4′-alkoxy-2′,3′-difluorophenyl-5-alkylpyridine, 2-4′-alkylbiphenyl-5-alkylpyrimidine, 2-4′-alkoxybiphenyl-5-alkylpyrimidine, 2-4′-alkoxybiphenyl-5-alkoxypyrimidine, 2-4′-alkylbiphenyl-5-alkylpyrimidine, 2-4′-alkylphenyl-5-4′-alkoxyphenylpyrimidine, 2-4′-alkoxyphenyl-5-4′-alkoxyphenylpyrimidine, 2-4′-alkoxyphenyl-5-4′-alkylphenylpyrimidine, 2-4′-alkylphenyl-5-4′-alkoxyphenylpyrimidine, 1-5′-alkyl-2′-pyridyl-2-4′-alkoxyphenylacetylene, 1-2′-alkyl-5′-pyridyl-2-4′-alkoxyphenylacetylene, 1-2′-alkyl-5′-pyridyl-2-4′-alkoxy-3′-fluorophenylacetylene, 1-2′-alkyl-5′-pyridyl-2-4′-alkoxy-2′,3′-difluorophenylacetylene, 1-3′-alkyl-5′-pyridazyl-2-4′-alkoxy-3′-fluorophenylacetylene, and 1-3′-alkoxy-5′-pyridazyl-2-4′-alkoxy-3′-fluorophenylacetylene.

Liquid crystal compositions [2] of the present invention comprise at least one liquid crystal compound of this invention as the essential component and optionally one or more other liquid crystal compounds, and contain at least one optically active liquid crystal compound which may be the compound of this invention or/and the other liquid crystal compound. Liquid crystal compositions [2] include, for example, ones comprising said liquid crystal composition [1] and at least one optically active compound, such as liquid crystal compounds showing Sc* phase and chiral compounds. Illustrative examples of suitable other liquid crystal compounds include ones showing Sc* phase, for example, oa p′-(2-methylbutyloxycarbonyl)phenyl 4-alkoxy-4′-bi-phenylcarboxylate, oa 2-methylbutyl 4-alkoxy-4′-biphenyl-carboxylate; oa 2-(2-methylbutyloxycarbonyl)naphthalene-6-4′-n-alkoxyphenylcarboxylate, oa 2-(1-methylheptyloxy-carbonyl)naphthalene-6-4′-n-alkoxyphenylcarboxylate, oa 2-(2-methylbutyloxy)naphthalene-6-4′n-alkoxyphenylcarboxyl-ate and oa 2-(1-methylheptyloxy)naphthalene-6-4′-n-alkoxy-phenylcarboxylate; and other optically active compounds, for instance, those written in JPN Patent Lay-open Nos. 99032/1988, 190843/1988, 138274/ 1990 and 27374/1991, and EP Patents 374,789, 365,820 and 395,078 (corresponding to JPN Patent Lay-open Nos.256673/ 1990, 262579/1990 and 286673/1990, respectively). Illustrative of these compounds are oa 2-(1-cyanoethyl)naphthalene-6-4′-n-alkylphenylcarboxylate, 2-(1-cyanoethyl)naphthalene-6-4′-n-alkoxyphenylcarboxylate, oa 4-(1-trifluoromethylheptyloxy-methyl)-4′-n-alkoxy-biphenyl, oa 4-(1-trifluoromethylheptyl-oxymethyl)-4′-n-alkylbiphenyl, oa trans 4-(4′-n-alkoxy-phenyl)-1-(1′-trifluoromethylheptyloxycarbonyl)cyclo-hexane, and the like.

Liquid crystal compositions may contain other components, for example, chiral compounds showing no liquid crystaline properties, pleochroic dyes (such as anthraquinone dyes, azo dyes and the like), and so on.

Content of said liquid crystal compound of this invention in liquid crystal compositions of the invention, which is not particularly restricted and can be varied widely, is usually 5-100 % by weight (5-99% in case of compositions [2]). Content of pleochroic dye is usually 0-5 % by weight.

By applied voltage, ferroelectric liquid crystal compositions of this invention cause photo-switching phenomena, which are applied for producing display elements of quick response [See for example, JPN Patent Lay-open Nos.107216/1981 and 118744/1984, and N.A.Clark and S.T.Lagerwall:Applied Physics Letters, 36, 899(1980)].

Liquid crystal compositions, showing ferroelectricity, according to the invention, bring about optical switching phenomena by applied voltage, and can provide display devices, such as those described in JPN Lay-open Patents No.107216/1981 and No.118744/1984, and N.A.Clark and S.T.Lagerwall, "Applied Physics Letters" 36, 899(1980).

The liquid crystal compositions of this invention can be used as photo-switching elements (display devices), for instance, by sealing in vacuum into liquid crystal cells having cell distance of 0.5-10 (preferably 0.5-3) micron m, followed by providing polarizers on both sides.

The above liquid crystal cells can be produced by combining, through a spacer, two surface-aligned glass substrates provided with transparent electrodes. Examples of suitable spacers are alumina beads, glass fiber and polyimide film. Alignment can be done by conventional alignment treatment, such as application of polyimide membrane, rubbing treatment, oblique vapor-deposition of SiO.

Having generally described the invention, a more complete understanding can be obtained by reference to certain specific examples, which are included for purposes of illustration only and not intended to be limiting unless otherwise specified. In the following Examples, % represents % by weight.

Example 1 [Compound No.7]

(1) Into 80 ml of anhydrous dimethylformamide (hereinafter referred to as DMF), were dissolved 50 g of 6-bromo-2-naphthol and 24.1 g of cuprous cyanide, and heated under reflux for 6 hours. After cooling, an aqueous solution containing 100 g of ferric chloride and 25 g of 35 % hydrochloric acid dissolved in 200 ml of water was added and stirred for 20 minutes at 60°C. After cooling to the room temperature, the product was extracted twice with 300 ml of ether. The organic layer was washed with water, followed by removing the ether. The resulting solid was recrystalized with toluene to obtain 26.2 g of brown 6-cyano-2-naphthol.
(2) Into 200 ml of anhydrous ethanol, were dissolved 26.2 g of 6-cyano-2-naphthol and cooled to a temperature below 5°C on an ice bath. Under stirring, 57 g of hydrogen chloride gas was introduced at a temperature below 15°C, and stirring was continued for 2 hours on an ice bath and then for 4 hours at room temperature, followed by allowing to stand over night. Thereafter, ethanol was removed with an aspirator to obtain a brown solid, which was used as such without any purification for the following reaction.
(3) To this solid, under cooling on an ice bath, were added dropwise 200 ml of anhydrous ethanol saturated with ammonia gas, and stirring was continued for an hour in that condition and then for 2 hours at room temperature, followed by allowing to stand over night. Thereafter, ethanol was removed, and the resulting solid was washed with ethanol to obtain 22 g of brown solid of amidine hydrochloric acid salt.
(4) To an ethanol solution of sodium ethoxide prepared from 200 ml of anhydrous ethanol and 4.7 g of metallic sodium, were added 16.1 g of alpha-n-nonyl-beta-dimethylaminoacrolein and 15.0 g of the amidine hydrochloric acid salt prepared in (3), followed by heating under refluxing for 20 hours. After reaction, ethanol was removed, and water was added and the product was washed with ether, followed by adding 1 N-hydrochloric acid to the aqueous layer to acidify it. The resulting precipitates were filtered and dried, followed by recrystalizing with methylene chloride/ hexane to obtain 10.0 g of a compound (a) of the formula: NON-Pym>-NAP-OH.
(5) To 50 ml of dimethylsulfoxide (hereinafter referred to as DMSO) containing the compound (a) dissolved therein, was added a solution of 550 mg of sodium hydroxide dissolved in 5 ml of water, and stirred into a homogeneous solution. Then, 1.6 g of n-octyl bromide were added and stirred for 2 days at room temperature. Thereafter, the reaction product was thrown into 100 ml of iced water and extracted twice with 50 ml of toluene. The toluene layer was washed with water, and then treated with silica gel column to remove toluene. The resulting solid was recrystalized thrice with ethanol to obtain 1.2 g of white crystalline Compound No.7 of the present invention, the structure of which was confirmed by IR and NMR spectra and elemental analysis.

IR ($cm^{-1}$) 2928 2856 1626 1586 1545 1489 1470 1431 1390 1259 1203 846 816 793
NMR (ppm) 0.83-0.93(m,6H) 1.20-1.41(m,20H) 1.41-1.57 (m,2H) 1.57-1.73(m,2H) 1.80-1.90(m,2H) 2.61 (t,2H) 4.07(t,2H) 7.15-7.18(m,2H) 7.80(d,1H) 7.87(d,1H) 8.47(dd,1H) 8.63(s,2H) 8.85(d,1H)

| | | | | |
|---|---|---|---|---|
| Elemental analysis,% | Observed | C: 80.93 | H: 9.42 | N: 5.97 |
| | Theoretical | C: 80.87 | H: 9.57 | N: 6.09 |

Example 2 [Compound No.6]

Example 1 (5) was repeated except that n-octyl bromide was substituted with n-heptyl bromide to obtain Compound No.6.

Example 3 [Compound No.1]

(1) Example 1 (4) was repeated except that alpha-n-nonyl-beta-dimethylaminoacrolein was substituted with alpha-n-heptyl-beta-dimethylaminoacrolein to obtain a compound (b) of the formula: HEP-Pym>-NAP-OH.
(2) Example 1 (5) was repeated except that n-octyl bromide and the compound (a) were substituted with n-nonyl bromide and the compound (b), respectively, to obtain Compound No.1.

Example 4 [Compound No.2]

Example 3 (2) was repeated except that n-nonyl bromide was substituted with n-decyl bromide to obtain Compound No.2.

Example 5 [Compound No.3]

(1) Example 1 (4) was repeated except that alpha-n-nonyl -beta-dimethylaminoacrolein was substituted with alpha-n-octyl-beta-dimethylaminoacrolein to obtain a compound (c) of the formula: OCT-Pym>-NAP-OH.
(2) Example 1 (5) was repeated except that the compound (a) was substituted with the compound (c) to obtain Compound No.2.

Example 6 [Compound No.4]

Example 5 (2) was repeated except that n-octyl bromide was substituted with n-nonyl bromide to obtain Compound No.4.

Example 7 [Compound No.5]

Example 5 (2) was repeated except that n-octyl bromide was substituted with n-decyl bromide to obtain Compound No.5.

Example 8 [Compound No.10]

(1) Example 1 (4) was repeated except that alpha-n-nonyl -beta-dimethylaminoacrolein was substituted with alpha-n-decyl-beta-dimethylaminoacrolein to obtain a compound (d) of the formula: DEC-Pym>-NAP-OH.
(2) Example 1 (5) was repeated except that n-octyl bromide and the compound (a) were substituted with n-heptyl bromide and the compound (d), respectively, to obtain Compound No.10.

Example 9 [Compound No.11]

Example 8 (2) was repeated except that n-heptyl bromide was substituted with n-octyl bromide to obtain Compound No.11.

Example 10 [Compound No.12]

Example 8 (2) was repeated except that n-heptyl bromide was substituted with n-nonyl bromide to obtain Compound No.12.

Example 11 [Compound No.16]

(1) Into 250 ml of DMSO containing 25.0 g of 6-bromo-2-naphthol dissolved therein, was added 20 ml of an aqueous solution of 4.9 g of sodium hydroxide, and stirred into a homogeneous solution. Then, 22.7 g of n-octyl bromide were added and stirred for 3 days at room temperature. Thereafter, the reaction product was thrown into iced water, and the resulting precipitates were separated by suction filtration, and recrystalize with methanol to obtain 33.4 g of 2-n-octyloxy-6-bromonaphthalene.
(2) To a diethyl ether solution of a Grignard's reagent prepared from 10.0 g of 2-n-octyloxy-6-bromonaphthalene and 5.6 g of 1,2-dibromoethane, was added 7.1 g of 2,5-dibiomopyridine, and the atmosphere was substituted with nitrogen. Under cooling with ice bath to a temperature below 10°C, 180 mg of dichloro[1,4-bis(diphenylphosphino)butane] palladium(II) was added, and stirring was continued for an hour in that condition and then for 2 hours without ice bath. Subsequently, water was added under cooling with ice bath. After addition of toluene, the organic layer was washed with water, and then the solvent was removed. The resulting solid was recrystalized with ethanol to obtain 7.3 g of a compound (e) of the formula: Br-Pyr>-NAP-O-OCT.
(3) In 50 ml of triethylamine, 1.9 g of the compound (e) and 1.0 g of 1-octyne were heated to reflux for 6 hours within an atmosphere of nitrogen in the presence of 5 mg of cuprous iodide (I), 20 mg of dichlorobistriphenylphosphine palladium(II) and 40 mg of triphenylphosphine. After reaction, the temperature was reduced to the room temperature and the triethyl amine was removed. After the product was extracted with toluene, the toluene layer was washed with 1N-hydrochloric acid and then water, followed by treatment with silica gel column. After removing the toluene, 150 ml of ethanol and 200 mg of 5% palladium carbon were added to the resulting solid, and hydrogenation was carried out at reflux temperature. After comfirming

that no absorption of hydrogen was observed, the catalyst was filtered off, followed by cooling to the room temperature. The resulting crystal was recrystalized twice with ethanol to obtain 1.3 g of white crystalline Compound No.16 of the present invention.

IR ($cm^{-1}$) 2924 2856 1630 1605 1493 1648 1390 1255 1207 1174 1027 890 861 839 816

NMR (ppm) 0.80-0.98(m,6H) 1.20-1.45(m,18H) 1.45-1.58 (m,2H) 1.58-1.72(m,2H) 1.80-1.90(m,2H) 2.65 (t,2H) 4.08(t,2H) 7.15-7.20(m,2H) 7.59 (dd,1H) 7.74-7.88(m,3H) 8.09(dd,1H) 8.40(d,1H) 8.57(d,1H)

| | | | | |
|---|---|---|---|---|
| Elemental analysis, % | Observed | C: 83.38 | H: 9.80 | N: 3.31 |
| | Theoretical | C: 83.59 | H: 9.66 | N: 3.15 |

Example 12 [Compound No.20]

Example 11 (3) was repeated except that 1-octyne was substituted with 1-nonyne to obtain Compound No.20. Example 13 [Compound No.13]

(1) Example 11 (1) and (2) were repeated except that n-octyl bromide was substituted with n-nonyl bromide to obtain a compound (f) of the formula: Br-Pyr>-NAP-O-NON.

(2) Example 11 (3) was repeated except that the compound (e) and 1-octyne were substituted with the compound (f) and 1-heptyne, respectively, to obtain Compound No.13.

Example 14 [Compound No.17]

Example 13 (2) was repeated except that 1-heptyne was substituted with 1-octyne to obtain Compound No.17.

Example 15 [Compound No.21]

Example 13 (2) was repeated except that 1-heptyne was substituted with 1-nonyne to obtain Compound No.21.

Example 16 [Compound No.14]

(1) Example 11 (1) and (2) were repeated except that n-octyl bromide was substituted with n-decyl bromide to obtain a compound (g) of the formula: Br-Pyr>-NAP-O-DEC.

(2) Example 11 (3) was repeated except that the compound (e) and 1-octyne were substituted with the compound (g) and 1-heptyne, respectively, to obtain Compound No.14.

Example 17 [Compound No.18]

Example 16 (2) was repeated except that 1-heptyne was substituted with 1-octyne to obtain Compound No.18.

Example 18 [Compound No.15]

(1) Example 11 (1) and (2) were repeated except that n-octyl bromide was substituted with n-heptyl bromide to obtain a compound (h) of the formula: Br-Pyr>-NAP-O-HEP.

(2) Example 11 (3) was repeated except that the compound (e) was substituted with the compound (h) to obtain Compound No.15.

Example 19 [Compound No.19]

Example 18 (2) was repeated except that 1-octyne was substituted with 1-nonyne to obtain Compound No.19.

Example 20 [Compound No.22]

Example 18 (2) was repeated except that 1-octyne was substituted with 1-decyne to obtain Compound

No.22.

Example 21 [Compound No.24]

To a diethyl ether solution of a Grignard's reagent prepared from 4.4 g of 2-n-octyloxy-6-bromonaphthalene and 2.5 g of 1,2-dibromoethane, was added 2.8 g of 2-n-octyl-5-iodopyridine (which was prepared by reacting 1-octyne and 2-chloro-5-nitropyridine in the presence of palladium catalyst and then hydrogenating and reducing the reaction product within hydrogen atmosphere in the presence of palladium carbon to obtain 2-n-octyl-5-aminopyridine, followed by diazotizing it with sodium nitrite and reacting potassium iodide), and the atmosphere was substituted with nitrogen. Under cooling with ice bath to a temperature below 10°C, 53 mg of dichloro[1,4-bis(diphenylphosphino)butane] palladium(II) was added, and stirring was continued for an hour in that condition and then for 2 hours without ice bath. Subsequently, water was added under cooling with ice bath. After addition of toluene, the organic layer was washed with water, and then the solvent was removed. The product was dissolved again in toluene and treated with silica gel column, followed by removing the toluene. The resulting solid was recrystalized thrice with ethanol to obtain 0.9 g of white crystalline Compound No.24 of the present invention.

IR ($cm^{-1}$) 2924 2856 1628 1605 1562 1497 1468 1394 1255 1209 1174 888 851 820 472

NMR (ppm) 0.80-0.98(m,6H) 1.20-1.45(m,18H) 1.45-1.58 (m,2H) 1.58-1.72(m,2H) 1.80-1.90(m,2H) 2.65 (t,2H) 4.08(t,2H) 7.15-7.20(m,2H) 7.59 (dd,1H) 7.74-7.88(m,3H) 8.09(dd,1H) 8.40(d,1H) 8.57(d,1H)

| | | | | |
|---|---|---|---|---|
| Elemental analysis, % | Observed | C: 83.71 | H: 9.53 | N: 3.26 |
| | Theoretical | C: 83.59 | H: 9.66 | N: 3.15 |

Example 22 [Compound No.36]

(1) To a diethyl ether solution of a Grignard's reagent prepared from 8.0 g of 2-n-octyloxy-6-bromonaphthalene and 4.5 g of 1,2-dibromoethane, was added 7.2 g of 1-bromo-3-fluoro-4-iodobenzene, and the atmosphere was substituted with nitrogen. Under cooling with ice bath to a temperature below 10°C, 143 mg of dichloro[1,4-bis(diphenylphosphino)butane] palladium(II) was added, and stirring was continued for an hour in that condition and then for 5 hours without ice bath. Subsequently, water was added under cooling with ice bath. After addition of toluene, the organic layer was washed with water, and then the solvent was removed. The resulting solid was recrystalized with ethanol to obtain 5.4 g of a compound (i) of the formula: Br-PhF-NAP-O-OCT.

(2) In 50 ml of triethylamine, 2.7 g of the compound (i) and 1.3 g of 1-decyne were heated to reflux for 6 hours within an atmosphere of nitrogen in the presence of 6 mg of cuprous iodide (I), 24 mg of dichlorobistriphenylphosphine palladium(II) and 48 mg of triphenylphosphine. After reaction, the temperature was reduced to the room temperature and the triethyl amine was removed. After the product was extracted with toluene, the toluene layer was washed with 1N-hydrochloric acid and then water, followed by treatment with silica gel column. After removing the toluene, 150 ml of ethanol and 200 mg of 5% palladium carbon were added to the resulting solid, and hydrogenation was carried out at reflux temperature. After comfirming that no absorption of hydrogen was observed, the catalyst was filtered off, followed by cooling to the room temperature. The resulting crystal was recystalized twice with ethanol to obtain 1.6 g of white crystalline Compound No.36 of the present invention

IR ($cm^{-1}$) 2922 2854 1605 1497 1470 1394 1257 1205 855

NMR (ppm) 0.86-0.96(m,6H) 1.20-1.46(m,22H) 1.46-1.58 (m,2H) 1.58-1.72(m,2H) 1.82-1.92(m,2H) 2.75(t,2H) 4.10(t,2H) 6.98-7.09(m,2H) 7.12-7.20(m,2H) 7.40-7.47(m,1H) 7.61-7.68(m,1H) 7.78(d,2H) 7.92(s,1H)

$^{19}$F-NMR(ppm) -42.8(t,1F)

| | | | | |
|---|---|---|---|---|
| Elemental analysis, % | Observed | C: 83.50 | H: 9.41 | N: 3.92 |
| | Theoretical | C: 83.26 | H: 9.59 | N: 3.88 |

Example 23 [Compound No.45]

(1) To a solution of 10.0 g of 2,3-difluorophenol, 11.5 g of sodium iodide and 3.1 g of sodium hydroxide dissolved in 200 ml of methanol, was added dropwise 135 ml of 4% aqueous solution of sodium hypochlorite, followed by continuing stirring for 2 hours in that condition. After reaction, 80 ml of 10% aqueous solution of sodium thiosulfate were added to the product, fo!lowed by adding 4N-hydrochloric acid thereto to weakly acidifying it. Separated oil was extracted with chloroform, the organic layer was washed with water, and then the solvent was removed. The resulting oil was recrystalized with hexane to obtain 8.7 g of white solid of 2,3-difluoro-4-iodophenol.

(2) In 70 ml of DMSO containing 8.7 g of 2,3-difluoro-4-iodophenol dissolved therein, was added a solution of 1.4 g of sodium hydroxide dissolved in 5 ml of water, and stirred into a homogeneous solution. Then, 4.5 g of benzyl chloride were added and stirred for 3 days at room temperature. Thereafter, water was added to the reaction product, and the product was extracted with hexane. The hexane layer was washed with water, and the hexane was removed. The resulting solid was recrystalized with methanol to obtain 10.1 g of 2,3-difluoro-4-benzyloxyiodobenzene.

(3) To a diethyl ether solution of a Grignard's reagent prepared from 5.0 g of 2-n-nonyloxy-6-bromonaphthalene and 2.7 g of 1,2-dibromoethane, was added 4.9 g of 2,3-difluoro-4-benzyloxyiodobenzene, and the atmosphere was substituted with nitrogen. Under cooling with ice bath to a temperature below 10°C, 143 mg of dichloro-[1,4-bis(di-phenylphosphino)butane] palladium(II) were added, and stirring was continued for an hour in that condition and then for 5 hours without ice bath. Then, water was added under.cooling with ice bath. After addition of toluene, the organic layer was washed with water, and then the solvent was removed. The resulting solid was recrystalized with ethanol to obtain 5.4 g of a compound (j) of the formula: H-Ph-$CH_2$O-FPhF-NAP-O-NON.

(4) To 5.4 g of the compound (j), were added 150 ml of ethanol and 300 mg of 5% palladium carbon, and hydrogenolysis was carried out at reflux temperature. After comfirming that no absorption of hydrogen was observed, the catalyst was filtered off, followed by removing the ethanol to obtain 3.9 g of a compound (k) of the formula

HO-FPhF-NAP-O-NON.

(5) Into 30 ml of pyridine, were dissolved 3.9 g of the compound (k), and 3.0 g of TFMSA were added dropwise thereto at a temperature below 10°C, followed by stirring for 1 day at the room temperature. After reaction, the product was thrown into iced water and extracted with toluene. The toluene layer was washed with 1N-hydrochloric acid and then with water, followed by removing the toluene to obtain a compound (1) of the formula:

$CF_3SO_2$-FPhF-NAP-O-NON.

(6) In 30 ml of triethylamine, 2.3 g of the compound (1) and 0.8 g of 1-nonyne were heated to reflux for 16 hours within an atmosphere of nitrogen in the presence of 5 mg of cuprous iodide (I), 20 mg of dichlorobistriphenylphosphine palladium(II) and 40 mg of triphenylphosphine. After reaction, the temperature was reduced to the room temperature and the triethyl amine was removed. After the product was extracted with toluene, the toluene layer was washed with 1N-hydrochloric acid and then water, followed by treatment with silica gel column. After removing the toluene, 100 ml of ethanol and 200 mg of 5% palladium carbon were added to the resulting solid, and hydrogenation was carried out at reflux temperature. After comfirming that no absorption of hydrogen was observed, the catalyst was filtered off, followed by cooling to the room temperature. The resulting crystal was recystalized twice with ethanol to obtain 0.8 g of white crystalline Compound No.45 of the present invention.

IR ($cm^{-1}$) 2922 2854 1605 1495 1470 1394 1257 1205 855

NMR (ppm) 0.87-0.98(m,6H) 1.20-1.47(m,22H) 1.47-1.59 (m,2H) 1.59-1.71(m,2H) 1.80-1.91(m,2H) 2.70 (t,2H) 4.10(t,2H) 6.97-7.04(m,1H) 7.12-7.24 (m,3H) 7.58-7.65(m,1H) 7.78(d,2H) 7.92(s,1H)

$^{19}$F-NMR(ppm) -67.9(dd,1F) -68.3(dd,1F)

| | | | | |
|---|---|---|---|---|
| Elemental analysis, % | Observed | C: 80.23 | H: 9.25 | N: 7.22 |
| | Theoretical | C: 80.31 | H: 9.06 | N: 7.48 |

Example 24 [Compound No.101 and Compound No.551]

(1) To 400 ml of dry ethanol, were added 25.0 g of 6-bromo-2-naphthol and 7.5 g of potassium hydroxide, and stirred to obtain a homogeneous solution. Then 27.4 g of oa 2-methylbutyl p-toluenesulfonate were added thereto and heated to reflux for 20 hours. After reaction, the ethanol was removed, and then water was added. The resulting solid was filterred and recrystalized with methanol to obtain 24.6 g of oa 6-bromo-2-(2′-methylbutyloxy)-naphthalene

(2) To a diethyl ether solution of a Grignard's reagent prepared from 5.0 g of the oa 6-bromo-2-(2′-methylbutyloxy)naphthalene and 3.2 g of 1,2-dibromoethane, were added 4.9 g of 2,5-dibromopyridine, and the atmosphere was substituted with nitrogen. After cooling to a temperature below 10°C with ice bath, 100 mg of dichloro[1,4-bis(diphenylphosphino)butane] palladium(II) were added and stirred for an hour in that condition and then for 2 hours without ice bath, followed by adding water thereto under cooling with ice bath. Thereafter, toluene was added, and the organic layer was washed with water, followed by removing the solvent. The resultant solid was recrystalized with ethanol to obtain a compound (m) of the formula:

Br-Pyr>-NAP-O-$CH_2$*CH(Me)$C_2H_5$.

(3) In 70 ml of triethylamine, within nitrogen atmosphere, 1.7 g of the compound (m) and 1.3 g of 1-decyne were reacted for 6 hours at reflux temperature in the presence of 5 mg of cuprous iodide(I), 20 mg of dichlorobistriphenylphosphine palladium(II) and 40 mg of triphenylphosphine. After reaction, cooling to the room temperature, the triethylamine was removed. The product was extracted with toluene, and the toluene layer was washed with 1N-hydrochloric acid and then with water, and then treated with silica gel column. The product was recrystalized thrice with ethanol to obtain 1.1 g of white crystalline Compound No.551 of the present invention.

IR ($cm^{-1}$) 2922 2855 2216 1628 1605 1545 1466 1388 1195 990 839

NMR (ppm) 0.88(t,3H) 0.98(t,3H) 1.06(d,3H) 1.17-1.40 m,8H) 1.40-1.53(m,2H) 1.53-1.70(m,4H) 1.87-2.01(m,1H) 2.47(t,2H) 3.82-3.99(m,2H) 7.12-7.20(m,2H) 7.72-7.84(m,4H) 8.08(dd,1H) 8.40 (d,1H) 8.72(d,1H)

| | | | | |
|---|---|---|---|---|
| Elemental analysis, % | Observed | C: 83.10 | H: 8.59 | N: 3.43 |
| | Theoretical | C: 84.31 | H: 8.66 | N: 3.28 |

(4) The above (3) was repeated except that no recrystalization with ethanol was carried out. After removing the toluene from the silica gel-treated product, 150 ml of ethanol and 200 mg of 5% palladium carbon were added,to the resulting solid to carry out hydrogenation at reflux temperature. After comfirming that no absorption of hydrogen was observed, the catalyst was filtered off, followed by cooling to the room temperature. The resulting crystal was recrystalized twice with ethanol to obtain 1.2 g of white crystalline Compound No.101 of the present invention.

IR ($cm^{-1}$) 2922 2854 1630 1605 1493 1468 1390 1255 1212 1300 816 470

NMR (ppm) 0.88(t,3H) 0.99(t,3H) 1.07(d,3H) 1.18-1.43 (m,15H) 1.55-1.70(m,3H) 1.88-2.01(m,1H) 2.64 (t,2H) 3.83-4.00(m,2H) 7.13-7.20(m,2H) 7.55 (dd,1H) 7.72-7.86(3,3H) 8.08(dd,1H) 8.38 (d,1H) 8.53(s,1H)

| | | | | |
|---|---|---|---|---|
| Elemental analysis,% | Observed | C: 83.41 | H: 9.73 | N: 3.39 |
| | Theoretical | C: 83.53 | H: 9.51 | N: 3.25 |

Example 25 [Compound No.201]

(1) To 500 ml of DMSO containing 50 g of 6-bromo-2-naphthol dissolved therein, were added 30 ml of aqueous solution containing 10 g of sodium hydroxide, and stirred to obtain a homogeneous solution. Then 30 g of benzyl chloride were added thereto and stirred for 3 days at the room temperature. The resulting solution was thrown into 2 l of iced water. The resultant precipitates were filterred and recrystalized with ethanol to obtain 67 g of 6-bromo-2-benzyloxynaphthalene.

(2) In 100 ml of triethylamine, 10.0 g of 6-bromo-2-benzyloxynaphthalene and 4.7 g of 1-nonyne were heated to reflux for 8 hours within an atmosphere of nitrogen in the presence of 120 mg of dichlorobistriphenylphosphine palladium(II), 28 mg of cuprous iodide(I), and 224 mg of triphenylphosphine. After reaction, the triethyl amine was removed. After the product was extracted with toluene, the toluene layer was washed with 1N-hydrochloric acid and then water, followed by removing the toluene. The product was recrystalized with ethanol to obtain 9.1 g of a compound (n) of the formula:

n-$C_7H_{15}$-#-NAP-O-$CH_2$-Ph-H.

(3) Within an atmosphere of hydrogen, 9.1 g of the compound (n) were hydrogenated and hydrogenolyzed in 150 ml of ethanol using 0.5 g of 5% palladium carbon. After comfirming that no absorption of hydrogen was observed, the catalyst was filtered off. After removing the ethanol, the resultant solid was recrystalized with hexane to obtain 5.9 g of 6-nonyl-2-hydroxynaphthalene.

(4) Into 100 ml of anhydrous pyridine, were dossolved 5.9 g of 6-nonyl-2-hydroxynaphthalene, and cooled to a temperature below 10°C. Then, 6.5 g of trifluoromethane sulfonic anhydride (TFMSA) were dropwise

added thereto at a temperature below 10°C, followed by stirring for 24 hours at the room temperature. The resulting solution was thrown into iced water and acidified with hydrochloric acid, and then extracted with hexane. The hexane phase was washed with water, followed by removing the hexane to obtain 8.4 g of an oily compound (o) of the formula:

$$NON\text{-}NAP\text{-}O\text{-}SO_2CF_3.$$

(5) To 100 ml of DMSO containing 10 g of 2,3-difluorophenol dissolved therein, were added 10 ml of aqueous solution containing 3.6 g of sodium hydroxide, and stirred to obtain a homogeneous solution. Then 13.1 g of n-heptyl bromide were added thereto and stirred for 3 days at the room temperature. The resulting solution was thrown into iced water and extracted with hexane. The hexane phase was washed with water and the hexane was removed to obtain 15.8 g of oily 1,2-difluoro-3-n-heptyloxybenzene.

(6) Into 200 ml of THF, were dissolved 15.8 g of 1,2-difluoro-3-n-heptyloxybenzene, and cooled with dry ice-acetone bath to a temperature below -60°C. Then, 47 ml of 15% solution of n-butyl lithium in hexane were added dropwise thereto at a temperature below -60°C, and stirring was continued for 3 hours in that conditions. Subsequently, 8.7 g of trimethyl borate were added dropwise at a temperature below -60°C, and stirring was continued for 30 minutes in that conditions. Thereafter, the dry ice-acetone bath was removed, and then stirring was continued until the temperature reaches 0°C. The reaction product was washed with water and then with 1N-hydrochloric acid, and extracted with diethyl ether. After washing the ether phase with water, the ether was removed to obtain 17.9 g of white solid of 2,3-difluoro-4-n-heptyloxyphenyl boric acid.

(7) In 70 ml of triethylamine, 3.0 g of the compound (o) and 2.0 g of 2,3-difluoro-4-n-heptyloxyphenyl boric acid were heated to reflux for 10 hours within an atmosphere of nitrogen in the presence of 170 mg of tetrakistriphenylphosphine palladium(0) and 170 mg of triphenylphosphine. After reaction, the triethyl amine was removed and water was added, followed by extracting the product with toluene. The resulting product was washed with 1N-hydrochloric acid and then with water, purified with silica gel colmun, and recrystalized thrice with ethanol to obtain 2.1 g of white crystalline Compound No.201 of the present invention.

| | | | | |
|---|---|---|---|---|
| Elemental analysis,% | Observed | C: 79.83 | H: 8.96 | F: 7.99 |
| | Theoretical | C: 80.00 | H: 8.75 | F: 7.92 |

Example 26 [Compound No.202]

(1) Example 25 (2)-(4) were repeated except that 1-nonyne was substituted with 1-decyne in Example 25 (2) to obtain a compound (p) of the formula:

$$DEC\text{-}NAP\text{-}OSO_2CF_3.$$

(2) Example 25 (7) was repeated except that the compound (o) was substituted with the compound (p) to obtain Compound No.202.

Example 27 [Compound No.204]

(1) Example 25 (2)-(4) were repeated except that 1-nonyne was substituted with 1-octyne in Example 25 (2) to obtain a compound (q) of the formula:

$$OCT\text{-}NAP\text{-}OSO_2CF_3.$$

(2) Example 25 (5) and (6) were repeated except that n-heptyl bromide was substituted with n-octyl bromide to obtain 2,3-difluoro-4-n-octyloxyphenyl boric acid.

(3) The compound (q) and 2,3-difluoro-4-n-octyloxyphenyl boric acid were reacted under the same conditions as in Example 25 (7) to obtain Compound No.204.

Example 28 [Compound No.210]

(1) Example 25 (2)-(4) were repeated except that 1-nonyne was substituted with 1-heptyne in Example 25 (2) to obtain a compound (r) of the formula:

$$HEP\text{-}NAP\text{-}OSO_2CF_3.$$

(2) Example 25 (5) and (6) were repeated except that n-heptyl bromide was substituted with n-decyl bromide to obtain 2,3-difluoro-4-n-decyloxyphenyl boric acid.

(3) The compound (r) and 2,3-difluoro-4-n-decyloxyphenyl boric acid were reacted under the same conditions as in Example 25 (7) to obtain Compound No.210.

Example 29 [Compound No.222]

(1) Into 220 ml of THF, were dissolved 16.6 g of orthodifluorobenzene, and cooled with dry ice-acetone bath to a temperature below -60°C. Then, 99 ml of 15% solution of n-butyl lithium in hexane were added dropwise thereto at a temperature below -60°C, and stirring was continued for 3 hours in that conditions. Subsequently, 20.0 g of trimethyl borate were added dropwise at a temperature below -60°C, and stirring was continued for 30 minutes in that conditions. Thereafter, the dry ice-acetone bath was removed, and then stirring was continued until the temperature reaches 0°C. The reaction product was washed with water and then with 1N-hydrochloric acid, and extracted with diethyl ether. After washing the ether phase with water, the ether was removed to obtain 21.1 g of white solid of 2,3-difluorophenyl boric acid.
(2) In 100 ml of triethylamine, 1.9 g of 2,3-difluorophenyl boric acid and 5.0 g of the compound (q) prepared in Example 27 (1) were heated to reflux for 10 hours within an atmosphere of nitrogen in the presence of 278 mg of tetrakistriphenylphosphine palladium(0) and 278 mg of triphenylphosphine. After reaction, the triethyl amine was removed and water was added, followed by extracting the product with toluene. The resulting product was washed with 1N-hydrochloric acid and then with water, followed by removing the toluene to obtain 3.9 g of 2-(2,3-difluorophenyl)-6-n-octylnaphthalene.
(3) Into 100 ml of THF, were dissolved 3.9 g of 2-(2,3-difluorophenyl)-6-n-octylnaphthalene, and cooled with dry ice-acetone bath to a temperature below -60°C. Then, 7 ml of 15% solution of n-butyl lithium in hexane were added dropwise thereto at a temperature below -60°C, and stirring was continued for 3 hours in that conditions. Subsequently, 2.0 g of trimethyl borate were added dropwise at a temperature below -60°C, and stirring was continued for 30 minutes in that conditions. Thereafter, the dry ice-acetone bath was removed, and then stirring was continued until the temperature reaches 0°C. The reaction product was washed with water and then with 1N-hydrochloric acid, and extracted with diethyl ether. After washing the ether phase with water, the ether was removed to obtain 4.1 g of obtain a compound (s) of the formula:

$$OCT\text{-}NAP\text{-}FPhF\text{-}B(OH)_2.$$

(4) Into diethyl ether containing 4.1 g of the compound (s) dissolved therein, were added 20 ml of 10 % aqueous hydrogen peroxide, and the mixture was stirred for 1 hour at the room temperature, and then heated for 2 hours to reflux. After reaction, the ether phase was washed with water, and the ether was removed to obtain 3.5 g of white solid of 2-(2,3-difluoro-4-hydroxyphenyl)-6-n-octylnaphthalene.
(5) Into 50 ml of DMSO, were dissolved 2.0 g of the 2-(2,3-difluoro-4-hydroxyphenyl)-6-n-octylnaphthalene, and 5 ml of aqueous solution containing 0.25 g of sodium hydroxide were added thereto and stirred to obtain a homogeneous solution. Then, to the solution were added 1.2 g of 10-bromo-1-decene, prepared by reacting triphenyl phosphine and carbon tetrabromide with 9-decene-1-ol, stirred for 3 days at the room temperature. The resulting solution was thrown into 200 ml of iced water and extracted with toluene. The toluene phase was washed with water and purified with silica gel colmun, and recrystalized thrice with ethanol to obtain 1.7 g of white crystalline Compound No.222 of the present invention.

| | | | | |
|---|---|---|---|---|
| Elemental analysis, % | Observed | C: 80.91 | H: 8.63 | F: 7.38 |
| | Theoretical | C: 80.63 | H: 8.70 | F: 7.51 |

Example 30 [Compound No.224]

(1) To 30 ml of diethyl ether containing 4.3 g of trimethyl borate dissolved therein, were added dropwise at a temperature below -30°C 30 ml of diethyl ether solution of a Grignard's reagent prepared from 10.0 g of 4-n-heptyloxy-3-fluoro-bromobenzene, prepared by reacting n-heptyl bromide with alkali metal 4-bromo-2-fluorophenolate. After addition, the solution was stirred for 1 hour in that conditions and then stirring was continued without using refrigerant until the temperature return to the room temperature. The resulting solution was thrown into iced water, and the separated organic phase was washed with 1N-hydrochloric acid and then with water, followed by removing the ether to obtain 7.1 g of 4-n-heptyloxy -3-fluorophenyl boric acid.
(2) In 60 ml of triethylamine, 1.5 g of 4-n-heptyloxy-3-fluorophenyl boric acid and 2.5 g of the compound (p) prepared in Example 26 (1) were heated to reflux for 10 hours within an atmosphere of nitrogen in the presence of 139 mg of tetrakistriphenylphosphine palladium(0) and 139 mg of triphenylphosphine. After reaction, the triethyl amine was removed and water was added, followed by extracting the product with toluene. The toluene phase was washed with 1N-hydrochloric acid and then with water, purified with silica gel colmun, and recrystalized thrice with ethanol to obtain 1.8 g of white crystalline Compound No.224 of

the present invention.

| Elemental analysis, % | Observed | C: 83.07 | H: 9.53 | F: 4.13 |
|---|---|---|---|---|
| | Theoretical | C: 83.20 | H: 9.45 | F: 3.99 |

Example 31 [Compound No.243]

(1) Into 30 ml of diethyl ether containing 3.8 g of trimethyl borate dissolved therein, were added dropwise at a temperature below -30°C 60 ml of diethyl ether solution of a Grignard's reagent prepared from 10.0 g of 4-(9-decenyloxy )-3-fluoro-bromobenzene, prepared by reacting alkali metal 4-bromo-2-fluorophenolate with p-toluenesulfonate of 9-decene-1-ol. After addition, the solution was stirred for 1 hour in that conditions and then stirring was continued without using refrigerant until the temperature return to the room temperature. The resulting solution was thrown into iced water, and the separated organic phase was washed with 1N-hydrochloric acid and then with water, followed by removing the ether to obtain 8.1 g of 4-(9-decenyloxy)-3-fluorophenyl boric acid.

(2) In 60 ml of triethylamine, 1.6 g of 4-(9-decenyloxy )-3-fluorophenyl boric acid and 2.5 g of the compound (r) prepared in Example 28 (1) were heated to reflux for 10 hours within an atmosphere of nitrogen in the presence of 124 mg of tetrakistriphenylphosphine palladium(0) and 124 mg of triphenylphosphine. After reaction, the triethyl amine was removed and water was added, followed by extracting the product with toluene. The toluene phase was washed with 1N-hydrochloric acid and then with water, purified with silica gel colmun, and recrystalized thrice with ethanol to obtain 1.8 g of white crystalline Compound No.243 of the present invention.

| Elemental analysis, % | Observed | C: 83.39 | H: 9.23 | F: 3.90 |
|---|---|---|---|---|
| | Theoretical | C: 83.54 | H: 9.07 | F: 4.01 |

Example 32 [Compound No.246]

(1) Into 30 ml of diethyl ether containing 4.3 g of trimethyl borate dissolved therein, were added dropwise at a temperature below -30°C 60 ml of diethyl ether solution of a Grignard's reagent prepared from 10.0 g of 4-n-nonyloxy -2-fluoro-bromobenzene, prepared by reacting n-nonyl bromide with alkali metal 4-bromo-3-fluorophenolate. After addition, the solution was stirred for 1 hour in that conditions and then stirring was continued without using refrigerant until the temperature return to the room temperature. The resulting solution was thrown into iced water, and the separated organic phase was washed with 1N-hydrochloric acid and then with water, followed by removing the ether to obtain 7.6 g of 4-n-nonylyoxy-2-fluorophenyl boric acid.

(2) In 60 ml of triethylamine, 1.4 g of 4-n-nonylyoxy-2-fluorophenyl boric acid and 2.0 g of the compound (o) prepared in Example 25 (4) were heated to reflux for 10 hours within an atmosphere of nitrogen in the presence of 115 mg of tetrakistriphenylphosphine palladium(0) and 115 mg of triphenylphosphine. After reaction, the triethyl amine was removed and water was added, followed by extracting the product with toluene. The toluene phase was washed with 1N-hydrochloric acid and then with water, purified with silica gel colmun, and recrystalized thrice with ethanol to obtain 1.6 g of white crystalline Compound No.246 of the present invention.

| Elemental analysis, % | Observed | C: 83.40 | H: 9.60 | F: 3.97 |
|---|---|---|---|---|
| | Theoretical | C: 83.26 | H: 9.59 | F: 3.88 |

Example 33 [Compound No.248 and Compound No.602]

(1) Example 1 (4) was repeated except that 16.1 g of alpha-n-nonyl-beta-dimethylaminoacrolein were substituted with 15.2 g of commercially available alpha-n-heptyloxy-beta-dimethylaminoacrolein to obtain 9.3 g of a compound (t) of the formula:

HEP-O-Pym>-NAP-OH.

(2) To 100 ml of dry pyridine solution containing 5.0 g of the compound (t), 4.6 g of TFMSA were added dropwise at a temperature below 10°C, followed by continuing stirring for 1 hour in that conditions and then for 24 hours at the room temperature. After reaction, the product was thrown into 300 ml of iced water, and extracted with toluene. The toluene phase was washed with 1N-hydrochloric acid and then with water, followed by removing the toluene and then recrystalizing with hexane to obtain 5.9 g of a solid compound (u) of the formula:

HEP-O-Pym>-NAP-$OSO_2CF_3$.

(3) In 60 ml of triethylamine, within nitrogen atmosphere, 2.5 g of the compound (u) an 0.9 g of 1-decyne were reacted for 8 hours at reflux temperature in the presence of 5 mg of cuprous iodide(I), 20 mg of dichlorobistriphenylphosphine palladium(II) and 40 mg of triphenylphosphine. After reaction, the triethylamine was removed, and the product was extracted with toluene, followed by washing the toluene phase with 1N-hydrochloric acid and then with water and subsequently recrystalizing thrice with ethanol to obtain 2.1 g of white crystalline Compound No.602 of the present invention.

| | | | | |
|---|---|---|---|---|
| Elemental analysis, % | Observed | C: 81.63 | H: 8.69 | N: 6.21 |
| | Theoretical | C: 81.58 | H: 8.77 | N: 6.14 |

(4) Within an atmosphere of hydrogen, 1.0 g of the compound No.602 was hydrogenated with 5% palladium carbon in 100 ml ethanol. After confirming that no absorption of hydrogen was observed, the catalyst was removed, followed by removing the solvent. The resulting solid was thrice recrystalized with ethanol to obtain 0.4 g of white crystalline Compound No.248 of the present invention.

| | | | | |
|---|---|---|---|---|
| Elemental analysis,% | Observed | C: 80.63 | H: 9.63 | N: 6.21 |
| | Theoretical | C: 80.87 | H: 9.56 | N: 6.09 |

Example 34 [Compound No.252]

(1) Example 32 (1) was repeated except that 4-n-nonyloxy-2-fluoro-bromobenzene was substituted with 4-n-decyloxy-bromobenzene, prepared by reacting n-decyl bromide with alkali metal 4-bromophenolate, to obtain 7.5 g of 4-n-decyloxyphenyl boric acid.

(2) In 60 ml of triethylamine, within nitrogen atmosphere, 1.5 g of 4-n-decyloxyphenyl boric acid and 2.5 g of the compound (q) prepared in Example 27 (1) were reacted for 10 hours at reflux temperature in the presence of 119 mg of tetrakistriphenylphosphine palladium(0) and 119 mg of triphenylphosphine. After reaction, the triethylamine was removed, and water was added. The product was extracted with toluene. The toluene phase was washed with 1N-hydrochloric acid and then with water, purified with silica gel colmun and subsequently recrystalized with thrice ethanol to obtain 1.4 g of white crystalline Compound No.252 of the present invention.

| | | | | |
|---|---|---|---|---|
| Elemental analysis,% | Observed | C: 86.59 | H: 10.0 | |
| | Theoretical | C: 86.44 | H: 10.17 | |

Example 35 [Compound No.309]

(1) Into 230 ml of anhydrous THF, were dissolved 20.0 g of 1,2-difluoro-3-n-nonyloxybenzene prepared by reacting n-nonyl bromide with alkali metal 2,3-difluorophenolate, and cooled with dry ice-acetone bath to a temperature below -60°C. Then, 53 ml of 15% solution of n-butyl lithium in hexane were added dropwise thereto at a temperature below -60°C, and stirring was continued for 3 hours in that conditions. Subsequently, 9.0 g of trimethyl borate were added dropwise at a temperature below -60°C, and stirring was continued for 30 minutes in that conditions. Thereafter, the dry ice-acetone bath was removed, and then stirring was continued until the temperature reaches 0°C. The reaction product was washed with water and then with 1N-hydrochloric acid, and extracted with diethyl ether. After washing the ether phase with water, the ether was removed to obtain 21.8 g of white solid of 2,3-difluoro-4-n-nonyloxyphenyl boric acid.

(2) In 60 ml of triethylamine, 2.0 g of 2,3-difluoro-4-n-nonyloxyphenyl boric acid and 2.3 g of 6-bromo-2-nonyloxy-naphthalene prepared by reacting n-nonyl bromide with alkali metal 6-bromonaphtholate were heated to reflux for 10 hours within an atmosphere of nitrogen in the presence of 154 mg of tetrakistriphenylphosphine palladium(0) and 154 mg of triphenylphosphine. After reaction, the triethyl amine was removed and the product was extracted with toluene. The toluene phase was washed with 1N-hydrochloric acid and then with water, purified with silica gel colmun, and subsequently recrystalized thrice with ethanol to obtain 2.1 g of of white crystalline Compound No.309 of the present invention.

| | | | | |
|---|---|---|---|---|
| Elemental analysis,% | Observed | C: 77.63 | H: 8.91 | F: 7.13 |
| | Theoretical | C: 77.86 | H: 8.78 | F: 7.25 |

Example 36 [Compound No.321]

(1) Into 200 ml of anhydrous THF, were dissolved 15.0 g of 2,3-difluoro-4-(9-decenyloxy)benzene prepared by reacting 9-decene-1-ol p-toluenesulfonate with alkali metal 2,3-difluorophenolate, and cooled with dry ice-acetone bath to a temperature below -60°C. Then, 40 ml of 15% solution of n-butyl lithium in hexane were added dropwise thereto at a temperature below -60°C, and stirring was continued for 3 hours in that conditions. Subsequently, 6.8 g of trimethyl borate were added dropwise at a temperature below -60°C, and stirring was continued for 30 minutes in that conditions. Thereafter, the dry ice-acetone bath was removed, and then stirring was continued until the temperature reaches 0°C. The reaction product was washed with water and then with 1N-hydrochloric acid, and extracted with diethyl ether. After washing the ether phase with water, the ether was removed to obtain 16.3 g of white solid of 2,3-difluoro-4-(9-decenyloxy )phenyl boric acid.

(2) In 60 ml of triethylamine, 2.0 g of 2,3-difluoro-4-(9-decenyloxy)phenyl boric acid and 2.0 g of 6-bromo-2-heptyloxynaphthalene prepared by reacting n-heptyl bromide with alkali metal 6-bromonaphtholate were heated to reflux for 10 hours within an atmosphere of nitrogen in the presence of 148 mg of tetrakistriphenylphosphine palladium (0) and 148 mg of triphenylphosphine. After reaction, the triethyl amine was removed and the product was extracted with toluene. The toluene phase was washed with 1N-hydrochloric acid and then with water, purified with silica gel colmun, and subsequently recrystalized thrice with ethanol to obtain 2.1 g of of white crystalline Compound No.321 of the present invention.

| | | | | |
|---|---|---|---|---|
| Elemental analysis,% | Observed | C: 78.02 | H: 8.13 | F: 7.40 |
| | Theoretical | C: 77.95 | H: 8.27 | F: 7.48 |

Example 37 [Compound No.324]

In 60 ml of triethylamine, within nitrogen atmosphere, 1.5 g of 2,3-difluoro-3-n-heptyloxyphenyl boric acid prepared by repeating Example 35 (1) except using 1,2-difluoro-3-n-heptyloxybenzene instead of 1,2-difluoro -3-n-nonyloxybenzene and 2.0 g of 6-bromo-2-(9-decenyloxy )-naphthalene prepared by reacting 9-decene-1-ol p-toluene-sulfonate with alkali metal 6-bromo-2-naphtholate were reacted for 10 hours at reflux temperature in the presence of 127 mg of tetrakistriphenylphosphine palladium (0) and 127 mg of triphenylphosphine. After reaction, the triethyl amine was removed and the product was extracted with toluene. The toluene phase was washed with 1N-hydrochloric acid and then with water, purified with silica gel colmun, and subsequently recrystalized thrice with ethanol to obtain 1.7 g of of white crystalline Compound No.324.

| | | | | |
|---|---|---|---|---|
| Elemental analysis,% | Observed | C: 77.81 | H: 8.33 | F: 7.55 |
| | Theoretical | C: 77.95 | H: 8.27 | F: 7.48 |

Example 38 [Compound No.337]

(1) Into 30 ml of diethyl ether containing 5.7 g of trimethyl borate dissolved therein and cooled to a temperature below -30°C, were added dropwise at a temperature below -30°C 30 ml of diethyl ether solution of a Grignard's reagent prepared from 15.0 g of 4-n-octyloxy-3-fluoro -bromobenzene, prepared by reacting n-octyl bromide with alkali metal 4-bromo-2-fluorophenolate. After addition, the solution was stirred for 1

hour in that conditions and then stirring was continued without using refrigerant until the temperature return to the room temperature. The resulting solution was thrown into iced water, and the separated organic phase was washed with 1N-hydrochloric acid and then with water, followed by removing the ether to obtain 11.7 g of 4-n-octyloxy-3-fluorophenyl boric acid.

(2) In 80 ml of triethylamine, 2.0 g of 4-n-octyloxy-3-fluorophenyl boric acid and 2.5 g of 6-bromo-2-octyloxy-naphthalene prepared by reacting n-octyl bromide with alkali metal 6-bromo-2-naphthol were heated to reflux for 10 hours within an atmosphere of nitrogen in the presence of 172 mg of tetrakistriphenylphosphine palladium(0) and 172 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 2.0 g of white crystalline Compound No.337 of the present invention.

| Elemental analysis,% | Observed | C: 80.45 | H: 8.83 | F: 3.73 |
|---|---|---|---|---|
| | Theoretical | C: 80.34 | H: 9.00 | F: 3.97 |

Example 39 [Compound No.356]

In 60 ml of triethylamine, within nitrogen atmosphere, 2.3 g of 6-bromo-2-octyloxy-naphthalene and 2.0 g of 4-(9-decenyloxy)-3-fluorophenyl boric acid, prepared by repeating Example 38 (1) except using 4-(9-decenyloxy)-3-fluoro-bromobenzene instead of 4-n-octyloxy-3-fluoro-bromobenzene were reacted for 10 hours at reflux temperature in the presence of 157 mg of tetrakistriphenylphosphine palladium(0) and 157 mg of triphenylphosphine. After reaction, the triethyl amine was removed and the product was extracted with toluene. The toluene phase was washed with 1N-hydrochloric acid and then with water, purified with silica gel colmun, and subsequently recrystalized thrice with ethanol to obtain 1.9 g of of white crystalline Compound No.356 of the present invention.

| Elemental analysis,% | Observed | C: 81.03 | H: 8.80 | F: 3.89 |
|---|---|---|---|---|
| | Theoretical | C: 80.95 | H: 8.93 | F: 3.77 |

Example 40 [Compound No.362]

In 80 ml of triethylamine, within nitrogen atmosphere, 2.5 g of 6-bromo-2-(9-decenyloxy)naphthalene and 2.0 g of 4-n-octyloxy-3-fluorophenyl boric acid were reacted for 10 hours at reflux temperature in the presence of 172 mg of tetrakistriphenylphosphine palladium(0) and 172 mg of triphenylphosphine. After reaction, the triethyl amine was removed and the product was extracted with toluene. The toluene phase was washed with 1N-hydrochloric acid and then with water, purified with silica gel colmun, and subsequently recrystalized thrice with ethanol to obtain 2.1 g of white crystalline Compound No.362 of the present invention.

| Elemental analysis,% | Observed | C: 80.51 | H: 8.73 | F: 4.07 |
|---|---|---|---|---|
| | Theoretical | C: 80.68 | H: 8.61 | F: 3.99 |

Example 41 [Compound No.379]

(1) Example 1 (4) was repeated except that 16.1 g of alpha-n-nonyl-beta-dimethylaminoacrolein were substituted with 17.0 g of commercially available alpha-n-nonyloxybeta-dimethylaminoacrolein to obtain 11.2 g of a compound (w) of the formula:

NON-O-Pym>-NAP-OH.

(2) Into 30 ml of DMSO, were dissolved 2.0 g of the compound (w), and 5 ml of aqueous solution containing 0.26 g of sodium hydroxide were added thereto and stirred to form homogeneous solution, followed by adding 1.1 g of n-octyl bromide and continuing stirring for 3 days at the room temperature. After reaction, the product was thrown into 100 ml of iced water, and extracted with toluene. The toluene phase was washed with 1N-hydrochloric acid and then with water and then purified with silica gel colmun, followed by recrystalizing trice with ethanol to obtain 1.4 g of of white crystalline Compound No.379 of the present invention.

| Elemental analysis,% | Observed | C: 78.30 | H: 9.31 | N: 5.63 |
|---|---|---|---|---|
| | Theoretical | C: 78.16 | H: 9.24 | N: 5.88 |

Example 42 [Compound No.383]

Example 41 (2) was repeated except that n-octyl bromide was substituted with 8-bromo-1-octene to obtain 1.2 g of of white crystalline Compound No.383 of the present invention.

| Elemental analysis,% | Observed | C: 78.21 | H: 8.99 | N: 6.04 |
|---|---|---|---|---|
| | Theoretical | C: 78.48 | H: 8.86 | N: 5.91 |

Example 43 [Compound No.388]

(1) To 100 ml of dry DMF containing 1.6 g of sodium hydride, were added dropwise 10.0 g of n-decyl alcohol at a temperature below 50°C in such a speed not causing vigorous evolution of hydrogen. After confirming that no evolution of hydrogen was observed, the DMF solution was cooled with ice bath to a temperature below 10°C, and 15.0 g of 2,5-dibromopyridine were added thereto, followed by continuing stirring for 1 hour in that conditions and then for 2 hours at 80°C. After cooling to the room temperature, the product was thrown into iced water, and the formed oil was extracted with hexane. The hexane phase was washed with water, followed by removing the hexane to obtain 18.9 g of liquid 5-bromo-2-decyloxypyridine.

(2) To 50 ml of diethyl ether solution of a Grignard's reagent prepared from 5.0 g of 6-bromo-2-octyloxy-naphthalene, 4.6 g of 5-bromo-2-decyloxypyridine were added. Within an atmosphere of nitrogen, after cooling to a temperature below 10°C with ice bath, were added 90 mg of dichloro[1,4-bis(diphenylphosphino)butane]palladium(II), followed by continuing stirring for 1 hour in that conditions and then for 5 hours without ice bath. Thereafter, water was added under cooling with ice bath. After addition of toluene, the organic phase was washed with water and then the solvents were removed. The resulting solid was dissolved in toluene, purified with silica gel column, and then recrystalized thrice with ethanol to obtain 2.6 g of white crystalline Compound No.388 of the present invention.

| Elemental analysis,% | Observed | C: 81.13 | H: 9.47 | F: 2.99 |
|---|---|---|---|---|
| | Theoretical | C: 80.98 | H: 9.61 | F: 2.86 |

Example 44 [Compound No.527]

(1) To 100 ml of diethyl ether solution of a Grignard's reagent prepared from 10.0 g of 6-bromo-2-octyloxynaphthalene, 9.0 g of 1-bromo-3-fluoro-4-iodobenzene were added. Within an atmosphere of nitrogen, after cooling to a temperature below 10°C with ice bath, were added 180 mg of dichloro[1,4-bis(diphenylphosphino)butane]palladium(II), followed by continuing stirring for 1 hour in that conditions and then for 5 hours without ice bath. Thereafter, water was added under cooling with ice bath. After addition of toluene, the organic phase was washed with water and then the solvents were removed. The resulting solid was recrystalized with ethanol to obtain 8.2 g of a compound (x) of the formula :

Br-PhF-NAP-O-OCT.

(2) In 50 ml of triethylamine, within nitrogen atmosphere, 2.5 g of the compound (x) and 1.0 g of 1-decyne were reacted for 8 hours at reflux temperature in the presence of 5 mg of cuprous iodide(I), 20 mg of dichlorobistriphenylphosphine palladium(II) and 40 mg of triphenylphosphine. After reaction, the triethylamine was removed, and the product was extracted with toluene. the toluene phase was washed with 1N-hydrochloric acid and then with water, purified with silica gel column and subsequently recrystalized thrice with ethanol to obtain 1.4 g of white crystalline Compound No.527 of the present invention.

| Elemental analysis,% | Observed | C: 84.01 | H: 8.63 | N: 4.12 |
|---|---|---|---|---|
| | Theoretical | C: 83.95 | H: 8.85 | N: 3.91 |

Example 45 [Compound No.501]

(1) Example 44 (1) was repeated except that 9.0 g of 1-bromo -3-fluoro-4-iodobenzene was substituted with 7.1 g of 2,5-dibromopyridine to obtain 8.7 g of a compound (x′) of the formula :

Br-Pyr>-NAP-O-OCT.

(2) In 50 ml of triethylamine, within nitrogen atmosphere, 3.0 g of the compound (x′) and 1.0 g of 1-octyne were reacted for 8 hours at reflux temperature in the presence of 6 mg of cuprous iodide, 24 mg of dichlorobistriphenylphosphine palladium(II) and 48 mg of triphenylphosphine. After reaction, the triethylamine was removed, and the product was extracted with toluene. The toluene phase was washed with 1N-hydrochloric acid and then with water, purified with silica gel column and subsequently recrystalized thrice with ethanol to obtain 1.9 g of white crystalline Compound No.501 of the present invention.

| | | | | |
|---|---|---|---|---|
| Elemental analysis,% | Observed | C: 84.13 | H: 9.01 | N: 3.22 |
| | Theoretical | C: 84.36 | H: 8.84 | N: 3.17 |

Example 46 [Compound No.547]

(1) In 200 ml of triethylamine, within nitrogen atmosphere, 10.0 g of 2,5-dibromopyridine and 5.8 g of 1-decyne were reacted for 8 hours at reflux temperature in the presence of 20 mg of cuprous iodide(I), 80 mg of dichlorobistriphenylphosphine palladium(II) and 160 mg of triphenylphosphine. After reaction, the triethylamine was removed, and the product was extracted with hexane. The hexane phase was washed with 1N-hydrochloric acid and then with water, followed by removing the hexane to obtain 10.8 g of a compound (y) of the formula :

OCT-#-<Pyr-Br.

(2) Example 43 (2) was repeated except that 4.6 g of 5-bromo-2-decyloxypyridine were substituted with 4.4 g of the compound (y) to obtain 1.7 g of white crystalline Compound No.547 of the present invention.

| | | | | |
|---|---|---|---|---|
| Elemental analysis,% | Observed | C: 84.21 | H: 9.33 | N: 3.05 |
| | Theoretical | C: 84.43 | H: 9.17 | N: 2.99 |

Example 47 [Compound No.515]

(1) In 200 ml of triethylamine, within nitrogen atmosphere, 5.2 g of 1-nonyne and 10.0 g of 1,2-difluoro-3-iodobenzene, prepared by reacting o-difluorobenzene with n-butyl lithium and then reacting with iodine, were reacted for 8 hours at the room temperature in the presence of 20 mg of cuprous iodide(I) and 80 mg of dichlorobistriphenylphosphine palladium(II). After reaction, the triethylamine was removed, and the product was extracted with hexane. The hexane phase was washed with 1N-hydrochloric acid and then with water, followed by removing the hexane to obtain 9.2 g of an oily compound (z) of the formula:

OCT-#-FPhF-H.

(2) Into 150 ml of anhydrous THF, were dissolved 9.2 g of the compound (z), and cooled with dry ice-acetone bath to a temperature below -60°C. Then, 26 ml of 15% solution of n-butyl lithium in hexane were added dropwise thereto at a temperature below -60°C, and stirring was continued for 3 hours in that conditions. Subsequently, 4.8 g of trimethyl borate were added dropwise at a temperature below -60°C, and stirring was continued for 30 minutes in that conditions. Thereafter, the dry ice-acetone bath was removed, and then stirring was continued until the temperature reaches 0°C. The reaction product was washed with water and then with 1N-hydrochloric acid, and extracted with diethyl ether. After washing the ether phase with water, the ether was removed to obtain 9.8 g of a white solid compound (z′) of the formula :

OCT-#-FPhF-$B(OH)_2$.

(3) In 60 ml of triethylamine, within nitrogen atmosphere, 2.0 g of the compound (z′) and 2.0 g of 6-bromo-2-nonyloxynaphthalene, prepared by reacting alkali metal 6-bromo-2-naphtholate with n-nonyl bromide, were reacted for 10 hours at reflux temperature in the presence of 165 mg of tetrakistriphenylphosphine palladium(0) and 165 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 2.0 g of white crystalline Compound No.515 of the present invention.

| Elemental analysis,% | Observed | C: 81.09 | H: 8.17 | F: 3.38 |
|---|---|---|---|---|
| | Theoretical | C: 80.95 | H: 8.33 | F: 3.54 |

Example 48 [Compound No.537]

(1) In 150 ml of triethylamine, within nitrogen atmosphere, 4.6 g of 1-decyne and 10.0 g of 1-bromo-3-fluoro-4-iodobenzene were reacted for 8 hours at the room temperature in the presence of 18 mg of cuprous iodide(I) and 72 mg of dichlorobistriphenylphosphine palladium(II). After reaction, the triethylamine was removed, and the product was extracted with hexane. The hexane phase was washed with 1N-hydrochloric acid and then with water, followed by removing the hexane to obtain 9.0 g of an oily compound (y′) of the formula :

OCT-#-FPh-Br.

(2) To 50 ml of a diethyl ether solution of a Grignard's reagent prepared from 5.0 g of 6-bromo-2-octyloxynaphthalene, were added 4.6 g of the compound (y′), and the atmosphere was substituted with nitrogen. After cooling to a temperature below 10°C with ice bath, 90 mg of dichloro-[1,4-bis(diphenylphosphino)butane] palladium(II) were added and stirred for an hour in that conditions and then for 5 hours without ice bath, followed by adding water thereto under cooling with ice bath. Thereafter, toluene was added, and the organic layer was washed with water, followed by removing the solvent. The resultant solid was dissolved into toluene, purified with silica gel column and recrystalized thrice with ethanol to obtain 1.8 g of white crystalline Compound No.537 of the present invention.

| Elemental analysis,% | Observed | C: 83.73 | H: 8.91 | N: 3.78 |
|---|---|---|---|---|
| | Theoretical | C: 83.95 | H: 8.85 | N: 3.91 |

Example 49 [Compound No.104]

Example 24 (1), (2) and (4) were repeated except that oa 1-methylheptyl p-toluenesulfonate was used instead of oa 2-methylbutyl p-toluenesulfonate in Example 24 (1) to obtain Compound No.104 of the present invention.

| Elemental analysis,% | Observed | C: 83.55 | H: 9.90 | H: 3.17 |
|---|---|---|---|---|
| | Theoretical | C: 83.72 | H: 9.94 | H: 2.96 |

Example 50 [Compound No.391]

(1) Into 200 ml of anhydrous THF, were dissolved 20.0 g of 1,2-difluoro-3-n-decyloxybenzene prepared by reacting n-decyl bromide with alkali metal 2,3-difluorophenolate, and cooled with dry ice-acetone bath to a temperature below -60°C. Then, 47 ml of 15% solution of n-butyl lithium in hexane were added dropwise thereto at a temperature below -60°C, and stirring was continued for 3 hours in that conditions. Thereafter, the dry ice-acetone bath was removed, and then stirring was continued until the temperature reached 0°C. Water and then 1N-hydrochloric acid were added, and then the product was extracted with diethylether, followed by washing the ether phase with water and removing the ether to obtain 21.6 g of white solid of 2,3-difluoro-4-n-nonyloxyphenyl boric acid.

(2) In 60 ml of triethylamine, within nitrogen atmosphere, 2.0 g of 2,3-difluoro-4-n-nonyloxyphenyl boric acid and 2.0 g of oa 6-bromo-2-(2-methylbutyloxy)naphthalene were reacted for 10 hours at reflux temperature in the presence of 147 mg of tetrakistriphenyl-phosphine palladium(0) and 147 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.9 g of white crystalline Compound No.391 of the present invention.

| | | | | |
|---|---|---|---|---|
| Elemental analysis,% | Observed | C: 77.03 | H: 8.51 | F: 7.74 |
| | Theoretical | C: 77.18 | H: 8.30 | F: 7.88 |

Example 51 [Compound No.393]

Example 50 was repeated except that oa 6-bromo-2(1-methylheptyloxy)naphthalene was used instead of oa 6-bromo-2-(2-methylbutyloxy)naphthalene in Example 51 (1) to obtain Compound No.393 of the present invention.

| | | | | |
|---|---|---|---|---|
| Elemental analysis,% | Observed | C: 77.61 | H: 8.51 | F: 7.41 |
| | Theoretical | C: 77.86 | H: 8.78 | F: 7.25 |

Example 52 [Compound No.161]

(1) Into 200 ml of anhydrous THF, were dissolved 15.2 g of o-difluorobenzene, and cooled with dry ice-acetone bath to a temperature below -60°C. Then, 90 ml of 15% solution of n-butyl lithium in hexane were added dropwise thereto at a temperature below -60°C, and stirring was continued for 3 hours in that conditions. Subsequently, 25 ml of n-decyl aldehyde were added dropwise at a temperature below -60°C, and stirring was continued for 30 minutes in that conditions. Thereafter, the dry ice-acetone bath was removed, and then stirring was continued until the temperature reached 0°C. Water and then 1N-hydrochloric acid were added, and subsequently the product was extracted with diethylether, followed by washing the ether phase with water and removing the ether to obtain 35.5 g of a liquid compound (a′) of the formula:

NON-CH(OH)-FPhF-H.

(2) To 1 l of toluene, 35.5 g of the compound (a′) and 4.0 g of p-toluenesulfonic acid were added, and heated for 3 days to reflux with removing formed water. Thereafter, toluene phase was washed with water, followed by removing the toluene and then distiling under reduced pressure to obtain 17.6 g of a liquid compound (b′) of the formula:

OCT-CH=CH-FPhF-H.

(3) Into 300 ml of ethanol, were dissolved 17.6 g of the compound (b′), followed by carrying out hydrogenation using 2.0 g of 5% palladium carbon. After confirming that no absorption of hydrogen was observed, the catalyst was filtered off, and then the ethanol was removed to obtain 17.5 g of liquid 1-n-decyl-2,3-difluorobenzene.

(4) Into 100 ml of anhydrous THF, were dissolved 8.7 g of 1-n-decyl-2,3-difluorobenzene, and cooled with dry ice-acetone bath to a temperature below -60°C. Then, 90 ml of 15% solution of n-butyl lithium in hexane were added dropwise thereto at a temperature below -60°C and stirring was continued for 3 hours in that conditions, followed by adding 4.3 g of trimethyl borate thereto at a temperature below -60°C and stirring was continued for 30 minutes in that conditions. Thereafter, the dry ice-acetone bath was removed, and then stirring was continued until the temperature reached 0°C. Water and then 1N-hydrochloric acid were added, and subsequently the product was extracted with diethylether, followed by washing the ether phase with water and removing the ether to obtain 9.3 g of white crystalline 4-n-decyl-2,3-difluorophenyl boric acid.

(5) In 60 ml of triethylamine, within nitrogen atmosphere, 2.0 g of 4-n-decyl-2,3-difluorophenyl boric acid and 2.0 g of oa 6-bromo-2-(2-methylbutyloxy)naphthalene were reacted for 10 hours at reflux temperature in the presence of 155 mg of tetrakistriphenylphosphine palladium (0) and 155 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.6 g of white crystalline Compound No.161 of the present invention.

| | | | | |
|---|---|---|---|---|
| Elemental analysis,% | Observed | C: 79.95 | H: 8.39 | F: 8.31 |
| | Theoretical | C: 79.83 | H: 8.58 | F: 8.16 |

Example 53 [Compound No.164]

Example 52 was repeated except that oa 6-bromo-2-(1-methylheptyloxy)-naphthalene was used instead of oa 6-bromo-2-(2-methylbutyloxy)-naphthalene to obtain white crystalline Compound No.164 of the present invention.

| | | | | |
|---|---|---|---|---|
| Elemental analysis,% | Observed | C: 80.09 | H: 9.24 | F: 7.24 |
| | Theoretical | C: 80.31 | H: 9.06 | F: 7.48 |

Example 54 [Compound No.395]

(1) Into 220 ml of anhydrous THF, were dissolved 16.6 g of o-difluorobenzene, and cooled with dry ice-acetone bath to a temperature below -60°C. Then, 99 ml of 15% solution of n-butyl lithium in hexane were added dropwise thereto at a temperature below -60°C, and stirring was continued for 3 hours in that conditions, followed by adding 20.0 g of trimethyl borate thereto at a temperature below -60°C and stirring was continued for 30 minutes in that conditions. Thereafter, the dry ice-acetone bath was removed, and then stirring was continued until the temperature reached 0°C. Water and then 1N-hydrochloric acid were added, and subsequently the product was extracted with diethylether, followed by washing the ether phase with water and removing the ether to obtain 21.1 g of white solid 2,3-difluorophenyl boric acid.
(2) In 120 ml of triethylamine, within nitrogen atmosphere, 2.2 g of 2,3-difluorophenyl boric acid and 5.0 g of 6-bromo-2-n-decyloxynaphthalene prepared by reacting n-decyl bromide with alkali metal 6-bromo-2-naphtholate were reacted for 10 hours at reflux temperature in the presence of 318 mg of tetrakistriphenylphosphine palladium(0) and 318 mg of triphenylphosphine. After reaction, the triethyl amine was removed and water was added. The product was extracted with toluene. The toluene phase was washed with 1N-hydrochloric acid and then with water, and the toluene was removed to obtain 4.7 g of 2-(2,3-difluorophenyl)-6-n-decyloxynaphthalene.
(3) Into 100 ml of anhydrous THF, were dissolved 4.7 g of 2-(2,3-difluoro-phenyl)-6-n-decyloxynaphthalene, and cooled with dry ice-acetone bath to a temperature below -60°C. Then, 8 ml of 15% solution of n-butyl lithium in hexane were added dropwise thereto at a temperature below -60°C, and stirring was continued for 3 hours in that conditions, followed by adding 1.9 g of trimethyl borate thereto at a temperature below -60°C and stirring was continued for 30 minutes in that conditions. Thereafter, the dry ice-acetone bath was removed, and then stirring was continued until the temperature reached 0°C. Water and then 1N-hydrochloric acid were added, and subsequently the product was extracted with diethylether, followed by washing the ether phase with water and removing the ether to obtain 4.8 g of white solid compound (c′) of the formula :

$$DEC\text{-}O\text{-}NAP\text{-}FPhF\text{-}B(OH)_2.$$

(4) Into diethyl ether solution containing the compound (c′) dissolved therein, were added 25 ml of 10% aqueous hydrogen peroxide, and stirred for 1 hour at the room temperature and then for 2 hours at the reflux temperature. Thereafter, the ethanol phase was washed with water, and then the ether was removed to obtain 3.5 g of 2-(2,3-difluoro-4-hydroxyphenyl)-6-n-decyloxynaphthalene.
(5) To 100 ml of ethanol, were added 2.5 g of 2-(2,3-difluoro-4-hydroxyphenyl)-6-n-decyloxynaphthalene and 0.45 g of potassium hydrooxide, and stirred to form homogeneous solution. Then, 1.6 g of oa 2-methylbutyl p-toluenesulfonate were added thereto and heated for 20 hours to reflux. Thereafter, the ethanol was removed and water was added. The resulting solid was filtered, dried, dissolved into toluene, treated with silica gel column and then recrystalized thrice with ethanol to obtain 1.4 g of white solid Compound No.395 of the present invention.

| | | | | |
|---|---|---|---|---|
| Elemental analysis,% | Observed | C: 77.35 | H: 8.14 | F: 7.93 |
| | Theoretical | C: 77.18 | H: 8.30 | F: 7.88 |

Example 55 [Compound No.254]

(1) In 100 ml of triethylamine, within nitrogen atmosphere, 5.3 g of 1-decyne and 10.0 g of 6-bromo-2-benzyloxynaphthalene prepared by reacting benzyl chloride with alkali metal 6-bromo-2-naphtholate were reacted for 8 hours at reflux temperature in the presence of 28 mg of cuprous iodide(I), 112 mg of dichlorobistriphenylphosphine palladium(II) and 224 mg of triphenyl-phosphine. After reaction, the triethylamine was removed, and the product was extracted with toluene. The toluene phase was washed with 1N-hydrochloric acid and then with water, followed by removing the toluene. The product was recrystalized with ethanol to obtain 10.0 g of a compound (d′) of the formula:

$$OCT\text{-}\#\text{-}NAP\text{-}OCH_2\text{-}Ph\text{-}H.$$

(2) Within an atmosphere of hydrogen, in 150 ml of ethanol, 10.0 g of the compound (d′) was hydrogenized and hydrogenolyzed using 0.5 g of 5% palladium carbon. After confirming that no absorption of hydrogen was observed, the catalyst was filtered off, followed by removing the ethanol and then recrystalizing the resulting solid with hexane to obtain 6.5 g of 6-n-decyl-2-hydroxynaphthalene.

(3) Into 100 ml of anhydrous pyridine, were dissolved 6.5 g of 6-n-decyl-2-hydroxynaphthalene, and cooled to a temperature below 10°C. Then, 7.1 g of TFMSA was added dropwise at a temperature below 10°C, followed by stirring for 24 hours at the room temperature. The resulting solution was thrown into iced water and acidified with hydrochloric acid, and then extracted with hexane. The hexane phase was washed with water and the hexane was removed to obtain an oily compound (e′) of the formula :

$$DEC\text{-}NAP\text{-}O\text{-}SO_2CF_3.$$

(4) Example 54 (2)-(5) were repeated except that the compound (e′) was used instead of 6-bromo-2-n-decyloxynaphthalene in Example 54 (2) to obtain Compound No.254 of the present invention.

| | | | | |
|---|---|---|---|---|
| Elemental analysis,% | Observed | C: 79.69 | H: 8.71 | F: 8.32 |
| | Theoretical | C: 79.83 | H: 8.58 | F: 8.16 |

Example 56 [Compound No.255]

Example 54 (2)-(5) were repeated except that the compound (e′) was used instead of 6-bromo-2-n-decyloxynaphthalene in Example 54 (2) and oa 1-methylheptyl p-toluenesulfonate was used instead of oa 2-methylbutyl p-toluenesulfonate in Example 54 (5) to obtain Compound No.255 of the present invention.

| | | | | |
|---|---|---|---|---|
| Elemental analysis,% | Observed | C: 80.53 | H: 8.82 | F: 7.33 |
| | Theoretical | C: 80.31 | H: 9.06 | F: 7.48 |

Example 57 [Compound No.401]

(1) To 30 ml of diethyl ether solution containing 5.7 g of trimethyl borate dissolved therein and cooled to a temperature below -30°C, were added dropwise at a temperature below -30°C 75 ml of ether solution of a Grignard's reagent prepared from 15 g of 4-n-octyloxy-3-fluorobromobenzene, prepared by reacting n-octyl bromide with alkali metal 4-bromo-2-fluorophenolate, followed by continuing stirring for 1 hour in that conditions and then with removing the refrigerant until the temperature reached the room temperature. The resulting product was thrown into iced water, and the separated organic phase was washed with 1N-hydrochloric acid and then with water, followed by removing the ether to obtain 11.7 g of 4-n-octyloxy-3-fluorophenyl boric acid.

(2) In 60 ml of triethylamine, within nitrogen atmosphere, 1.5 g of 4-n-octyloxy-3-fluorophenyl boric acid and 1.9 g of oa 6-bromo-2-(1-metylheptyloxy)-naphthalene were reacted for 10 hours at reflux temperature in the presence of 129 mg of tetrakistriphenylphosphine palladium (0) and 129 mg of triphenylphosphine. After reaction, the triethyl amine was removed and water was added. The product was extracted with toluene. The toluene phase was washed with 1N-hydrochloric acid and then with water, treated with silica gel column and then recrystalized thrice with ethanol to obtain 1.5 g of white crystalline Compound No.401 of the present invention.

| | | | | |
|---|---|---|---|---|
| Elemental analysis,% | Observed | C: 80.14 | H: 8.88 | F: 4.21 |
| | Theoretical | C: 80.33 | H: 9.00 | F: 3.97 |

Example 58 [Compound No.832]

(1) To diethyl ether solution of a Grignard's reagent prepared from 10.0 g of 6-bromo-2-benzyloxynaphthalene, prepared by reacting benzyl chloride with alkali metal 6-bromo-2-naphtholate, were added 7.6 g of 2,5-dibromopyridine, and the atmosphere was substituted with nitrogen. After cooling with ice bath to a temperature below 10°C, 193 mg of dichloro[1,4-bis(diphenylphosphino)butane]palladium(II) were added and sitirred for 1 hour in that conditions and then 2 hours without ice bath. After addition of toluene, the organic phase was washed with water and the solvent was removed. The resulting solid was washed with methanol and dried to obtain 7.5 g of 6-(5-bromopyridine-2-yl)-2-benzyloxynaphthalene.
(2) In 200 ml of triethylamine, within nitrogen atmosphere, 3.2 g of 1-decyne and 7.5 g of 6-(5-dibromopyridine-2-yl)-2-benzyloxynaphthalene were reacted for 8 hours at reflux temperature in the presence of 17 mg of cuprous iodide(I), 68 mg of dichlorobistriphenylphosphine palladium(II) and 136 mg of triphenylphosphine. After reaction, the temperature was reduced to the room temperature and the triethylamine was removed, followed by extracting the product with toluene. The toluene phase was washed with 1N-hydrochloric acid and then with water, and recrystalized with ethanol to obtain 5.2 g of a compound (f′) of the formula:

OCT-#-Pyr>-NAP-$OCH_2$-Ph-H.

(3) In 300 ml of ethanol, 5.2 g of the compound (f′) was hydrogenized and hydrogenolyzed using 0.5 g of 5% palladium carbon. After confirming that no absorption of hydrogen was observed, the catalyst was filtered off, followed by removing the ethanol to obtain 3.4 g of 6-(5-n-decylpyridine -2-yl)-2-naphthol.
(4) Into 100 ml of anhydrous pyridine, were dissolved 3.4 g of 6-(5-n-decylpyridine-2-yl)-2-naphthol, and cooled to a temperature below 10°C with ice bath. Then, 3.2 g of TFMSA was added dropwise at a temperature below 10°C, followed by stirring for 1 hour in that conditions and then for 24 hours at the room temperature. The resulting solution was thrown into iced water and then extracted with toluene. The toluene phase was washed successively with 1N-hydrochloric acid, water, aqueous sodium hydrogen carbonate and then water, followed by removing the toluene and then recrystalizing the product with hexane to obtain 3.9 g of 6-(5-n-decylpyridine-2-yl)-2-naphthol trifluoromethanesulfonate.
(5) Into an autoclave, were charged 3.9 g of 6-(5-n-decylpyridine -2-yl)-2-naphthol trifluoromethanesulfonate, 21 ml of methanol, 1.8 ml of triethylamine and 55 mg of dichlorobistriphenylphosphine palladium(II), and reacted under pressure of 20kg/$cm^2$ carbon monooxide. After reaction, the solvent was removed and the product was extracted with toluene. The toluene phase was washed with 1N-hydrochloric acid and then with water, followed by removing the toluene and recrystalizing the product with methanol to obtain 2.7 g of methyl 6-(5-n-decylpyridine-2-yl)-2-naphthoate.
(6) Into 150 ml of ethanol, were dissolved 2.7 g of methyl 6-(5-n-decylpyridine-2-yl)-2-naphthoate, and solution of 1.0 g of potassium hydroxide dissolved in 10 ml was added thereto, followed by heating for 2 hours to reflux. Thereafter, the ethanol was removed, water was added, followed by acidifying the product with hydrochloric acid and then filtering the resulting precipitates to obtain 2.5 g of 6-(5-n-decylpyridine-2-yl)2-naphthoic acid.
(7) Into 50 ml of anhydrous THF, were dissolved 2.5 g of 6-(5-n-decylpyridine-2-yl)-2-naphthoic acid, 2.0 g of triphenylphosphine and 1.0 g of oa 1-methyloctanol, and cooled with ice bath to a temperature below 10°C. To this solution, was added dropwise 1.2 g of diethyl azodicarboxylate at a temperature below 10°C, and stirring was continued for 1 hour in that conditions and then for 24 hours at the room temperature, followed by removing the solvent. Hexane-soluble matter was dissolved in toluene and purified with silica gel column, and then recrystalized thrice with ethanol to obtain Compound No.832 of the present invention.

| Elemental analysis,% | Observed | C: 81.19 | H: 9.52 | N: 2.65 |
|---|---|---|---|---|
| | Theoretical | C: 81.44 | H: 9.38 | N: 2.79 |

Example 59 [Compound No.714]

In 60 ml of triethylamine, within nitrogen atmosphere, 2.0 g of oa trifluoromethanesulfonate of 2-methylbutyl 6-hydroxy-2-naphthoate and 1.5 g of 2,3-difluoro-4-n-decyloxyphenyl boric acid prepared in Example 50 (1) were reacted for 10 hours at reflux temperature in the presence of 110 mg of tetrakistriphenylphosphine palladium(0) and 110 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.2 g of white crystalline Compound

No.714 of the present invention.

| | | | | |
|---|---|---|---|---|
| Elemental analysis,% | Observed | C: 75.13 | H: 8.01 | F: 7.27 |
| | Theoretical | C: 75.30 | H: 7.84 | F: 7.45 |

Example 60 [Compound No.715]

Example 59 was repeated except that oa trifluoromethanesulfonate of 1-methylheptyl 6-hydroxy-2-naphthoate was used instead of oa trifluoromethanesulfonate of 2-methylbutyl 6-hydroxy-2-naphthoate to obtain Compound No.715 of the present invention.

| | | | | |
|---|---|---|---|---|
| Elemental analysis,% | Observed | C: 76.21 | H: 8.44 | F: 6.65 |
| | Theoretical | C: 76.09 | H: 8.33 | F: 6.88 |

Example 61 [Compound No.839]

In 60 ml of triethylamine, within nitrogen atmosphere, 2.2 g of oa trifluoromethanesulfonate of 1-methylheptyl 6-hydroxy-2-naphthoate and 1.5 g of 4-n-decyl-2,3-difluorophenyl boric acid prepared in Example 52 (4) were reacted for 10 hours at reflux temperature in the presence of 116 mg of tetrakistriphenylphosphine palladium(0) and 116 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.3 g of white crystalline Compound No.839 of the present invention.

| | | | | |
|---|---|---|---|---|
| Elemental analysis,% | Observed | C: 78.19 | H: 8.51 | N: 7.28 |
| | Theoretical | C: 78.36 | H: 8.58 | N: 7.09 |

Example 62 [Compound No.764]

(1) Into 100 ml of anhydrous THF, were dissolved 7.0 g of N,N,N′,N′-tetramethylmethylenediamine and 8.0 g of 2-(2,3-difluorophenyl)-6-n-decyloxynaphthalene as prepared in Example 54 (2), and cooled with dry ice-acetone bath to a temperature below -60°C. Then, 14 ml of 15% solution of n-butyl lithium in hexane were added dropwise thereto at a temperature below -60°C, and stirring was continued for 3 hours in that conditions, followed by introducing carbon dioxide at a temperature below -60°C. Thereafter, stirring was continued without dry ice-acetone bath until the temperature reached 0°C. Water and then 1N-hydrochloric acid were added, and subsequently the product was extracted with diethylether, followed by washing the ether phase with water and removing the ether to obtain 6.7 g of white solid 2-(2,3-difluoro-4-carboxyphenyl)-6-n-decyloxynaphthalene.

(2) Into 50 ml of anhydrous THF, were dissolved 2.0 g of 2-(2,3-difluoro-4-carboxyphenyl)-6-n-decyloxynaphthalene, 1.4 g of triphenylphosphine and 0.6 g of oa 4-methylhexanol, and cooled with ice bath to a temperature below 10°C. To this solution, was added dropwise 0.9 g of diethyl azodicarboxylate at a temperature below 10°C, and stirring was continued for 1 hour in that conditions and then for 24 hours at the room temperature, followed by removing the solvent. Hexane-soluble matter was dissolved in toluene and purified with silica gel column, and then recrystalized thrice with ethanol to obtain Compound No.764 of the present invention.

| | | | | |
|---|---|---|---|---|
| Elemental analysis,% | Observed | C: 75.68 | H: 8.31 | F: 7.14 |
| | Theoretical | C: 75.84 | H: 8.18 | F: 7.06 |

Example 63 [Compound No.765]

Example 62 was repeated except that oa 2-methylbutanol was used instead of oa 4-methylhexanol to obtain Compound No.765 of the present invention.

| | | | | |
|---|---|---|---|---|
| Elemental analysis,% | Observed | C: 75.17 | H: 7.99 | F: 7.40 |
| | Theoretical | C: 75.30 | H: 7.84 | F: 7.45 |

Example 64 [Compound No.766]

Example 62 was repeated except that oa 1-methylheptanol was used instead of oa 4-methylhexanol to obtain Compound No.766 of the present invention.

| | | | | |
|---|---|---|---|---|
| Elemental analysis,% | Observed | C: 76.13 | H: 8.19 | F: 6.75 |
| | Theoretical | C: 76.09 | H: 8.33 | F: 6.88 |

Example 65 [Compound No.912]

(1) In 100 ml of triethylamine, within nitrogen atmosphere, 1.9 g of 2,3-difluorophenyl boric acid and 4.0 g of the compound (e') [6-n-decyl-2-hydroxynaphthalene trifluoromethanesulfonate] prepared in Example 55 (3) were reacted for 10 hours at reflux temperature in the presence of 278 mg of tetrakistriphenylphosphine palladium (0) and 278 mg of triphenylphosphine. After reaction, the triethyl amine was removed and water was added. The product was extracted with toluene. The toluene phase was washed with 1N-hydrochloric acid and then with water, and the toluene was removed to obtain 3.9 g of 2-(2,3-difluorophenyl)-6-n-decylnaphthalene.
(2) Example 62 (1) was repeated except that 2-(2,3-difluorophenyl )-6-n-decylnaphthalene was used instead of 2-(2,3-difluorophenyl)-6-n-decyloxynaphthalene to obtain white solid 2-(2,3-difluoro-4-carboxyphenyl)-6-n-decylnaphthalene.
(3) Into 50 ml of anhydrous THF, were dissolved 1.5 g of 2-(2,3-difluoro-4-carboxyphenyl)-6-n-decylnaphthalene, 1.1 g of triphenylphosphine and 0.5 g of oa 1-methyloctanol, and cooled with ice bath to a temperature below 10°C. To this solution, was added dropwise 0.7 g of diethyl azodicarboxylate at a temperature below 10°C, and stirring was continued for 1 hour in that conditions and then for 24 hours at the room temperature, followed by removing the solvent. Hexane-soluble matter was dissolved in toluene and purified with silica gel column, and then recrystalized thrice with ethanol to obtain Compound No.912 of the present invention.

| | | | | |
|---|---|---|---|---|
| Elemental analysis,% | Observed | C: 78.14 | H: 8.71 | F: 7.23 |
| | Theoretical | C: 78.36 | H: 8.58 | F: 7.09 |

Example 66 [Compound No.834]

(1) Example 58 (1)-(6) were repeated except that 1-bromo -3-fluoro-4-iodobenzene was used instead of 2,5-dibromopyridine to obtain 6-(4-n-decyl-2-fluorophenyl)-2-naphthoic acid.
(2) Into 50 ml of anhydrous THF, were dissolved 1.5 g of 6-(4-n-decyl-2-fluorophenyl)-2-naphthoic acid, 1.5 g of triphenylphosphine and 0.7 g of oa 4-methylhexanol, and cooled with ice bath to a temperature below 10°C. To this solution, was added dropwise 1.0 g of diethyl azodicarboxylate at a temperature below 10°C, and stirring was continued for 1 hour in that conditions and then for 24 hours at the room temperature, followed by removing the solvent. Hexane-soluble matter was dissolved in toluene and purified with silica gel column, and then recrystalized thrice with ethanol to obtain white crystalline Compound No.834 of the present invention.

| | | | | |
|---|---|---|---|---|
| Elemental analysis,% | Observed | C: 81.02 | H: 8.79 | F: 3.85 |
| | Theoretical | C: 80.95 | H: 8.93 | F: 3.77 |

Example 67 [Compound No.47]

In 60 ml of triethylamine, within nitrogen atmosphere, 2.2 g of 2-n-octyloxy-6-bromonaphthalene and 2.0 g of 4-n-decyl-2,3-difluorophenyl boric acid prepared in Example 52 (4) were reacted for 10 hours at reflux temperature in the presence of 155 mg of tetrakistriphenylphosphine palladium(0) and 155 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.7 g of white crystalline Compound No.47 of the present invention.

| | | | | |
|---|---|---|---|---|
| Elemental analysis,% | Observed | C: 80.09 | H: 9.20 | F: 7.59 |
| | Theoretical | C: 80.31 | H: 9.06 | F: 7.48 |

Example 68 [Compound No.50]

(1) In 50 ml of triethylamine, within nitrogen atmosphere, 4.6 g of 1-decyne and 10.0 g of 1-bromo-3-fluoro-4-iodobenzene were reacted for 8 hours at the room temperature in the presence of 18 mg of cuprous iodide(I) and 72 mg of dichlorobistriphenylphosphine palladium(II). After reaction, the triethylamine was removed, and the product was extracted with hexane. The hexane phase was washed with 1N-hydrochloric acid and then with water, followed by removing the hexane to obtain 9.0 g of a compound (g′) of the formula:

OCT-#-FPh-Br.

(2) To diethyl ether solution of a Grignard's reagent prepared from 10.0 g of 6-bromo-2-octyloxy-naphthalene, were added 4.6 g of the compound (g′), and the atmosphere was substituted with nitrogen. After cooling with ice bath to a temperature below 10°C, 90 mg of dichloro[1,4-bis(diphenylphosphino)butane]palladium(II) were added and stirred for 1 hour in that conditions and then 5 hours without ice bath, followed by adding water under cooling with ice bath. After addition of toluene, the organic phase was washed with water and the solvent was removed. The resulting solid was dissolved into ethanol, and hydrogenated within an atmosphere of hydrogen using 5% palladium carbon. After confirming that no absorption of hydrogen was observed, the catalyst was removed and the ethanol was removed. The resulting solid was dissolved into toluene, purified with silica gel column, and recrystalized thrice with ethanol to obtain 1.6 g of white crystalline Compound No.50 of the present invention.

| | | | | |
|---|---|---|---|---|
| Elemental analysis,% | Observed | C: 83.41 | H: 9.62 | F: 3.65 |
| | Theoretical | C: 83.26 | H: 9.59 | F: 3.88 |

Example 69 [Compound No.69]

(1) To 30 ml of diethyl ether solution of a Grignard's reagent prepared from 3.0 g of 6-bromo-2-decyloxynaphthalene, were added 2.6 g of p-n-octyliodobenzene prepared by diazotizing p-n-octylaniline with sodium nitrite followed by reacting potassium iodide, and the atmosphere was substituted with nitrogen. After cooling with ice bath to a temperature below 10°C, 50 mg of dichloro[1,4-bis(diphenylphosphino)butane]palladium(II) were added and stirred for 1 hour in that conditions and then 5 hours without ice bath, followed by adding water under cooling with ice bath. After addition of toluene, the organic phase was washed with water and the solvent was removed. The resulting solid was dissolved into toluene, purified with silica gel column, and recrystalized thrice with ethanol to obtain 1.6 g of white crystalline Compound No.69 of the present invention.

| | | | |
|---|---|---|---|
| Elemental analysis,% | Observed | C: 86.19 | H: 10.36 |
| | Theoretical | C: 86.44 | H: 10.17 |

Example 70 [Compound No.53]

Example 8 (2) was repeated except that 8-bromo-1-octene was used instead of n-heptylbromide to obtain Compound No.53 of the present invention.

| Elemental analysis,% | Observed | C: 81.33 | H: 9.65 | N: 5.83 |
|---|---|---|---|---|
| | Theoretical | C: 81.36 | H: 9.32 | N: 5.93 |

Example 71 [Compound No.56]

(1) To diethyl ether solution of a Grignard's reagent prepared from 10.0 g of 6-bromo-2-benzyloxynaphthalene prepared by reacting benzyl chloride with alkali metal 6-bromo-2-naphtholate, were added 7.6 g of 2,5-dibromopyridine, and the atmosphere was substituted with nitrogen. After cooling with ice bath to a temperature below 10°C, 193 mg of dichloro[1,4-bis(diphenylphosphino)butane]palladium(II) were added and stirred for 1 hour in that conditions and then 2 hours without ice bath, followed by adding water under cooling with ice bath. After addition of toluene, the organic phase was washed with water and the solvent was removed. The resulting solid was washed with methanol and then dried to obtain 7.5 g of 6-(5-bromopyridine-2-yl)-2-benzyloxynaphthalene.

(2) In 200 ml of triethylamine, within nitrogen atmosphere, 3.2 g of 1-decyne and 7.5 g of 6-(5-bromopyridine-2-yl )-2-benzyloxynaphthalene were reacted for 8 hours at reflux temperature in the presence of 17 mg of cuprous iodide(I), 68 mg of dichlorobistriphenylphosphine palladium (II) and 136 mg of triphenylphosphine. After reaction and cooling to the room temperature, the triethylamine was removed, and the product was extracted with toluene. The toluene phase was washed with 1N-hydrochloric acid and then with water, followed by recrystalizing the product with ethanol to obtain 5.2 g of a compound (h') of the formula:

OCT-#-Pyr>-NAP-$OCH_2$-Ph-H.

(3) Within an atmosphere of hydrogen, in 300 ml of ethanol, 5.2 g of the compound (h') was hydrogenized and hydrogenolyzed using 0.5 g of 5% palladium carbon. After confirming that no absorption of hydrogen was observed, the catalyst was filtered off, followed by removing the ethanol to obtain 3.4 g of 6-(5-n-decylpyridine-2-yl)-2-naphthol.

(4) To 30 ml of DMSO containing 1.0 g of 6-(5-n-decylpyridine-2-yl)-2-naphthol dissolved therein, were added 5 ml of aqueous solution of 0.13 g of sodium hydroxide, and stirred to obtain homogeneous solution. Then, 0.6 g of 8-bromo-1-octene was added and stirred fo 3 days at the room temperature. Thereafter, the resulting product was thrown into water and extracted with toluene. The toluene phase was washed with water, purified with silica gel column and recrystalized twice with ethanol to obtain white crystalline Compound No.56 of the present invention.

| Elemental analysis,% | Observed | C: 84.22 | H: 9.39 | N: 3.05 |
|---|---|---|---|---|
| | Theoretical | C: 84.08 | H: 9.55 | N: 2.97 |

Example 72 [Compound No.62]

(1) Example 71 (1)-(3) were repeated except that 1-bromo-3-fluoro-4-iodobenzene was used instead of 2,5-dibromopyridine in Example 71 (1) to obtain 6-(4-n-decyl-2-fluorophenyl)-2-naphthol.

(2) Example 71 (4) was repeated except that 6-(4-n-decyl-2-fluorophenyl)-2-naphthol was used instead of 6-(5-n-decylpyridine-2-yl)-2-naphthol to obtain Compound No.62 of the present invention.

| Elemental analysis,% | Observed | C: 83.75 | H: 9.23 | F: 3.62 |
|---|---|---|---|---|
| | Theoretical | C: 83.61 | H: 9.22 | F: 3.89 |

Example 73 [Compound No.63]

(1) Into 120 ml of anhydrous THF, were dissolved 10.0 g of 1-n-octyl-2,3-difluorobenzene, prepared by reacting o-difluorobenzene with n-butyllithium and subsequently with n-octylaldehyde followed by dehydration and then hydrogenation, and cooled with dry ice-acetone bath to a temperature below -60°C. Then, 30 ml of 15% solution of n-butyl lithium in hexane were added dropwise thereto at a temperature below -60°C, and stirring was continued for 3 hours in that conditions, followed by adding 5.5 g of trimethyl borate thereto at a temperature below -60°C and stirring was continued for 30 minutes in that conditions. Thereafter, the dry ice-acetone bath was removed, and then stirring was continued until the temperature reached

0°C. Water and then 1N-hydrochloric acid were added, and subsequently the product was extracted with diethylether, followed by washing the ether phase with water and removing the ether to obtain 10.7 g of white solid 4-n-octyl -2,3-difluorophenyl boric acid.

(2) In 60 ml of triethylamine, within nitrogen atmosphere, 1.5 g of 4-n-octyl-2,3-difluorophenyl boric acid and 2.0 g of 6-bromo-2-(9-decenyloxy)naphthalene prepared by reacting 10-bromo-1-decene with alkali metal 6-bromo-2-naphtholate were reacted for 10 hours at reflux temperature in the presence of 128 mg of tetrakistriphenylphosphinepalladium (0) and 128 mg of triphenylphosphine. After reaction, the triethyl amine was removed and the product was extracted with toluene. The toluene phase was washed with 1N-hydrochloric acid and then with water, purified with silica gel column and recrystalized thrice with ethanol to obtain 1.3 g of white crystalline Compound No.63 of the present invention.

| | | | | |
|---|---|---|---|---|
| Elemental analysis,% | Observed | C: 80.51 | H: 8.66 | F: 7.80 |
| | Theoretical | C: 80.63 | H: 8.70 | F: 7.51 |

Example 74 [Compound No.707]

In the same manner as Example 59 (2), 2.1 g of trifluoromethanesulfonate of octyl 6-hydroxy-2-naphthoate and 1.5 g of 2,3-difluoro-4-n-decyl-oxyphenyl boric acid were reacted and the reaction product was treated to obtain 1.2 g of white crystalline Compound No.707 of the present invention.

| | | | | |
|---|---|---|---|---|
| Elemental analysis,% | Observed | C: 76.18 | H: 8.20 | F: 7.01 |
| | Theoretical | C: 76.09 | H: 8.33 | F: 6.88 |

Example 75 [Compound No.725]

In 60 ml of triethylamine, within nitrogen atmosphere, 2.2 g of trifluoromethanesulfonate of octyl 6-hydroxy -2-naphthoate and 1.5 g of 4-n-decyloxy-3-fluorophenyl boric acid prepared by reaction of trimethyl borate with a Grignard's reagent prepared from 4-n-decyloxy-3-fluorobromobenzene followed by hydrolysis were reacted for 10 hours at reflux temperature in the presence of 117 mg of tetrakistriphenylphosphine palladium(0) and 117 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.2 g of white crystalline Compound No.725 of the present invention.

| | | | | |
|---|---|---|---|---|
| Elemental analysis,% | Observed | C: 78.41 | H: 8.92 | F: 3.71 |
| | Theoretical | C: 78.65 | H: 8.80 | F: 3.56 |

Example 76 [Compound No.757]

Into 50 ml of anhydrous THF, were dissolved 2.0 g of 2-(2,3-difluoro-4-carboxyphenyl)-6-n-decyloxynaphthalene, 1.4 g of triphenylphosphine and 0.7 g of n-octanol, and cooled with ice bath to a temperature below 10°C. To this solution, was added dropwise 0.9 g of diethyl azodicarboxylate at a temperature below 10°C, and stirring was continued for 1 hour in that conditions and then for 24 hours at the room temperature, followed by removing the solvent. Hexane-soluble matter was dissolved in toluene and purified with silica gel column, and then recrystalized thrice with ethanol to obtain Composed No.757 of the present invention.

| | | | | |
|---|---|---|---|---|
| Elemental analysis,% | Observed | C: 75.92 | H: 8.55 | F: 6.69 |
| | Theoretical | C: 76.09 | H: 8.33 | F: 6.88 |

Example 77 [Compound No.776]

In 60 ml of triethylamine, within nitrogen atmosphere, 1.8 g of 4-n-octyloxycarbonyl-3-fluoroiodobenzene

and 1.5 g of 6-n-decyloxynaphthalene-2-yl boric acid prepared by reaction of trimethyl borate with a Grignard's reagent prepared from 6-bromo-2-n-decyloxynaphthalene followed by hydrolysis were reacted for 10 hours at reflux temperature in the presence of 106 mg of tetrakistriphenylphosphine palladium(0) and 106 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.2 g of white crystalline Compound No.776 of the present invention.

| | | | | |
|---|---|---|---|---|
| Elemental analysis,% | Observed | C: 78.83 | H: 8.62 | F: 3.47 |
| | Theoretical | C: 78.65 | H: 8.80 | F: 3.56 |

Example 78 [Compound No.817]

In 60 ml of triethylamine, within nitrogen atmosphere, 2.2 g of trifluoromethanesulfonate of octyl 6-hydroxy -2-naphthoate and 1.5 g of 2,3-difluoro-4-n-decylphenyl boric acid were reacted for 10 hours at reflux temperature in the presence of 116 mg of tetrakistriphenylphosphine palladium(0) and 116 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.3 g of white crystalline Compound No.817 of the present invention.

| | | | | |
|---|---|---|---|---|
| Elemental analysis,% | Observed | C: 78.22 | H: 8.67 | F: 7.21 |
| | Theoretical | C: 78.36 | H: 8.58 | F: 7.09 |

Example 79 [Compound No.907]

Example 76 was repeated except that 2.0 g of 2-(2,3-difluoro-4-carboxyphenyl)-6-n-decyloxynaphthalene was substituted with 1.9 g of 2-(2,3-difluoro-4-carboxyphenyl)-6-n-decylnaphthalene to obtain 1.0 g of white crystalline Compound No.907 of the present invention.

| | | | | |
|---|---|---|---|---|
| Elemental analysis,% | Observed | C: 78.51 | H: 8.60 | F: 6.82 |
| | Theoretical | C: 78.36 | H: 8.58 | F: 7.09 |

Example 80 [Compound No.657]

In 60 ml of triethylamine, within nitrogen atmosphere, 2.0 g of 6-n-octyl-2-naphthol trifluoromethanesulfonate and 1.5 g of 4-n-nonyl-2,3-difluorophenyl boric acid were reacted for 10 hours at reflux temperature in the presence of 122 mg of tetrakistriphenylphosphine palladium (0) and 122 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.1 g of white crystalline Compound No.657 of the present invention.

| | | | | |
|---|---|---|---|---|
| Elemental analysis,% | Observed | C: 82.73 | H: 9.44 | F: 7.83 |
| | Theoretical | C: 82.84 | H: 9.21 | F: 7.95 |

Example 81 [Compound No.74]

(1) Into 80 ml of anhydrous THF, were dissolved 3.0 g of 6-bromo-2-naphthol, 3.9 g of triphenylphosphine and 2.2 g of oa 2-fluorooctanol prepared by reacting hydrogen fluoride-pyridine complex with oa 1,2-epoxyoctane, and cooled with ice bath to a temperature below 10°C. To this solution, was added dropwise 2.8 g of diethyl azodicarboxylate at a temperature below 10°C, and stirring was continued for 1 hour in that conditions and then for 24 hours at the room temperature, followed by removing the solvent. Hexane-soluble matter was dissolved in toluene and purified with silica gel column, and then recrystalized with methanol to obtain 3.7 g of oa 6-bromo-2-(2-fluoro-octyloxy)naphthalene.

(2) In 60 ml of triethylamine, within nitrogen atmosphere, 1.8 g of oa 6-bromo-2-(2-fluoro-octyloxy)naphthalene and 1.5 g of 4-n-decyl-2,3-difluorophenyl boric acid were reacted for 10 hours at reflux temperature in the presence of 116 mg of tetrakistriphenylphosphine palladium (0) and 116 mg of triphenylphosphine. After reaction, the triethyl amine was removed and water was added, followed by extracting the product with toluene. The toluene phase was washed with 1N-hydrochloric acid and then with water, purified with silica gel colmun, and recrystalized thrice with ethanol to obtain 1.1 g of white crystalline Compound No.74 of the present invention.

| | | | | |
|---|---|---|---|---|
| Elemental analysis,% | Observed | C: 77.41 | H: 8.52 | F: 10.97 |
| | Theoretical | C: 77.56 | H: 8.56 | F: 10.84 |

Examples 82-84

According to the following formulation (% by weight) shown in Table 12, liquid crystal compositions were prepared, having response speed as shown in Table 12 and being free from zigzag deffects.

Table 12

| Example No. | 82 | 83 | 84 |
|---|---|---|---|
| Compound No.764 | 10.0 | - | - |
| Compound No.20 | 25.0 | 25.0 | 18.0 |
| Compound No.10 | 25.0 | 25.0 | - |
| Compound No.714 | - | 10.0 | - |
| Compound No.101 | - | - | 18.0 |
| Compound No.308 | - | - | 18.0 |
| DEC-O-Ph-#-FPhF-COO-$(CH_2)_5$*CH(Me)Et | 2.5 | 2.5 | 4.5 |
| DEC-O-Ph-#-PhF-COO-$(CH_2)_5$*CH(Me)Et | 2.5 | 2.5 | 4.5 |
| DEC-O-Ph-#-PhF-COO-$(CH_2)_3$*CH(Me)Et | 2.5 | 2.5 | 4.5 |
| DOD-O-Ph-#-Ph-COO-$(CH_2)$*CH(Me)Et | 2.5 | 2.5 | 4.5 |
| DEC-< Pyr-#-FPhF-O-OCT | 10.0 | 10.0 | 9.0 |
| OCT-< Pyr-#-FPhF-O-DEC | 10.0 | 10.0 | 9.0 |
| DEC-O-Ph-#-Ph-$CH_2O$*CH($CF_3$)-HEX | 10.0 | 10.0 | 10.0 |
| Response Speed (micro second) | 39 | 44 | 52 |

Phase transition temperatures-of compounds and compositions of Examples 1-84 were as shown in Table 13, wherein the following abbreviated words are used.

Cry: crystal phase; $S_1$ : unidentified smectic phase;
$S_C$ : smectic C phase; $S_A$ : smectic A phase;
Nem: nematic phase; Ch :cholesteric phase;
Iso: isotropic liquid phase; ( ) :monotropic phase;
• : the phase is present; – : the phase is not present

Table 13

| Example No. | Compound No. | Cry | $S_1$ | $S_C$ ($S_C^*$) | $S_A$ | Nem (Ch) | Iso |
|---|---|---|---|---|---|---|---|
| 1 | 7 | · 78.5 | – | · 83.7 | · 90.1 | · 104.1 | · |
| 2 | 6 | · 84.0 | – | (· 78.8) | · 87.7 | · 102.8 | · |
| 3 | 1 | · 80.9 | – | – | – | · 99.9 | · |
| 4 | 2 | · 76.9 | – | – | – | · 100.2 | · |
| 5 | 3 | · 65.1 | – | (· 64.3) | – | · 101.4 | · |
| 6 | 4 | · 67.3 | – | · 70.9 | – | · 98.7 | · |
| 7 | 5 | · 66.1 | – | · 71.2 | – | · 99.3 | · |
| 8 | 10 | · 74.6 | – | · 86.1 | · 92.2 | · 99.3 | · |
| 9 | 11 | · 75.4 | – | · 89.6 | · 94.9 | · 101.8 | · |
| 10 | 12 | · 70.1 | – | · 86.8 | · 94.3 | · 99.1 | · |
| 11 | 16 | · 99.2 | – | · 117.8 | – | · 119.9 | · |
| 12 | 20 | · 83.6 | – | · 120.7 | · 121.5 | – | · |
| 13 | 13 | · 96.9 | – | · 112.3 | – | · 115.8 | · |
| 14 | 17 | · 91.7 | – | · 114.9 | – | – | · |
| 15 | 21 | · 84.4 | – | · 118.6 | · 118.9 | – | · |
| 16 | 14 | · 93.2 | – | · 114.5 | · 116.0 | · 117.6 | · |
| 17 | 18 | · 90.7 | – | · 115.7 | – | – | · |
| 18 | 15 | · 88.0 | – | · 111.7 | – | · 114.8 | · |
| 19 | 19 | · 78.2 | – | · 115.5 | · 116.0 | · 116.9 | · |
| 20 | 22 | · 70.0 | – | · 115.9 | – | – | · |
| 21 | 24 | · 86.8 | · 95.5 | – | · 125.5 | – | · |
| 22 | 36 | · 46.7 | | · 48.9 | · 68.7 | · 79.1 | · |
| 23 | 45 | · 58.3 | | (· 48.2) | · 81.0 | – | · |

Table 13 (continued)

| Example No. | Compound No. | Cry | S1 | SC ($S_C^*$) | SA | Nem (Ch) | Iso |
|---|---|---|---|---|---|---|---|
| 24(3) | 551 | · 69.7 | – | · 81.0 | – | – | · |
| 24(4) | 101 | · 74.0 | – | · 81.4 | · 88.3 | – | · |
| 25 | 201 | · 65.5 | – | · 69.5 | · 73.8 | · 81.6 | · |
| 26 | 202 | · 65.3 | – | · 68.6 | · 76.4 | · 79.8 | · |
| 27 | 204 | · 60.3 | – | · 71.2 | · 73.5 | · 82.1 | · |
| 28 | 210 | · 59.2 | – | · 72.5 | · 76.5 | · 83.6 | · |
| 29 | 222 | · 53.0 | – | · 54.2 | · 71.1 | · 72.8 | · |
| 30 | 224 | · 67.3 | – | (· 43.3) | · 89.8 | – | · |
| 31 | 243 | · 58.6 | – | | · 89.5 | – | · |
| 32 | 246 | · 37.9 | – | – | · 69.7 | · 79.1 | · |
| 33(3) | 602 | · 75.0 | – | – | · 112.9 | – | · |
| 33(4) | 248 | · 63.9 | – | – | · 104.2 | · 106.2 | · |
| 34 | 252 | · 78.0 | – | · 106.9 | · 122.7 | – | · |
| 35 | 309 | · 79.3 | – | · 101.9 | · 103.7 | · 106.6 | · |
| 36 | 321 | · 69.3 | – | · 85.8 | · 91.1 | · 98.5 | · |
| 37 | 324 | · 69.9 | – | · 86.4 | · 96.2 | · 100.0 | · |
| 38 | 337 | · 96.4 | – | · 104.5 | · 127.5 | – | · |
| 39 | 356 | · 88.1 | – | · 96.4 | · 122.3 | – | · |
| 40 | 362 | · 83.1 | – | · 94.3 | · 117.6 | – | · |
| 41 | 379 | · 70.0 | – | (· 59.5) | · 94.3 | · 126.7 | · |
| 42 | 383 | · 78.1 | – | – | · 91.3 | · 121.3 | · |
| 44 | 527 | · 37.0 | – | · 47.6 | – | · 74.9 | · |

Table 13 (continued)

| Example No. | Compound No. | Cry | | $S_1$ | | $S_C$ ($S_C^*$) | | $S_A$ | | Nem (Ch) | | Iso |
|---|---|---|---|---|---|---|---|---|---|---|---|---|
| 45 | 501 | · | 98.0 | – | | · | 118.8 | – | | · | 120.7 | · |
| 46 | 547 | · | | | | | | · | 143.0 | | | · |
| 47 | 515 | · | 29.1 | – | | – | | · | 83.6 | – | | · |
| 49 | 104 | · | 45.4 | – | | – | | (· | 33.2) | – | | · |
| 50 | 391 | · | 57.7 | – | | (· | 56.8) | – | | (· | 68.4) | · |
| 51 | 393 | · | 42.6 | – | | – | | – | | – | | · |
| 52 | 161 | · | 57.5 | – | | – | | – | | – | | · |
| 53 | 164 | · | 41.3 | – | | – | | – | | – | | · |
| 54 | 395 | · | 42.6 | – | | – | | – | | – | | · |
| 55 | 254 | · | 72.7 | – | | – | | – | | – | | · |
| 56 | 255 | · | 21.6 | – | | – | | – | | · | 53.0 | · |
| 57 | 401 | · | 55.3 | – | | – | | – | | – | | · |
| 58 | 832 | · | 48.0 | – | | – | | – | | – | | · |
| 59 | 714 | · | 29.8 | – | | – | | · | 71.0 | – | | · |
| 60 | 715 | · | 24.9 | – | | – | | – | | – | | · |
| 61 | 839 | · | 22.2 | – | | – | | – | | – | | · |
| 62 | 764 | · | 70.1 | – | | – | | (· | 69.3) | – | | · |
| 63 | 765 | · | 101.6 | – | | – | | – | | – | | · |
| 64 | 766 | · | 62.5 | – | | – | | – | | – | | · |
| 65 | 912 | · | 35.4 | – | | – | | – | | – | | · |
| 66 | 834 | · | 28.4 | – | | – | | – | | – | | · |
| 67 | 47 | · | 40.6 | – | | · | 40.9 | · | 78.6 | · | 79.4 | · |
| 68 | 50 | · | 59.7 | (· | 58.4) | – | | · | 103.5 | – | | · |

Table 13 (continued)

| Example No. | Compound No. | Phase transition temperatures (°C) Cry | | $S_1$ | | $S_C$ ($S_C^*$) | | $S_A$ | | Nem (Ch) | | Iso |
|---|---|---|---|---|---|---|---|---|---|---|---|---|
| 69 | 69 | · | 72.0 | · | 106.8 | – | | · | 128.3 | – | | · |
| 70 | 53 | · | 68.5 | – | | · | 73.8 | · | 89.2 | · | 95.3 | · |
| 71 | 56 | · | 66.0 | – | | · | 107.5 | · | 111.0 | – | | · |
| 72 | 62 | · | 42.2 | – | | – | | · | 62.7 | · | 70.6 | · |
| 73 | 63 | · | 39.0 | – | | – | | · | 67.6 | – | | · |
| 74 | 707 | · | 61.2 | – | | – | | · | 79.6 | – | | · |
| 75 | 725 | · | 73.5 | – | | – | | · | 89.1 | – | | · |
| 76 | 757 | · | 60.4 | – | | – | | · | 83.7 | – | | · |
| 77 | 776 | · | 63.2 | – | | – | | · | 103.4 | – | | · |
| 78 | 817 | · | <RT | – | | – | | – | | · | 68.2 | · |
| 79 | 907 | · | 62.1 | – | | – | | (· | 50.3) | – | | · |
| 80 | 657 | · | 51.8 | – | | – | | (· | 46.0) | (· | 47.8) | · |
| 81 | 74 | · | 32.7 | – | | · | 35.1 | · | 67.5 | · | 74.2 | · |
| – | 397 | · | 15.4 | – | | – | | – | | – | | · |
| 82 | | · | <RT | – | | (· | 45.2) | · | 80.1 | – | | · |
| 83 | | · | <RT | – | | (· | 50.5) | · | 80.6 | – | | · |
| 84 | | · | <RT | – | | (· | 39.1) | · | 80.8 | – | | · |

(Note) <RT : below room temperature

IR ($cm^{-1}$) and NMR (ppm) spectra of some of the compounds of Examples 25-80 are as follows:

Example 25, Compound 201

IR 2922 2854 1638 1524 1464 1321 1301 1100 895 799
NMR 0.84-0.95(m,6H) 1.18-1.45(m,18H) 1.45-1.57(m,2H) 1.63-1.79(m,2H) 1.79-1.91(m,2H) 2.78(t,2H) 4.09 (t,2H) 6.78-6.87(m,1H) 7.15-7.23(m,1H) 7.36(dd,1H) 7.57-7.65 (m,2H) 7.82(t,2H) 7.93(s,1H)
$^{19}$F-NMR -66.0(dd,1F) -83.1(dd,1F)

Example 29, Compound 222

IR 2922 2854 1640 1524 1464 1319 1301 1098 895 814 799
NMR 0.88(t,3H) 1.21-1.55(m,20H) 1.62-1.78(m,2H) 1.78-1.92(m,2H) 2.00-2.11(m,2H) 2.78(t,2H) 4.08(t,2H) 4.92-5.06(m,2H) 5.73-5.91(m,1H) 6.79-6.88(m,1H) 7.15-7.24 (m,1H) 7.37(dd,1H) 7.57-7.66(m,2H) 7.82(t,2H) 7.94(s,1H)
$^{19}$F-NMR -66.0(dd,1F) -83.1(dd,1F)

Example 30, Compound 224

IR 2922 2854 1528 1470 1307 1270 1129 866 804
NMR 0.84-0.95(m,6H) 1.18-1.43(m,20H) 1.43-1.55 (m,2H) 1.65-1.78(m,2H) 1.78-1.91(m,2H) 2.77(t,2H) 4.06 (t,2H) 7.03(t,1H) 7.33-7.48(m,3H) 7.60-7.67(m,2H) 7.82 (m,2H) 7.93(s,1H)
$^{19}$F-NMR -58.6(t,1F)

Example 32, Compound 246

IR 2922 2854 1626 1516 1468 1317 1238 1166 1108 832
NMR 0.83-0.94(m,6H) 1.18-1.53(m,24H) 1.64-1.88 (m,4H) 2.78(t,2H) 3.99(t,2H) 6.68-6.82(m,2H) 7.35(d,1H) 7.43 (t,1H) 7.58-7.65(m,2H) 7.80(t,2H) 8.93(s,1H)
$^{19}$F-NMR -39.9(t,1F)

Example 33(3), Compound 602

IR 2922 2854 2218 1580 1551 1439 1288 897 820
NMR 0.84-0.96(m,6H) 1.21-1.40(m,14H) 1.40-1.56(m,4H) 1.56-1.70(m,2H) 1.75-1.89(m,2H) 2.45(t,2H) 4.06(t,2H) 7.47(d,1H) 7.81-7.96(m,3H) 8.42-8.51(m,3H) 8.80(s,1H)

Example 33(4), Compound 248

IR 2924 2856 1549 1441 1284 1009 905 812
NMR 0.82-0.93(m,6H) 1.20-1.43(m,20H) 1.43-1.52(m,2H) 1.64-1.77(m,2H) 1.77-1.89(m,2H) 2.75(t,2H) 4.09(t,2H) 7.35(dd,1H) 7.62(s,1H) 7.86(t,2H) 8.42(dd,1H) 8.48(s,2H) 8.81(d,1H)

Example 34, Compound 252

IR 2922 2854 1607 1520 1502 1473 1286 1257 890 839 818
NMR 0.83-0.92(m,6H) 1.17-1.42(m,22H) 1.42-1.54(m,2H) 1.55-1.86(m,4H) 2.77(t,2H) 3.99(t,2H) 7.00(d,2H) 7.34 (dd,1H) 7.46(d,1H) 7.64(d,2H) 7.68(dd,1H) 7.80(t,2H) 7.95(d,1H)

Example 35, Compound 309

IR 2922 2854 1636 1607 1522 1466 1214 1172 1096 1079 859 814
NMR 0.89(t,6H) 1.20-1.43(m,20H) 1.43-1.56(m,4H) 1.79-1.91(m,4H) 4.08(t,4H) 6.77-6.86(m,1H) 7.12-7.21(m,3H) 7.54-7.61(m,1H) 7.78(d,2H) 7.88(s,1H)
$^{19}$F-NMR -66.1(dd,1F) -83.0(dd,1F)

Example 36, Compound 321

IR 2922 2856 1638 1607 1522 1466 1214 1172 1096 1079 859 814
NMR 0.91(t,3H) 1.25-1.45(m,14H) 1.45-1.56(m,4H) 1.78-1.91(m,4H) 2.01-2.11(m,2H) 4.01-4.12(m,4H) 4.91-5.04 (m,2H) 5.75-5.90(m,1H) 6.78-6.85(m,1H) 7.12-7.21(m,3H) 7.55-7.61(m,1H) 7.77(d,2H) 7.88(s,1H)
$^{19}$F-NMR -66.1(dd,1F) -83.1(dd,1F)

Example 37, Compound 324

IR 2922 2856 1636 1607 1466 1392 1261 1212 1170 1096 1079 893 855 814
NMR 0.91(t,3H) 1.25-1.45(m,14H) 1.45-1.67(m,4H) 1.78-1.91(m,4H) 2.01-2.11(m,2H) 4.08(t,4H) 4.91-5.04(m,2H) 5.75-5.90(m,1H) 6.78-6.85(m,1H) 7.12-7.22(m,3H) 7.55-7.60(m,1H) 7.76(d,2H) 7.88(s,1H)
$^{19}$F-NMR -66.1(dd,1F) -83.1(dd,1F)

Example 38, Compound 337

IR 2924 1665 1533 1504 1394 1313 1276 1218 1174 1135 1000 855 799
NMR 0.84-0.96(m,6H) 1.23-1.57(m,20H) 1.79-1.91(m,4H) 4.02-4.12(m,4H) 7.00-7.07(m,1H) 7.11-7.21(m,2H) 7.53-7.47(m,2H) 7,62(dd,1H) 7.77(d,2H) 7.88(s,1H)

$^{19}$F-NMR -58.6(t,1F)

Example 39, Compound 356

IR 2924 2854 1603 1531 1504 1468 1394 1313 1274 1218 1172 1135 1019 855
NMR 0.91(t,3H) 1.22-1.51(m,20H) 1.75-1.93(m,4H) 1.99-2.10(m,2H) 4.01-4.13(m,4H) 4.89-5.05(m,2H) 5,72-5.90 (m,1H) 7.03(t,1H) 7.10-7.21(m,2H) 7.32-7.48(m,2H) 7.63 (dd,1H) 7.71-7.84(m,2H) 7.88(d,1H)
$^{19}$F-NMR -58.6(t,1F)

Example 41, Compound 379

IR 2924 2854 1630 1605 1549 1475 1441 1390 1288 1255 1205 855
NMR 0.83-0.95(m,6H) 1.19-1.55(m,26H) 1.76-1.90(m,2H) 4.07(t,2H) 7.11-7.19(m,2H) 7.78(d,1H) 7.85(d,1H) 8.41 (dd,1H) 8.49(s,2H) 8.77(s,1H)

Example 42, Compound 383

IR 2924 2854 1630 1549 1475 1441 1390 1255 1205 907 855
NMR 0.88(t,3H) 1.20-1.57(m,20H) 1.76-1.92(m,4H) 2.03-2.16(m,2H) 4.07(t,2H) 4.91-5.08(m,2H) 5.75-5.90(m,1H) 7.11-7.25(m,2H) 7.79(d,1H) 7.86(d,1H) 8.42(dd,1H) 8.49 (s,2H) 8.78(s,1H)

Example 43, Compound 388

IR 2924 2856 1628 1605 1497 1392 1284 1257 1212 1174 1004 893 822

Example 44, Compound 527

IR 2924 2854 2226 1605 1549 1499 1468 1392 1257 1203 1170 1120 855
NMR 0.84-0.95(m,6H) 1.20-1.55(m,20H) 1.55-1.67(m,2H) 1.79-1.91(m,2H) 2.42(t,2H) 4.08(t,2H) 7.11-7.29(m,4H) 7.44(t,1H) 7.62(d,1H) 7.75(s,1H) 7.78(s,1H) 7.93(s,1H)
$^{19}$F-NMR -42.4(t,1F)

Example 45, Compound 501

IR 2928 2856 2232 1628 1605 1466 1381 1274 1257 1205 1174 1044 841
NMR 0.82-0.99(m,6H) 1.21-1.43(m,12H) 1.43-1.58(m,4H) 1.58-1.72(m,2H) 1.79-1.91(m,2H) 2.46(t,2H) 4.08(t,2H) 7.12-7.20(m,2H) 7.72-7.84(m,4H) 8.08(dd,1H) 8.40(d,1H) 8.72(d,1H)

Example 46, Compound 547

IR 2926 2230 1628 1603 1549 1493 1468 1394 1255 1209 1174 1015 849 820
NMR 0.84-0.96(m,6H) 1.22-1.58(m,20H) 1.59-1.72(m,2H)1.78-1.92(t,2H) 2.47(t,2H) 4.08(t,2H) 7.12-7.23(m,2H) 7.46(d,1H) 7.65(dd,1H) 7.76-7.85(m,2H) 7.88-7.97(m,2H) 8.89(d,1H)

Example 47, Compound 515

IR 2928 2858 2252 1630 1605 1502 1462 1218 1093 853 826
NMR 0.84-0.95(m,6H) 1.18-1.41(m,16H) 1.41-1.58(m,4H) 1.58-1.70(m,2H) 1.79-1.91(m,2H) 2.47(t,2H) 4.09(t,2H) 7.11-7.22(m,4H) 7.57-7.64(m,1H) 7.78(d,2H) 7.92(s,1H)
$^{19}$F-NMR -67.3(dd,1F) -59,9(dd,1F)

Example 49, Compound 104

IR 2926 2856 1630 1605 1493 1468 1386 1255 1207 1176 1123 1025 845 810
NMR 0.82-0.92(m,6H) 1.18-1.88(m,29H) 2.64(t,2H) 4.47-4.58(m,1H) 7.11-7.17(m,2H) 7.56(dd,1H) 7.72-7.85(m,3H) 8.08(dd,1H) 8.37(d,1H) 8.53(d,1H)

Example 50, Compound 391

IR 2928 2856 1632 1607 1502 1468 1392 1261 1212 1125 1096 1079 893 859 818 799
NMR 0.89(t,3H) 0.98(t,3H) 1.06(d,3H) 1.20-1.55(m,15H) 1.55-1.69(m,1H) 1.78-1.99(m,3H) 3.82-3.98(m,2H) 4.08 (t,2H) 6.75-6.85(m,1H) 7.11-7.21(m,3H) 7.55-7.60(m,1H) 7.75(s,1H) 7.78(s,1H) 7.88(s,1H)
$^{19}$F-NMR -66.1(dd,1F) -83.1(dd,1F)

Example 51, Compound 393

IR 2922 2854 1634 1605 1520 1468 1392 1257 1210 1096 1077
NMR 0.82-0.93(m,6H) 1.19-1.69(m,26H) 1.74-1.89(m,3H) 4.08(t,2H) 4.47-4.57(m,1H) 6.76-6.85(m,1H) 7.12-7.21 (m,3H) 7.54-7.59(m,1H) 7.72-7.79(m,2H) 7.87(s,1H)
$^{19}$F-NMR -66.1(dd,1F) -83.1(dd,1F)

Example 52, Compound 161

IR 2918 2852 1630 1605 1454 1276 1027 1000 812 719
NMR 0.88(t,3H) 0.98(t,3H) 1.06(d,3H) 1.19-1.41(m,15H) 1.57-1.70(m,3H) 1.88-1.99(m,1H) 2.78(t,2H) 3.83-3.99 (m,2H) 6.97-7.06(m,1H) 7.11-7.19(m,3H) 7.58-7.64(m,1H) 7.74-7.79(m,2H) 7.92(s,1H)
$^{19}$F-NMR -64.6(dd,1F) -67.7(dd,1F)

Example 53, Compound 164

IR 2922 2854 1628 1605 1499 1458 1396 1259 1220 1127 897 851 824
NMR 0.85-0.95(m,6H) 1.20-1.92(m,29H) 2.70(t,2H) 4.49-4.60(m,1H) 6.98-7.07(m,1H) 7.15-7.23(m,3H) 7.59-7.65 (m,1H) 7.75-7.81(m,2H) 7.93(s,1H)
$^{19}$F-NMR -68.1(dd,1F) -68.5(dd,1F)

Example 54, Compound 395

IR 2922 2856 1605 1510 1470 1394 1236 1166 1116 1027 948 849 808
NMR 0.89(t,3H) 0.97(t,3H) 1.05(d,3H) 1.20-1.55(m,15H) 1.55-1.70(m,1H) 1.78-1.98(m,3H) 3.78-3.94(m,2H) 4.04 (t,2H) 6.77-6.84(m,1H) 7.07-7.18(m,3H) 7.58-7.63(m,1H) 7.75(s,1H) 7.78(s,1H) 7.87(s,1H)
$^{19}$F-NMR -66.2(dd,1F) -83.1(dd,1F)

Example 55, Compound 254

IR 2926 2856 1634 1520 1491 1468 1313 1197 1096 1075 884 822
NMR 0.88 (t,3H) 0.98 (t,3H) 1.05 (d,3H) 1.21-1.42(m,16H) 1.65-1.78(m,2H) 1.88-2.00(m,1H) 2.78(t,2H) 3.81-3.98 (m,2H) 6.78-6.88(m,1H) 7.15-7.23(m,1H) 7.36(dd,1H) 7.56-7.65(m,2H) 7.81(t,2H) 7.92(e,1H)
$^{19}$F-NMR -66.0(dd,1F) -83.1(dd,1F)

Example 56, Compound 255

IR 2928 2858 1632 1580 1514 1466 1379 1296 1120 1056 878 812
NMR 0.82-0.93(m,6H) 1.20-1.89(m,29H) 2.76(t,2H) 4.37-4.47(m,1H) 6.79-6.86(m,1H) 7.13-7.21(m,1H) 7.36(dd,1H) 7.58-7.65(m,2H) 7.81(t,2H) 7.92(s,1H)
$^{19}$F-NMR -65.7(dd,1F) -81.8(dd,1F)

Example 59, Compound 714

IR 2922 2854 1717 1630 1520 1464 1284 1224 1199 1098 1081 984 803
NMR 0.87(t,3H) 0.98(t,3H) 1.05(d,3H) 1.20-1.62(m,16H) 1.78-1.97(m,3H) 4.08(t,2H) 4.15-4.31(m,2H) 6.78-6.88 (m,1H) 7.17-7.27(m,1H) 7.66-7.72(m,1H) 7.90(d,2H) 7.96-8.05(m,2H) 8.09(dd,2H) 8.61(s,1H)
$^{19}$F-NMR -65.8(dd,1F) -82.8(dd,1F)

Example 60, Compound 715

IR 2928 2858 1715 1630 1520 1466 1383 1342 1286 1226 1199 1098 917 801
NMR 0.82-0.98(m,6H) 1.18-1.53(m,25H) 1.62-1.73(m,1H) 1.73-1.92(m,3H) 4.09(t,2H) 5.14-5.29(m,1H) 6.80-6.89 (m,1H) 7.18-7.28(m,1H) 7.69(dd,1H) 7.90(d,1H) 7.96-8.05 (m,2H) 8.09(dd,1H) 8.61(s,1H)
$^{19}$F-NMR -65.7(dd,1F) -82.8(dd,1F)

Example 61, Compound 839

IR 2930 2860 1717 1634 1464 1286 1224 1189 1098 812
NMR 0.82-0.94(m,6H) 1.19-1.39(m,22H) 1.42(d,3H) 1.59-1.74(m,3H) 1.74-1.89(m,1H) 2.72(t,2H) 5.15-5.30(m,1H) 7.00-7.09(m,1H) 7.19-7.28(m,1H) 7.72(dd,1H) 7.80(d,1H) 7.99-8.08(m,2H) 8.10(dd,1H) 8.61(s,1H)
$^{19}$F-NMR -67.6(dd,1F) -68.1(dd,1F)

Example 62, Compound 764

IR 2926 2856 1715 1628 1462 1394 1301 1280 1218 1205 1125 843 775
NMR 0.83-0.94(m,9H) 1.12-1.43(m,15H) 1.43-1.56(m,4H) 1.71-1.92(m,4H) 4.09(t,2H) 4.36(t,2H) 7.13-7.23(m,2H) 7.32-7.40(m,1H) 7.59-7.68(m,1H) 7.73-7.84(m,3H) 7.97 (s,1H)
$^{19}$F-NMR -58.7(dd,1F) -66.4(dd,1F)

Example 63, Compound 765

IR 2926 2856 1715 1628 1497 1460 1394 1303 1205 1125 843 775
NMR 0.88(t,3H) 0.98(t,3H) 1.04(d,3H) 1.20-1.43(m,11H) 1.43-1.60(m,4H) 1.78-1.94(m,4H) 4.08(t,2H) 4.13-4.31 (m,2H) 7.12-7.23(m,2H) 7.32-7.40 (m,1H) 7.59-7.67(m,1H) 7.74-7.84(m,3H) 7.98(s,1H)
$^{19}$F-NMR -58.6(dd,1F) -66.4(dd,1F)

Example 64, Compound 766

IR 2926 2856 1711 1628 1605 1497 1460 1394 1301 1280 1218 1203 1125 843 774
NMR 0.84-0.95(m,6H) 1.20-1.82(m,29H) 4.11(t,2H) 5.15-5.28(m,1H) 7.15-7.25(m,2H) 7.33-7.39(m,1H) 7.60-7.66 (m,1H) 7.74-7.85(m,3H) 7.99(s,1H)
$^{19}$F-NMR -58.9(dd,1F) -66.6(dd,1F)

Example 65, Compound 912

IR 2926 2856 1715 1626 1456 1303 1197 1151 1118 814 777
NMR 0.84-0.95(m,6H) 1.20-1.50(m,25H) 1.60-1.85(m,4H) 2.80(t,2H) 5.16-5.28(m,1H) 7.33-7.42(m,2H) 7.61-7.69 (m,2H) 7.74-7.89(m,3H) 8.02(s,1H)
$^{19}$F-NMR -58.9(dd,1F) -66.4(dd,1F)

Example 66, Compound 834

IR 2928 2858 1717 1632 1568 1475 1284 1216 1181 1139 1100 814 772
NMR 0.83-0.96(m,9H) 1.12-1.56(m,19H) 1.56-1.72(m,2H) 1.72-1.93(m,2H) 2.65(t,2H) 4.38(t,2H) 6.99-7.10(m,2H) 7.46(t,1H) 7.72-7.78(m,1H) 7.91(d,1H) 7.98-8.05(m,2H) 8.09(dd,1H) 8.62(s,1H)
$^{19}$F-NMR -42.8(t,1F)

Example 68, Compound 50

IR 2922 2852 1624 1605 1506 1470 1394 1253 1212 1176 1122 1029 1002 888 851
NMR 0.84-0.98(m,6H) 1.20-1.45(m,22H) 1.45-1.59(m,2H) 1.59-1.71(m,2H) 1.80-1.92(m,2H) 2.68(t,2H) 4.08(t,2H) 7.12-7.44(m,5H) 7.65(dd,1H) 7.78(d,2H) 7.95(d,1H)
$^{19}$F-NMR -43.0(t,1F)

Example 69, Compound 69

IR 2922 2854 1630 1605 1504 1468 1394 1253 1205 1176 1021 818
NMR 0.85-0.95(m,6H) 1.21-1.45(m,22H) 1.45-1.59(m,2H) 1.59-1.73(m,2H) 1.80-1.92(m,2H) 2.67(t,2H) 4.08(t,2H) 7.12-7.20(m,2H) 7.29(d,2H) 7.62(d,2H) 7-66-7.82(m,3H) 7.95(d,1H)

Example 70, Compound 53

IR 2924 2854 1630 1605 1549 1487 1466 1437 1390 1259 1201 909 861
NMR 0.88(t,3H) 1.20-1.58(m,20H) 1.58-1.71(m,2H) 1.79-1.90(m,2H) 2.01-2.11(m,2H) 2.61(t,2H) 4.08(t,2H) 4.91-5.05(m,2H) 5.75-5.89(m,1H) 7.12-7.19(m,2H) 7.80(d,1H) 7.88(d,1H) 8.47 (dd,1H) 8.64(s,2H) 8.85(d,1H)

Example 71, Compound 56

IR 2920 2854 1630 1605 1504 1470 1392 1253 1212 1172 1027 909 864 816
NMR 0.82-0.97(t,3H) 1.20-1.58(m,20H) 1.58-1.78(m,2H) 1.80-1.92(m,2H) 2.63(t,2H) 4.08(t,2H) 4.91-5.08(m,2H) 5.74-5.92(m,1H) 7.11-7.22(m,2H) 7.57(dd,1H) 7.72-7.88 (m,3H) 8.08(dd,1H) 8.39(s,1H) 8.55(d,1H)

Example 72, Compound 62

IR 2924 2854 1630 1605 1502 1473 1392 1255 1201 1120 1031 857
NMR 0.90(t,3H) 1.24-1.60(m,20H) 1.60-1.72(m,2H) 1.82-1.94(m,2H) 2.05-2.15(m,2H) 2.65(t,2H) 4.10(t,2H) 4.94-5.09(m,2H) 5.78-5.93(m,1H) 7.00-7.10(m,2H) 7.15-7.22 (m,2H) 7.45(t,1H) 7.62-7.70(m,1H) 7.79(d,1H) 7.95(s,1H)
$^{19}$F-NMR -43.0(t,1F)

Example 73, Compound 63

IR 2922 2854 1633 1607 1502 1462 1392 1261 1216 1023 893 859 814
NMR 0,89 (t,3H) 1.21-1.59(m,20H) 1.59-1.72(m,2H) 1.79-1.92(m,2H) 1.99-2.11(m,2H) 2.69(t,2H) 4.09(t,2H) 4.90-5.05(m,2H) 5.75-5.92(m,1H) 6.95-7.05(m,2H) 7.12-7.25(m,3H) 7.56-7.65(m,1H) 7.77(d,2H) 7.92(s,1H)
$^{19}$F-NMR -68.1(dd,1F) -68.5(dd,1F)

Example 74, Compound 707

IR 2922 2854 1715 1632 1520 1466 1288 1228 1201 1102 1079 977 797
NMR 0.82-0.95(m,6H) 1.18-1.55(m,24H) 1.74-1.93(m,4H) 4.09(t,2H) 4.38(t,2H) 6.80-6.89(m,1H) 7.16-7.25(m,1H) 7.66-7.72(m,1H) 7.90(d,1H) 7.97-8.03(m,2H) 8.09(dd,1H) 8.61(s,1H)
$^{19}$F-NMR -65.7(dd,1F) -82.8(dd,1F)

Example 75, Compound 725

IR 2922 2854 1767 1717 1618 1528 1473 1313 1253 1226 1133 1106 866 804
NMR 0.82-0.97(m,6H) 1.13-1.42(m,20H) 1.42-1.54(m,4H) 1.75-1.92(m,4H) 4.08(t,2H) 4.38(t,2H) 7.00-7.10(m,1H) 7.38-7.51(m,2H) 7.72(dd,1H) 7.90(d,1H) 7.96-8.03(m,2H) 8.08(dd,1H) 8.60(s,1H)
$^{19}$F-NMR -58.2(t,1F)

Example 76, Compound 757

IR 2922 2856 1715 1626 1454 1394 1323 1272 1207 1156 1021 845 816
NMR 0.81-0.96(m,6H) 1.17-1.58(m,24H) 1.72-1.93(m,4H) 4.08(t,2H) 4.36(t,2H) 7.12-7.23(m,2H) 7.31-7.41(m,1H) 7.58-7.67(m,1H) 7.72-7.84(m,3H) 7.98(s,1H)
$^{19}$F-NMR -58.7(dd,1F) -66.5(dd,1F)

Example 77, Compound 776

IR 2922 2856 1700 1622 1566 1504 1470 1406 1296 1278 1214 1189 1147 1015 841

NMR 0.82-0.96(m,6H) 1.19-1.57(m,24H) 1.72-1.92(m,4H) 4.08(t,2H) 4.34(t,2H) 7.12-7.23(m,2H) 7.45(dd,1H) 7.53 (dd,1H) 7.68(dd,1H) 7.80(d,2H) 7.96-8.08(m,2H)
$^{19}$F-NMR -33.4(t,1F)

Example 78, Compound 817

IR 2926 2856 1754 1692 1630 1468 1373 1319 1251 1214 1187 1058 812
NMR 0.83-0.98(m,6H) 1.13-1.43(m,20H) 1.42-1.54(m,4H) 1.76-1.93(m,4H) 2.74(t,2H) 4.38(t,2H) 7.01-7.10(m,1H) 7.19-7.28(m,1H) 7.69-7.79(m,1H) 7.82(dd,1H) 7.96(t,2H) 8.06(s,1H) 8.31(s,1H)
$^{19}$F-NMR -67.6(dd,1F) -68.1(dd,1F)

Example 79, Compound 907

IR 2920 2854 1713 1626 1454 1323 1220 1156 882 820
NMR 0.82-0.96(m,6H) 1.20-1.53(m,24H) 1.64-1.87(m,4H) 2.78(t,2H) 4.38(t,2H) 7.32-7.44(m,2H) 7.59-7.69(m,2H) 7.74-7.96(m,3H) 8.02(s,1H)
$^{19}$F-NMR -58.7(dd,1F) -66.3(dd,1F)

Example 80, Compound 657

IR 2924 2856 1607 1495 1460 1379 1307 1216 1174 1116 1085 886 824
NMR 0.83-0.96(m,6H) 1.20-1.47(m,22H) 1.60-1.79(m,4H) 2.71(t,2H) 2.79(t,2H) 6.98-7.08(m,1H) 7.15-7.25(m,1H) 7.37(dd,1H) 7.58-7.68(m,2H) 7.82(t,2H) 7.79(s,1H)
$^{19}$F-NMR -68.0(dd,1F) -68.4(dd,1F)

Example 31, Compound 243

IR 2924 2854 1618 1531 1468 1435 1313 1272 1131 1017 911 870 803
NMR 0.91(t,3H) 1.22-1.58(m,18H) 1.62-1.78(m,2H) 1.78-1.92(m,2H) 2.00-2.10(m,2H) 2.78(t,2H) 4.08(t,2H) 4.91-5.07(m,2H) 5.76-5.90(m,1H) 7.05(t,1H) 7,32-7.50(m,3H) 7.58-758(m,2H) 7.82(t,1H) 7.94(d,1H)
$^{19}$F-NMR -58.6(t,1F)

Example 40, Compound 362

IR 2924 2856 1605 1533 1468 1396 1315 1274 1218 1172 1135 1025 891 855 816
NMR 0.91(t,3H) 1.22-1.60(m,20H) 1.80-1.94(m,4H) 1.96-2.12(m,2H) 4.01-4.14(m,4H) 4.91-5.06(m,2H) 5.25-5.93 (m, 1H) 6.99-7.09(m, 1H) 7.11-7.23(m,2H) 7.35-7.50(m,2H) 7.63(d,1H) 7.76(d,2H) 7.90(s,1H)
$^{19}$F-NMR -58.6(t,1F)

Example 57, Coupound 401

IR 2924 2856 1705 1605 1510 1470 1396 1309 1274 1210 1137 1062 866 801
NMR 0.82-1.00(m,6H) 1.22-1.66(m,34H) 1.75-1.94(m,3H) 4.08-4.15(t,2H) 4.48-4.58(m,1H) 7.05(t,1H) 7.12-7.22 (m,2H) 7.35-7.50(m,2H) 7.64(dd,1H) 7.72-7.84(m,2H) 7.89 (s,1H)
$^{19}$F-NMR -58.6(t,1F)

Example 58, Compound 832

IR 2926 2856 1718 1626 1490 1462 1377 1285 1065 1028 928
NMR 0.83-0.95(m,6H) 1.19-1.39(m,22H) 1.42(d,3H) 1.59-1.74(m,3H) 1.74-1.89(m,1H) 2.64(t,2H) 5.16-5.31(m,1H) 6.98-7.10(m,2H) 7.65(dd;1H) 7.90-8.05(m,3H) 8.08(dd,1H) 8.47(s,1H) 8.62(s,1H)

Example 81, Compound 74

IR 2925 2856 1630 1600 1497 1440 1250 1220 1125 850 823 791
NMR 0.85-0-95(m,6H) 1.15-2.00(m,26H) 2.70(t,2H) 4.14 (dd,2H) 4.81(m,1H) 6.98-7.07(m,1H) 7.15-7.23(m,3H) 7.59-7.65(m,1H) 7.75-7.81(m,2H) 7.93(m,1H)

Compound 397

IR 2934 2860 1632 1605 1504 1468 1394 1216 1170 1129 1056 973 895 853 822
NMR 0.83-0.96(m,6H) 1.21-1.57(m,25H) 1.57-1.70(m,1H) 1.71-1.92(m,3H) 4.00-4.14 (m,2H) 4.36-4.49(m,1H) 6.78-6.88(m,1H) 7.05-7.22(m,3H) 7.54-7.15(m,2H) 7.77(d,2H) 7.8(s,1H)
$^{19}$F-NMR -65.8(dd,1F) -81.8(dd,1F)

## Claims

**1.** A liquid crystal compound, represented by the following formula (1):

R-Z-A-NAP-Z'-R' (1)

wherein R represents an alkyl group containing 1 to 18 carbon atoms; R' represent an alkyl group containing 1 to 21 carbon atoms; NAP represents 2,6-naphthylene group; A is a cyclic group selected from the group consisting of Pyr>, <Pyr, Pym>, FPhF, PhF, FPh and Ph; Z is -, #, O or OCO, and Z' is O, COO, - or #; wherein Pyr>, <Pyr, Pym>, FPhF, PhF, FPh, Ph, - and # represent respectively

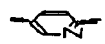 ,

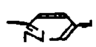 , 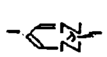 , 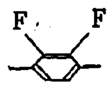 , 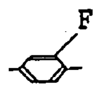 , 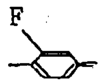 , 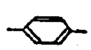 ,

a single bond and C≡C with the provisos that
1) if A is Pyr>,
(a) Z is - or # and Z' is O, or
(b) Z is - and Z' is COO;
2) if A is Pym>,
(a) Z is - and Z' is O, or
(b) Z is O and Z' is -, O or #;
3) if A is FPhF,
(a) Z is - or O and Z' is -, O or COO,
(b) Z is OCO and Z' is - or O, or
(c) Z is # and Z' is O;
4) if A is PhF,
(a) Z is - or # and Z' is O,
(b) Z is O and Z' is -, or
(c) Z is - and Z' is COO;
5) if A is FPh,
(a) Z is O and Z' is -,
(b) Z is -, O or # and Z' is O,
(c) Z is O or # and Z' is COO, or
(d) Z is OCO and Z' is O;
6) if A is <Pyr, Z is -, O or # and Z' is O; and
7) if A is Ph, one of Z and Z' is - and the other is O.

**2.** A compound according to Claim 1, wherein A is Pyr>, <Pyr or Pym>.

**3.** A compound according to Claim 1, wherein A is FPhF, PhF, FPh or Ph.

**4.** A compound according to any one of Claims 1-3, wherein
(i) Z is - and Z' is O,
(ii) Z is O and Z' is -,
(iii) Z is O and Z' is O, or
(iv) Z is # and Z' is O.

**5.** A compound according to Claim 2, wherein A is Pyr>, and Z is - or # and Z' is O.

**6.** A compound according to Claim 2, wherein A is Pym>, and Z is - or O and Z′ is O.

**7.** A compound according to Claim 3, wherein A is FPhF, and one of Z and Z′ is O and the other is - or O.

**8.** A compound according to Claim 3, wherein A is PhF, and Z is - and Z′ is O.

**9.** A compound according to Claim 3, wherein A is FPh, and Z is O and Z′ is O or -.

**10.** A compound according to Claim 1, wherein at least one of R and R′ is an optically active alkyl group, particularly a group represented by the formula:

$$-(CH_2)_n-{}^{*}\overset{\overset{CH_3}{/}}{CH}-R''$$

wherein n is an integer of 0-9 and R″ represents an alkyl group containing 2-10 carbon atoms.

**11.** A liquid crystal composition, containing as components thereof a mixture of a plurality of crystal compounds, said liquid crystal compounds comprising at least one compound according to any one of Claims 1-10, said composition optionally containing a two-tone dyestuff.

**12.** A liquid crystal display element, containing the liquid crystal compound according to any one of Claims 1-10 or liquid crystal composition according to Claim 11.

European Patent Office

# EUROPEAN SEARCH REPORT

Application Number

EP 92 30 5079

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-9 008 119 (SECRETARY OF STATE FOR DEFENCE)<br>* page 1, line 1 - line 3 *<br>* page 2, line 1 - line 23 *<br>* page 3, line 4 - line 26 *<br>* claims 1,3,15,16; examples 2,6 * | 1,3,4, 7-9,11 | C09K19/32<br>C09K19/34<br>C09K19/42<br>C07C43/225<br>C07C43/20<br>C07C43/205<br>C07C69/76<br>C07C69/94<br>C07D213/30<br>C07D213/06<br>C07D213/55<br>C07D213/64<br>C07D213/79<br>C07D239/26<br>C07D239/34 |
| X | EP-A-0 151 294 (CHISSO CORPORATION)<br>* page 1, line 1 - line 2 *<br>* page 2, line 18 - page 3, line 18 *<br>* claims 1,3; examples 5-8,13; table 1 * | 1,2,4,6, 11,12 | |
| X | BULLETIN DE LA SOCIETE CHIMIQUE DE FRANCE.<br>no. 11, 1975, PARIS FR<br>pages 2521 - 2525;<br>K. CHEBAANE,M.GUYOT,D.MOLHO: 'synthèse d'aryl-2 naphtalènes et de dibenzocoumarines.'<br>* tables 1,COMPOUND,2K * | 1,3,4 | |
| A | GB-A-2 111 992 (HOFFMANN-LA ROCHE)<br>* claim 1 * | 1 | TECHNICAL FIELDS SEARCHED (Int. Cl.5 )<br>C09K<br>C07C |

The present search report has been drawn up for all claims

| Place of search | Date of completion of the search | Examiner |
|---|---|---|
| THE HAGUE | 08 SEPTEMBER 1992 | PUETZ C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)